# EUROPEAN PATENT APPLICATION

(11) **EP 2 305 671 A1**
(43) Date of publication of application: **06.04.2011**
(21) Application number: 10184816.6
(22) Date of filing: 24.12.2004
(51) Int. Cl.: C07D 409/12, C07D 453/02, C07D 333/38, C07D 409/14, C07D 413/12, C07D 413/14, C07D 417/14, A61K 31/41, A61K 31/40, A61K 31/435, A61K 31/495, A61K 31/55, A61P 35/00

(54) **Thiophene and thiazole derivatives as CHK1 inhibitors**

(30) Priority: 05.01.2004 US 534310 P; 15.03.2004 US 553305 P
(62) Divisional of application: 04806196.4
(71) Applicant: AstraZeneca AB, 151 85 Södertälje (SE)
(72) Inventor: Ashwell, Susan, Waltham, MA 02451 (US); Gero, Thomas, Waltham, MA 02451 (US); Ioannidis, Stephanos, Waltham, MA 02451 (US); Janetka, James, Waltham, MA 02451 (US); Lyne, Paul, Waltham, MA 02451 (US); Su, Mei, Waltham, MA 02451 (US); Toader, Dorin, Waltham, MA 02451 (US); Yu, Dingwei, Waltham, MA 02451 (US); Yu, Yan, Waltham, MA 02451 (US)
(74) Representative: Padget, Lucy Clare

(57) **Abstract**

This invention relates to novel compounds having the structural formula (I) 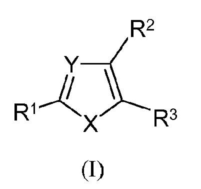 and to their pharmaceutical compositions and to their methods of use. These novel compounds provide a treatment or prophylaxis of cancer.

## Description

### Field of the invention

The present invention relates to novel substituted heterocycles, their pharmaceutical compositions and methods of use. In addition, the present invention relates to therapeutic methods for the treatment and prevention of cancers.

### Background of the invention

Chemotherapy and radiation exposure are currently the major options for the treatment of cancer, but the utility of both these approaches is severely limited by drastic adverse effects on normal tissue, and the frequent development of tumor cell resistance. It is therefore highly desirable to improve the efficacy of such treatments in a way that does not increase the toxicity associated with them. One way to achieve this is by the use of specific sensitizing agents such as those described herein.

An individual cell replicates by making an exact copy of its chromosomes, and then segregating these into separate cells. This cycle of DNA replication, chromosome separation and division is regulated by mechanisms within the cell that maintain the order of the steps and ensure that each step is precisely carried out. Key to these processes are the cell cycle checkpoints (Hartwell et al., Science, Nov 3, 1989, 246(4930):629-34) where cells may arrest to ensure DNA repair mechanisms have time to operate prior to continuing through the cycle into mitosis. There are two such checkpoints in the cell cycle - the G1/S checkpoint that is regulated by p53 and the G2/M checkpoint that is monitored by the Ser/Thr kinase checkpoint kinase 1 (CHK1).

As the cell cycle arrest induced by these checkpoints is a crucial mechanism by which cells can overcome the damage resulting from radio- or chemotherapy, their abrogation by novel agents should increase the sensitivity of tumor cells to DNA damaging therapies. Additionally, the tumor specific abrogation of the GI/S checkpoint by p53 mutations in the majority of tumors can be exploited to provide tumor selective agents. One approach to the design of chemosensitizers that abrogate the G2/M checkpoint is to develop inhibitors of the key G2/M regulatory kinase CHK1, and this approach has been shown to work in a number of proof of concept studies. (Koniaras et al., Oncogene, 2001, 20:7453; Luo et al., Neoplasia, 2001, 3:411; Busby et al., Cancer Res., 2000, 60:2108; Jackson et al., Cancer Res., 2000, 60:566).

Smithkline Beecham Corporation describes 2-ureidothiophene compounds in WO03029241 and 3-ureidothiophene compounds in WO03028731 as CHK1 inhibitors. The present invention provides novel CHK1 inhibitors with improved properties.

### Summary of the invention

In accordance with the present invention, the applicants have hereby discovered novel compounds that are potent inhibitors of the kinase CHK1 and therefore possess the ability to prevent cell cycle arrest at the G2/M checkpoint in response to DNA damage. These compounds are accordingly useful for their anti-proliferative (such as anti-cancer) activity and are therefore useful in methods of treatment of the human or animal body. The invention also relates to processes for the manufacture of said compounds, to pharmaceutical compositions containing them and to their use in the manufacture of medicaments for use in the production of an anti-proliferative effect in warm-blooded animals such as man.

The present invention includes pharmaceutically acceptable salts or prodrugs of such compounds. Also in accordance with the present invention applicants provide pharmaceutical compositions and a method to use such compounds in the treatment of cancer.

Such properties are expected to be of value in the treatment of disease states associated with cell cycle arrest and cell proliferation such as cancers (solid tumors and leukemias), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

### Detailed Description of the Invention

Accordingly, the present invention provides a compound of formula (I) 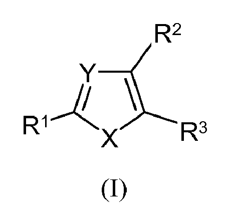 wherein:
**X** is selected from NH, S and O; **Y** is selected from CH or N;
**R¹** is selected from cyano, isocyano, C₁₋₆alkyl, -NR¹¹R¹², C₁₋₆alkoxy, C₂₋₆alkenyl, C₂₋₆alkynyl, cycloalkyl, cycloalkenyl, aryl, and heterocyclyl, provided R¹ is not thienyl; and wherein R¹ may be optionally substituted on one or more carbon atoms by one or more R⁹; and wherein if said R¹ contains an -NH- moiety, the nitrogen of said moiety may be optionally substituted by a group selected from R¹⁰;
**R² and R³** are each independently selected from -C(=O)NR⁶R⁷, -SO₂NR¹⁶R¹⁷, -NHC(=O)NHR⁴, and -NHC(=NR⁸)NH₂;
**R⁴** is selected from H, OH, -NR¹¹R¹², benzyl, C₁₋₆alkoxy, cycloalkyl, cylcoalkenyl, aryl, heterocyclyl, mercapto, CHO, -COaryl, -CO(C₁₋₆alkyl), -CONR³⁰R³¹, -CO₂(C₁₋₆alkyl), -CO₂aryl, -CO₂NR³⁰R³¹, -Salkyl, -SO(C₁₋₆alkyl), -SO₂(C₁₋₆alkyl), -Saryl, -SOaryl, -SO₂aryl,-SO₂NR³⁰R³¹, and -(C₁₋₆alkyl)SO₂NR³⁰R³¹ wherein R⁴ may be optionally substituted on one or more carbon atoms by one or more R¹⁵; and wherein if said heterocyclyl contains a -NH- moiety, the nitrogen may be optionally substituted by a group selected from R¹⁴;
**R⁶ and R⁷** are each independently selected from H, OH, OCH₃, C₁₋₆alkoxy, -NH₂, - NHCH₃, -N(CH₃)₂, (C₁₋₃alkyl)NR¹¹R¹², -CH₂CH₂OH, cycloalkyl, and a 5, 6, or 7- membered heterocyclyl ring containing at least one nitrogen atom, provided R⁶ and R⁷ are not both H; alternatively R⁶ and R⁷ taken together with the N to which they are attached form a heterocyclic ring; wherein R⁶ and R⁷ independently of each other may be optionally substituted on one or more carbon atoms by one or more R¹⁸; and wherein if said heterocyclyl contains a -NH- moiety, the nitrogen of said moiety may be optionally substituted by a group selected from R¹⁹;
**R⁸** is selected from cyano, isocyano, -SO₂(C₁₋₆alkyl), -SO₂-aryl; -SO₂cycloalkyl, - SO₂cycloalkenyl, -SO₂heterocyclyl, and CF₃; wherein R⁸ may be optionally substituted on one or more carbon atoms by one or more R²³;
**R⁹, R¹⁵, R¹⁸, R²³, R²⁴ and R³³** are each independently selected from halogen, nitro, -NR³⁰R³¹, cyano, isocyano, C₁₋₆alkyl, C₂₋₆alkenl C₂₋₆alkynyl aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R⁹, R¹⁵, R¹⁸, R²³, R²⁴ and R³³ independently of each other may be optionally substituted on carbon by one or more R²⁰ and on nitrogen of any moiety that contains an NH or NH₂ by R²¹;
**R¹⁰, R¹⁴, R¹⁹, R²⁵ and R³⁴** are each independently selected from halogen, nitro, -NR³⁰R³¹, cyano, isocyano, C₁₋₆alkyl C₂₋₆alkenyl C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R¹⁰, R¹⁴, R¹⁹, R²⁵ and R³⁴ independently of each other may be optionally substituted on carbon by one or more R²² and on nitrogen of any moiety that contains an NH or NH₂ by R²³ ;
**R¹¹ and R¹²** are independently selected from H, C₁₋₆alkyl, cycloalkyl, aryl, heterocyclyl; alternatively R¹¹ and R¹² taken together with the N to which they are attached form a heterocyclic ring; wherein R¹¹ and R¹² independently of each other may be optionally substituted on carbon by one or more R³³_{;} and wherein if said heterocyclyl contains a -NH- moiety, the nitrogen of said moiety may be optionally substituted by a group selected from R³⁴;
**R¹⁶ and R¹⁷** are each independently selected from H, OH, OCH₃, C₁₋₆alkoxy, NH₂, -NHCH₃, -N(CH₃)₂, (C₁₋₃alkyl)NR¹¹R¹², -CH₂CH₂OH, cycloalkyl, aryl, or a 5, 6 or 7-membered heterocyclyl ring containing at least one nitrogen atom, provided R¹⁶ and R¹⁷ are not both H; alternatively R¹⁶ and R¹⁷ taken together with the N to which they are attached form an optionally substituted heterocyclic ring; wherein R¹⁶ and R¹⁷ independently of each other may be optionally substituted on one or more carbon atoms by one or more R²⁴; and wherein if said heterocyclyl contains an -NH- moiety, the nitrogen of said moiety may be optionally substituted by a group selected from R²⁵;
**R²⁰, R²² and R³²** are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHC0₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁-₆alkyl), -COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R²⁰, R²¹ and R³² independently of each other may be optionally substituted on carbon by one or more R²⁶ and on nitrogen of any moiety that contains an NH or NH₂ by R²⁷;
**R^{21,} R²³ and R³⁵** are each independently selected from halogen, nitro, -NR³⁰R³¹ cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁₋₆alkyl) CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R²¹, R²³ and R³⁵ independently of each other may be optionally substituted on carbon by one or more R²⁸ and on nitrogen of any moiety that contains an NH by R²⁹ ;
**R²⁶ and R²⁸** are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹;
**R²⁷ and R²⁹** are each independently selected from halogen, nitro, -NR³⁰R³¹, cyano, isocyano, C₁_₆alkyl, C₂-₆alkenyl, C₂-₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁₋₆alkyl)CO(C₁-₆alkyl), -NHSO₂(C₁₋₆alkyl), carboxy, -amidino, -CHO, -CONR³⁰R³¹, -CO(C₁₋₆alkyl), - COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹;
**R³⁰ and R³¹** are each independently selected from halogen, nitro, -NH₂, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR¹¹R¹², -N(C₁₋₆alkyl)CONR¹¹R¹¹, -NHCOalkyl, -NHCO₂(C₁₋₆alkyl); -NHCO₂H, -N(C₁₋₆alkyl)CO(C₁-₆alkyl), -NHSO₂(C₁₋₆alkyl), carboxy, -amidino, -CHO, -CONR³⁰R³¹, -CO(C₁₋₆alkyl),-COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO2NR¹¹R¹²; wherein R³⁰ and R³¹ independently of each other may be optionally substituted on carbon by one or more R³²; and wherein if said heterocyclyl contains a -NH- or NH₂ moiety, the nitrogen of said moiety may be optionally substituted by a group selected from R³⁵;
or a pharmaceutically acceptable salt thereof;
provided that when X is S; Y is CH; R₂ is C(=O)NR⁶R⁷; and R³ is NHC(=O)NHR⁴; then R¹ cannot be 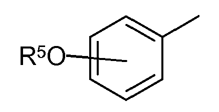 wherein R⁵ is selected from H, optionally substituted carbocyclyl, or optionally substituted C₁₋₆alkyl;
with the further proviso that said compound is not
5-Methyl-2-ureido-thiophene-3-carboxylic acid (1-ethyl-piperidin-3-yl)-amide;
[3-((S)-3-Amino-azepane-1-carbonyl)-5-ethyl-thiophen-2-yl]-urea;
2-Morpholin-4-yl-4-ureido-thiazole-5-carboxylic acid (S)-piperidin-3-ylamide;
2-Methyl-5-ureido-oxazole-4-carboxylic acid (S)-piperidin-3-ylamide;
5-(4-Chloro-phenyl)-3- {3-[(R)-1 -(2,2,2-trifluoro-acetyl)-piperidin-3-yl]-ureido} - thiophene-2-carboxylic acid (S)-piperidin-3-ylamide; or
N-(3- {[(3S)-3-aminoazepan-1-yl]carbonyl} -5-pyridin-2-yl-2-thienyl)urea.

The following substituents for the variable groups contained in formula (I) are further embodiments of the invention. Such specific substituents may be used, where appropriate, with any of the definitions, claims or embodiments defined hereinbefore or hereinafter.
X is S.
X is O.
X is NH.
Y is CH.
Y is N.
R¹ is -NR¹¹R¹², Cl-6alkoxy, C₂₋₆alkenyl, C₂₋₆alkynyl, cycloalkyl, cycloalkenyl, aryl, or heterocyclyl, provided R¹ is not thienyl; and wherein R¹ may be optionally substituted on one or more carbon atoms by one or more R⁹; and wherein if said heterocyclyl contains an -NH- moiety, the nitrogen of said moiety may be optionally substituted by a group selected from R¹⁰.
R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰.
R¹ is a heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰.
R¹ is a heterocyclyl selected from the group consisting of pyridinyl, pyrrolyl, pyrazinyl, pyrimidinyl, furanyl, isoxazolyl, isoindolyl, benzofuranoyl, piperdinyl, and morpholinyl wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰.
R¹ is aryl optionally substituted on one or more carbon atoms by one or more R⁹.
R¹ is phenyl optionally substituted on one or more carbon atoms by one or more R⁹.
R¹ is phenyl optionally substituted on one or more carbon atoms by one or more by R⁹, wherein R⁹ is selected from halogen, amino, C₁₋₆alkyl, and C₁₋₆alkoxy.
R¹ is phenyl optionally substituted on one or more carbon atoms by one or more by R⁹, wherein R⁹ is selected from halogen, C₁₋₆alkyl, and C₁₋₆alkoxy.
R¹ is not t-butyl.
R² and R³ are not both C(=O)NR⁶R⁷.
One of R² and R³ is C(=O)NR⁶R⁷ and the other is -NHC(=0)NHR⁴.
One of R² and R³ is C(=O)NR⁶R⁷ and the other is -NHC(=O)NHR⁴, wherein R⁴ is H.
One of R² and R³ is C(=O)NR⁶R⁷ and the other is -NHC(=O)NHR⁴, wherein R⁶ is H and R⁷ is a 5, 6, or 7-membered heterocycyclyl ring containing at least one nitrogen atom wherein said heterocyclyl may be optionally substituted on one or more carbon atoms by one or more R¹⁸; and further wherein if said heterocyclyl contains an -NH- moiety, the nitrogen of said moiety may be optionally substituted by a group selected from R¹⁹.
One of R² and R³ is C(=O)NR⁶R⁷ and the other is -NHC(=O)NHR⁴, wherein R⁶ is H and R⁷ is pyrrolin-3-yl, piperdin-3-yl, or azepan-3-yl wherein said pyrrolidin-3-yl, piperdin-3-yl, or azepan-3-yl may be optionally substituted on one or more carbon atoms by one or more R¹⁸; and further wherein said pyrrolidin-3-yl, piperdin-3-yl, or azepan-3-yl may be optionally substituted on N by a group selected from R¹⁹.
One of R² and R³ is C(=O)NR⁶R⁷ and the other is -NHC(=O)NHR⁴, wherein R⁴ and R⁶ are H and R⁷ is pyrrolidin-3-yl, piperdin-3-yl, or azepan-3-yl wherein said pyrrolidin-3-yl, piperdin-3-yl, or azepan-3-yl may be optionally substituted on one or more carbon atoms by one or more R⁹; and further wherein said pyrrolidin-3-yl, piperdin-3-yl, or azepan-3-yl may be optionally substituted on N by a group selected from R¹⁰.
One of R² and R³ is -SO₂N R¹⁶R¹⁷ and the other is -NHC(=O)NHR⁴.
One of R² and R³ is -SO₂N R¹⁶R¹⁷ and the other is -NHC(=O)NHR⁴ and R⁴ is H.
R⁶ and R⁷ taken together with the N to which they are attached form an optionally substituted heterocyclic ring which may be optionally substituted on one or more carbon atoms by one or more R¹⁸; and wherein if said heterocyclyl contains a -NH- moiety, the nitrogen of said moiety may be optionally substituted by a group selected from R¹⁹.
R⁶ and R⁷ taken together with the N to which they are attached form an optionally substituted heterocyclic ring which ring contains a second N atom and further which ring may be optionally substituted on one or more carbon atoms by one or more R¹⁸; and wherein if said heterocyclyl contains a -NH- moiety, the nitrogen of said moiety may be optionally substituted by a group selected from R¹⁹_{;}
R¹⁶ and R¹⁷ taken together with the N to which they are attached form an optionally substituted heterocyclic ring which may be optionally substituted on one or more carbon atoms by one or more R²⁴; and wherein if said heterocyclyl contains a -NH- moiety, the nitrogen of said moiety may be optionally substituted by a group selected from R²⁵.
R¹⁶ and R¹⁷ taken together with the N to which they are attached form an optionally substituted heterocyclic ring which ring contains a second N atom and further which ring may be optionally substituted on one or more carbon atoms by one or more R²⁴; and wherein if said heterocyclyl contains a -NH- moiety, the nitrogen of said moiety may be optionally substituted by a group selected from R²⁵.

In another embodiment of the present invention, when R⁶ or R⁷ is a 5, 6, or 7-membered heterocycyclyl ring containing at least one nitrogen atom, said 5, 6, or 7-membered heterocycyclyl ring is selected from azepanyl, pyrrolidinyl, imidazolidinyl, piperdinyl, morpholinyl and thiomorpholinyl.

In a further embodiment, the present invention provides a compound of formula (I) wherein when X is S and Y is N and one of R² and R³ is -NHC(=O)NHR⁴, then R⁴ is H; or a pharmaceutically acceptable salt thereof.

A particular embodiment of the compounds of formula (I ) of the present invention are compounds of formula (II) and pharmaceutically acceptable salts thereof 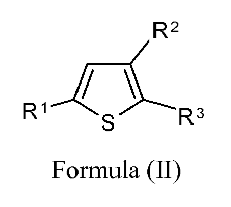 wherein R¹, R² and R³ are as defined for formula (I).

In another embodiment, the present invention provides a compound of formula (I) or formula (II) wherein:
R² is -C(=O)NR⁶R⁷
R³ is -NHC(=O)NHR⁴;
R⁶ is H; R⁷ is a 5, 6, or 7-membered heterocyclyl ring containing at least one nitrogen atom; wherein said heterocyclyl may be optionally substituted on one or more carbon atoms by one or more R¹⁸; and further wherein if said heterocyclyl contains an -NH- moiety, the nitrogen of said moiety may be optionally substituted by a group selected from R¹⁹;and
R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰; or a pharmaceutically acceptable salt thereof.

In a further embodiment, the present invention provides a compound of formula (I) or formula (II) wherein:
R² is -C(=O)NR⁶R⁷;
R³ is -NHC(=O)NHR⁴;
R⁶ is H; R⁷ is a 5, 6, or 7-membered heterocyclyl ring containing at least one nitrogen atom; wherein R⁷ may be optionally substituted on one or more carbon atoms by one or more R¹⁸; and wherein if said heterocyclyl contains a -NH- moiety, the nitrogen of said moiety may be optionally substituted by a group selected from R¹⁹; and
R¹ is selected from cycloalkyl, cycloalkenyl, aryl, and heterocyclyl provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰; or a pharmaceutically acceptable salt thereof.

In another embodiment, the present invention provides a compound of formula (I) or (II) wherein:
R³ is -C(=O)NR⁶R⁷;
R² is -NHC(=O)NHR⁴;
R⁶ is H; R⁷ is a 5, 6, or 7-membered heterocyclyl ring containing at least one nitrogen atom wherein R⁷ may be optionally substituted on one or more carbon atoms by one or more R¹⁸; and wherein if said heterocyclyl contains a -NH- moiety, the nitrogen of said moiety may be optionally substituted by a group selected from R¹⁹; and
R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰; or a pharmaceutically acceptable salt thereof.

In a further embodiment, the present invention provides a compound of formula (I) or (II) wherein:
R³ is -C(=O)NR⁶R⁷;
R² is -NHC(=O)NHR⁴;
R⁶ is H; R⁷ is a 5, 6, or 7-membered heterocyclyl ring containing at least one nitrogen atom; wherein said heterocyclyl ring may be optionally substituted on one or more carbon atoms by one or more R¹⁸; and further wherein if said heterocyclyl contains a -NH- moiety, the nitrogen of said moiety may be optionally substituted by a group selected from R¹⁹; and
R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰; or a pharmaceutically acceptable salt thereof.

In a still further embodiment, the present invention provides a compound of formula (I) or (II) wherein:
R¹ is aryl optionally substituted on one or more carbon atoms by R⁹;
R² and R³ are independently selected from -C(=O)NR⁶R⁷ and -NHC(=O)NHR⁴;
R⁴ and R⁶ are both H; and
R⁷ is a 5, 6, or 7-membered heterocyclyl ring containing at least one nitrogen wherein said heterocyclyl ring may be optionally substituted on one or more carbon atoms by one or more R¹⁸; and further wherein if said heterocyclyl contains a -NH- moiety, the nitrogen of said moiety may be optionally substituted by a group selected from R¹⁹;
or a pharmaceutically acceptable salt thereof.

In a further embodiment, the present invention provides novel compounds having formula (Ia): 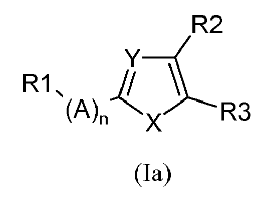 wherein:
X is selected from CH, substituted C, NH, substituted N, S, 0;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl or optionally substituted fused heterocyclyl;
n is 0 or 1; R¹ is H, OH, F, Cl, Br, I, NH₂, NO₂, CF₃, CH₃, OCH₃, -O(CH₂)₁-₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R^{a}, -(C₆H₄)(CH₂)₀₋₃R^{a}, -(C₆H₄)(R^{b})CH₂R^{a}, -(C₆H₄)CH₂ NHR^{a}, -(C₆H₄)C(=O)R^{a} -(C₆H₄)NHC(=O)R^{a}, -(C₆H₄)CH₂NH(CH₂)₁₋₃R^{a}R^{b}, - (C₆H₄)NHS0₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocycle, optionally substituted heterocyclyl, or optionally substituted C₁₋₆alkyl;
R^{a} and R^{b} are independently selected from: H, OH, OCH₃, CH₃, optionally substituted C₁₋₆alkyl, C₁₋₆alkoxy, NH₂, NHCH₃, N(CH₃)₂, (CH₂)₂N(CH₃)₂, CH₂C(CH₃)₂, CH₂CH₂NH, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms;
or a pharmaceutically acceptable salt thereof.

In another embodiment the present invention provides a compound having formula (Ia) wherein:
X is S;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH₃, OCH₃, -O(CH₂)₁₋₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R^{a}, -(C₆H₄)(CH₂)₀₋₃R^{a}, -(C₆H₄)(R^{b})CH₂R^{a}, -(C₆H₄)CH₂ NHR^{a}, -(C₆H₄)C(=O)R^{a} -(C₆H₄)NHC(=O)R^{a}, -(C₆H₄)CH₂NH(CH₂)₁₋₃R^{a}R^{b}, - (C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocyclyl, optionally substituted heterocyclyl, or optionally substituted C₁₋₆alkyl;
R^{a} is independently selected from: H, OH, OCH₃, CH₃, optionally substituted C₁₋₆alkyl, C₁₋₆alkoxy, NH₂, NHCH₃, N(CH₃)₂, (CH₂)₂N(CH₃)₂, CH₂C(CH₃)₂, CH₂CH₂NH, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is CH;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl,1, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH₃, OCH₃, -O(CH₂)₁₋₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁-₃R^{.}, -(C₆H₄)CH₂N(CH₃)(CH₂)₁₋₃R^{a}, -(C₆H₄)(CH₂)₀₋₃R^{a}, -(C₆H₄)(R^{b})CH₂R^{a}, -(C₆H₄)CH₂ NHR^{a}, -(C₆H₄)C(=O)R^{a} -(C₆H₄)NHC(=O)R^{a}, -(C₆H₄)CH₂NH(CH₂)₁₋₃R^{a}R^{b}-(C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocycle, optionally substituted heterocyclyl, or optionally substituted C₁₋₆alkyl;
R^{a} is independently selected from: H, OH, OCH₃, CH₃, optionally substituted C₁₋₆alkyl, C₁₋₆alkoxy, NH₂, NHCH₃, N(CH₃)₂, (CH₂)₂N(CH₃)₂, CH₂C(CH₃)₂, CH₂CH₂NHR^{a}, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is selected from CH, substituted C, NH, substituted N, S, O;
A is selected from optionally substituted aryl, optionally substituted phenyl, or optionally substituted heterocyclyl;
n is 0 or 1;
R¹ is H, OH, F, Cl, Br, I, NH₂, NO₂, CF₃, CH₃, OCH₃, -O(CH₂)₁₋₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R^{a}, -(C₆H₄)(CH₂)₀₋₃R^{a}, -(C₆H₄)(R^{b})CH₂R^{a}, -(C₆H₄)CH₂ NHR^{a}, -(C₆H₄)C(=O)R^{a} -(C₆H₄)NHC(=O)R^{a}, -(C₆H₄)CH₂NH(CH₂)₁₋₃R^{a}R^{b}, - (C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocycle, optionally substituted heterocyclyl, or optionally substituted C₁₋₆alkyl;
R^{a} is independently selected from: H, OH, OCH₃, CH₃, optionally substituted C₁_₆alkyl, C₁₋₆alkoxy, NH₂, NHCH₃, N(CH₃)₂, (CH₂)₂N(CH₃)₂, CH₂C(CH₃)₂, CH₂CH₂NHR^{a}, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH₃, OCH₃, -0(CH₂)₂N(CH₂CH₃)₂;
R²1s C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocycle, optionally substituted heterocyclyl, or optionally substituted C₁₋₆alkyl;
R^{a} is independently selected from: H, OH, OCH₃, CH₃, optionally substituted C₁_₆alkyl, C₁₋₆alkoxy, NH₂, NHCH₃, N(CH₃)₂, (CH₂)₂N(CH₃)₂, CH₂C(CH₃)₂, CH₂CH₂NHR^{a}, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH₃, OCH₃, -O(CH₂)₁-₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R^{a}, -(C₆H₄)(CH₂)₀₋₃R^{a}, - (C₆H₄)(R^{b})CH₂R^{a}, -(C₆H₄)CH₂ NHR^{a}, -(C₆H₄)C(=O)R^{a} -(C₆H₄)NHC(=O)R^{a}, - (C₆H₄)CH₂NH(CH₂)₁₋₃R^{a}R^{b}, -(C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a} ;
R³ is C(=O)NR^{a}R^{a}, SO₂N R^{a}R^{a}, NHC(=O)NR^{a}R⁴, C(=O)OR^{a};
R⁴ is selected from H, optionally substituted carbocycle, optionally substituted heterocyclyl, or optionally substituted C₁₋₆alkyl;
R^{a} is independently selected from: H, OH, OCH₃, CH₃, optionally substituted C₁_₆alkyl, C₁₋₆alkoxy, NH₂, NHCH₃, N(CH₃)₂, (CH₂)₂N(CH₃)₂, CH₂C(CH₃)₂, CH₂CH₂NHR^{a}, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH_{3,} OCH₃, -O(CH₂)₁₋₃N(CH₂CH₃)_{2,} - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R , -(C₆H₄)(CH₂)₀₋₃Ra, - (C₆H₄)(R^{b})CH₂R^{a}, -(C₆H₄)CH₂ NHR^{a}, -(C₆H₄)C(=O)R^{a} -(C₆H₄)NHC(=O)R^{a}, - (C₆H₄)CH₂NH(CH₂)₁₋₃R^{a}R^{b}, -(C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, NHC(=O)NR^{a}R⁴;
R⁴ is selected from H, optionally substituted carbocycle, optionally substituted heterocyclyl, or optionally substituted C₁₋₆alkyl;
R^{a} is independently selected from: H, OH, OCH₃, CH₃, optionally substituted C₁_₆alkyl, C₁₋₆alkoxy, NH₂, NHCH₃, N(CH₃)₂, (CH₂)₂N(CH₃)₂, CH₂C(CH₃)₂, CH₂CH₂NH, optionally substituted phenyl, optionally substituted cycloalkyl, optionally substituted 5 or 6 or 7 membered heterocyclyl having 1 or 2 oxygen or 1 or 2 nitrogen or 1 nitrogen and 1 oxygen or 1 nitrogen and 1 sulfur or 1 oxygen and 1 sulfur ring atoms.

In another embodiment the present invention provides a compound having formula (Ia) wherein:
X is selected from CH, substituted C, NH, substituted N, S, O;
Y is selected from CH, substituted C, NH, substituted N, S, O;
A is selected from optionally substituted alkyl, optionally substituted N-alkyl, optionally substituted O-alkyl, optionally substituted alkenyl, optionally substituted alkynyl, optionally substituted cycloalkyl, optionally substituted cycloalkenyl, optionally substituted aryl, optionally substituted phenyl, optionally substituted heterocyclyl, or optionally substituted fused heterocyclyl;
n is 0 or 1;
R¹ is H, OH, F, Cl, Br, I, NH₂, NO₂, CF₃, CH₃, OCH₃, -O(CH₂)₁-₃N(CH₂CH₃)₂, - C(=O)OR^{a}, -C(=O)NHNH₂, -NH(CH₂)₁₋₃R^{a}, -CH₂NH(CH₂)₁₋₃R^{a}, -NR^{a}C(=O)OR^{a}, -NR^{a}C(=O)R^{a}, -(C₆H₄)CH₂NH(CH₂)₁₋₃R^{a}, -(C₆H₄)CH₂N(CH₃)(CH₂)₁₋₃R^{a}, -(C₆H₄)(CH₂)₀₋₃Ra, - (C₆H₄)(R^{b})CH₂R^{a}, -(C₆H₄)CH₂ NHR^{a}, -(C₆H₄)C(=O)R^{a} -(C₆H₄)NHC(=O)R^{a}, - (C₆H₄)CH₂NH(CH₂)₁₋₃R^{a}R^{b}, -(C₆H₄)NHSO₂CH₃, -C(=O)NR^{a}R^{a}, optionally substituted alkyl, optionally substituted N-alkyl, optionally substituted alkenyl, optionally substituted alkynyl, optionally substituted cycloalkyl, optionally substituted cycloalkenyl, optionally substituted aryl, optionally substituted alkoxy, optionally substituted phenyl, optionally substituted heterocyclyl, or optionally substituted fused heterocyclyl;
R² is C(=O)NR^{a}R^{a}, SO₂N R^{a}R^{a}, NHC(=O)NR^{a}R⁴, C(=O)OR^{a}
R³ is C(=O)NR^{a}R^{a}, SO₂N R^{a}R^{a}, NHC(=O)NR^{a}R⁴, C(=O)OR^{a}
R⁴ is selected from H, optionally substituted carbocycle, optionally substituted heterocyclyl, or optionally substituted C₁₋₆alkyl;
R^{a} is independently selected from: H, or optionally substituted 5 or 6 or 7 membered heterocyclyl having 1 or 2 nitrogen ring atoms.

In another embodiment the present invention provides a compound having formula (Ia) wherein:
X is S;
Y is CH;
A is phenyl;
n is 1;
R¹ is H;
R² is C(=O)NR^{a}R^{a};
R³ is NHC(=O)NH₂;
R^{a} is independently selected from: H, or an optionally substituted 6 or 7 membered heterocyclyl having 1 nitrogen ring atom.
Additional embodiments of the invention are as follows. These additional embodiments relate to compounds of formula (I), (II) and (Ia) and it is to be understood where compounds of formula (I) and/or formula (II) are referred to, this also applies in the alternative to compounds of formula (Ia).
In a further embodiment of the invention, particularly useful compounds of the invention are any one of the Examples or a pharmaceutically acceptable salt thereof.
In another embodiment, the invention is directed to Examples 13, 15, 24, 30, 34, 47, 48, 104, 107, 110, 114, 126, 129, 160, 173, and 176.

In another embodiment the present invention provides the following compounds of formula (I):
5-Phenyl-2-ureido-thiophene-3-carboxylic acid (S)-azepan-3-ylamide;
5-Phenyl-2-ureido-thiophene-3-carboxylic acid (S)-piperidin-3-ylamide;
5-Phenyl-2-(3-pyrazin-2-yl-ureido)-thiophene-3-carboxylic acid (S)-azepan-3-ylamide;
5-Phenyl-2-(3-pyrazin-2-yl-ureido)-thiophene-3-carboxylic acid (S)-piperidin-3-ylamide;
5-(1H-Pyrazol-4-yl)-2-ureido-thiophene-3-carboxylic acid (S)-piperidin-3-ylamide;
5-(1H-Pyrazol-4-yl)-2-ureido-thiophene-3-carboxylic acid (S)-azepan-3-ylamide;
5-(1H-Pyrazol-4-yl)-2-(3-pyrazin-2-yl-ureido)-thiophene-3-carboxylic acid (S)-piperidin-3-ylamide;
5-(1H-Pyrazol-4-yl)-2-(3-pyrazin-2-yl-ureido)-thiophene-3-carboxylic acid (S)-azepan-3-ylamide;
5-(1H-Pyrrol-3-yl)-2-ureido-thiophene-3-carboxylic acid (S)-piperidin-3-ylamide;
5-(1H-Pyrrol-3-yl)-2-ureido-thiophene-3-carboxylic acid (S)-azepan-3-ylamide;
5-(1H-Pyrrol-3-yl)-2-(3-pyrazin-2-yl-ureido)-thiophene-3-carboxylic acid (S)-piperidin-3-ylamide;
5-(1H-Pyrrol-3-yl)-2-(3-pyrazin-2-yl-ureido)-thiophene-3-carboxylic acid (S)-azepan-3-ylamide;
5-(1H-Pyrrol-2-yl)-2-ureido-thiophene-3-carboxylic acid (S)-piperidin-3-ylamide;
5-(1H-Pyrrol-2-yl)-2-ureido-thiophene-3-carboxylic acid (S)-azepan-3-ylamide;
5-(1H-Pyrrol-2-yl)-2-(3-pyrazin-2-yl-ureido)-thiophene-3-carboxylic acid (S)-piperidin-3-ylamide;
5-(1H-Pyrrol-2-yl)-2-(3-pyrazin-2-yl-ureido)-thiophene-3-carboxylic acid (S)-azepan-3-ylamide;
5-Pyridin-2-yl-2-ureido-thiophene-3-carboxylic acid (S)-piperidin-3-ylamide;
5-Pyridin-2-yl-2-ureido-thiophene-3-carboxylic acid (S)-azepan-3-ylamide;
5-Pyridin-2-yl-2-(3-pyrazin-2-yl-ureido)-thiophene-3-carboxylic acid (S)-piperidin-3-ylamide;
5-Pyridin-2-yl-2-(3-pyrazin-2-yl-ureido)-thiophene-3-carboxylic acid (S)-azepan-3-ylamide;
5-Pyridin-3-yl-2-ureido-thiophene-3-carboxylic acid (S)-piperidin-3-ylamide;
5-Pyridin-3-yl-2-ureido-thiophene-3-carboxylic acid (S)-azepan-3-ylamide;
5-Pyridin-3-yl-2-(3-pyrazin-2-yl-ureido)- thiophene-3-carboxylic acid (S)-piperidin-3-ylamide;
5-Pyridin-3-yl-2-(3-pyrazin-2-yl-ureido)- thiophene-3-carboxylic acid (S)-azepan-3-ylamide;
5-Pyridin-4-yl-2-ureido-thiophene-3-carboxylic acid (S)-piperidin-3-ylamide;
5-Pyridin-4-yl-2-ureido-thiophene-3-carboxylic acid (S)-azepan-3-ylamide;
5-Pyridin-4-yl-2-(3-pyrazin-2-yl-ureido)- thiophene-3-carboxylic acid (S)-piperidin-3-ylamide;
5-Pyridin-4-yl-2-(3-pyrazin-2-yl-ureido)- thiophene-3-carboxylic acid (S)-azepan-3-ylamide;
5-(4-Fluoro-phenyl)-2-ureido-thiophene-3-carboxylic acid (S)-piperidin-3-ylamide;
5-(4-Fluoro-phenyl)-2-ureido-thiophene-3-carboxylic acid (S)-azepan-3-ylamide;
5-(4-Fluoro-phenyl)-2-(3-pyrazin-2-yl-ureido)-thiophene-3-carboxylic acid (S)-piperidin-3-ylamide;
5-(4-Fluoro-phenyl)-2-(3-pyrazin-2-yl-ureido)-thiophene-3-carboxylic acid (S)-azepan-3-ylamide;
5-(4-Chloro-phenyl)-2-ureido-thiophene-3-carboxylic acid (S)-piperidin-3-ylamide;
5-(4-Chloro-phenyl)-2-ureido-thiophene-3-carboxylic acid (S)-azepan-3-ylamide;
5-(4-Chloro-phenyl)-2-(3-pyrazin-2-yl-ureido)-thiophene-3-carboxylic acid (S)-piperidin-3-ylamide;
5-(4-Chloro-phenyl)-2-(3-pyrazin-2-yl-ureido)-thiophene-3-carboxylic acid (S)-azepan-3-ylamide;
5-(3-Fluoro-phenyl)-2-ureido-thiophene-3-carboxylic acid (S)-piperidin-3-ylamide;
5-(3-Fluoro-phenyl)-2-ureido-thiophene-3-carboxylic acid (S)-azepan-3-ylamide;
5-(3-Fluoro-phenyl)-2-(3-pyrazin-2-yl-ureido)-thiophene-3-carboxylic acid (S)-piperidin-3-ylamide;
5-(3-Fluoro-phenyl)-2-(3-pyrazin-2-yl-ureido)-thiophene-3-carboxylic acid (S)-azepan-3-ylamide;
5-(3-Chloro-phenyl)-2-ureido-thiophene-3-carboxylic acid (S)-piperidin-3-ylamide;
5-(3-Chloro-phenyl)-2-ureido-thiophene-3-carboxylic acid (S)-azepan-3-ylamide;
5-(3-Chloro-phenyl)-2-(3-pyrazin-2-yl-ureido)-thiophene-3-carboxylic acid (S)-piperidin-3-ylamide;
5-(3-Chloro-phenyl)-2-(3-pyrazin-2-yl-ureido)-thiophene-3-carboxylic acid (S)-azepan-3-ylamide;
5-(3,4-Difluoro-phenyl)-2-ureido-thiophene-3-carboxylic acid (S)-piperidin-3-ylamide;
5-(3,4-Difluoro-phenyl)-2-ureido-thiophene-3-carboxylic acid (S)-azepan-3-ylamide;
5-(3,4-Difluoro-phenyl)-2-(3-pyrazin-2-yl-ureido)-thiophene-3-carboxylic acid (S)-piperidin-3-ylamide;
5-(3,4-Difluoro-phenyl)-2-(3-pyrazin-2-yl-ureido)-thiophene-3-carboxylic acid (S)-azepan-3-ylamide;
5-(2,4-Difluoro-phenyl)-2-ureido-thiophene-3-carboxylic acid (S)-piperidin-3-ylamide;
5-(2,4-Difluoro-phenyl)-2-ureido-thiophene-3-carboxylic acid (S)-azepan-3-ylamide;
5-(2,4-Difluoro-phenyl)-2-(3-pyrazin-2-yl-ureido)-thiophene-3-carboxylic acid (S)-piperidin-3-ylamide;
5-(2,4-Difluoro-phenyl)-2-(3-pyrazin-2-yl-ureido)-thiophene-3-carboxylic acid (S)-azepan-3-ylamide;
5-(3-Chloro-4-fluoro-phenyl)-2-ureido-thiophene-3-carboxylic acid (S)-piperidin-3-ylamide;
5-(3-Chloro-4-fluoro-phenyl)-2-ureido-thiophene-3-carboxylic acid (S)-azepan-3-ylamide;
5-(3-Chloro-4-fluoro-phenyl)-2-(3-pyrazin-2-yl-ureido)-thiophene-3-carboxylic acid (S)-piperidin-3-ylamide;
5-(3-Chloro-4-fluoro-phenyl)-2-(3-pyrazin-2-yl-ureido)-thiophene-3-carboxylic acid (S)-azepan-3-ylamide;
5-Pyrimidin-5-yl-2-ureido-thiophene-3-carboxylic acid (S)-piperidin-3-ylamide;
5-Pyrimidin-5-yl-2-ureido-thiophene-3-carboxylic acid (S)-azepan-3-ylamide;
5-Pyrimidin-5-yl-2-(3-pyrazin-2-yl-ureido)-thiophene-3-carboxylic acid (S)-piperidin-3-ylamide;
5-Pyrimidin-5-yl-2-(3-pyrazin-2-yl-ureido)-thiophene-3-carboxylic acid (S)-azepan-3-ylamide;
5-[(Aminocarbonyl)amino]-2-phenyl-*N*-[(3*S*)-piperidin-3-yl]-1,3-thiazole-4-carboxamide;
*N*-[(3*S*)-piperidin-3-yl]-2-phenyl-5-{[(pyrimidin-4-ylamino)carbonyl]-amino-1,3-thiazole-4-carboxamide;
5-[(Ammocarbonyl)ammo]-*N*-[(3*S*)-azepan-3-yl]-2-phenyl-1,3-thiazole-4-carboxamide;
*N*-[(3*S*)-azepan-3-yl]-2-phenyl-5-{[(pyrimidin-4-ylamino)carbonyl]camino}-1,3-thiazole-4-carboxamide;
3-Ureido-thiophene-2-carboxylic acid (*S*)-azepan-3-ylamide;
5-Phenyl-3-ureido-thiophene-2-carboxylic acid (*S*)-azepan-3-ylamide;
5-(4-Chloro-phenyl)-3-ureido-thiophene-2-carboxylic acid (*S*)-azepan-3-ylamide:
5-(4-tert-Butyl-phenyl)-3-ureido-thiophene-2-carboxylic acid (*S*)-azepan-3-ylamide;
5-(4-iso-Butyl-phenyl)-3-ureido-thiophene-2-carboxylic acid (*S*)-azepan-3-ylamide;
5-tert-Butyl-phenyl-3-ureido-thiophene-2-carboxylic acid (*S*)-azepan-3-ylamide;
5-(4-Chloro-phenyl)-3-ureido-thiophene-2-carboxylic acid (*S)*-piperidin-3-ylamide;
5-(4-Fluoro)-3-ureido-thiophene-2-carboxylic acid (*S*)-piperidin-3-ylamide;
5-[4-(2-Thienyl)]-3-ureido-thiophene-2-carboxylic acid (*S*)-azepan-3-ylamide;
5-Benzyl-2-ureido-thiophene-3-carboxylic acid (S)-azepan-3-ylamide;
5-Methyl-2-ureido-thiophene-3-carboxylic acid (S)-azepan-3-ylamide;
5-Ethyl-2-ureido-thiophene-3-carboxylic acid (S)-azepan-3-ylamide;
5-iso-Propyl-2-ureido-thiophene-3-carboxylic acid (S)-azepan-3-ylamide;
2-Ureido-thiophene-3-carboxylic acid (*S*)-azepan-3-ylamide;
5-Bromo-2-ureido-thiophene-3-carboxylic acid (*S*)-azepan-3-ylamide;
5-Bromo-2-ureido-thiophene-3-carboxylic acid (*S*)-piperidin-3-ylamide;
2-Phenyl-5-ureido-thiazole-4-carboxylic acid (*S*)-azepan-3-ylamide;
2-((4-Methyl)-phenyl)-5-ureido-thiazole-4-carboxylic acid (*S*)-piperidin-3-ylamide;
2-Phenyl-5-ureido-thiazole-4-carboxylic acid (*S*)-piperidin-3-ylamide;
2-Methyl-5-ureido-thiazole-4-carboxylic acid (*S*)-azepan-3-ylamide;
2-(4-Fluoro-phenyl)-5-ureido-thiazole-4-carboxylic acid (S)-piperidin-3-ylamide;
2-(4-Chloro-phenyl)-5-ureido-thiazole-4-carboxylic acid (S)-piperidin-3-ylamide;
2-(4-Methoxy-phenyl)-5-ureido-thiazole-4-carboxylic acid (S)-piperidin-3-ylamide;
2-(3-Cyano-phenyl)-5-ureido-thiazole-4-carboxylic acid (S)-piperidin-3-ylamide;
2-Morpholin-4-yl-4-ureido-thiazole-5-carboxylic acid (S)-piperidin-3-ylamide;
2-(4-Methoxy-phenylamino)-4-ureido-thiazole-5-carboxylic acid (S)-piperidin-3-ylamide;
2-Methylsulfanyl-4-ureido-thiazole-5-carboxylic acid (S)-piperidin-3-ylamide;
2-Methanesulfinyl-4-ureido-thiazole-5-carboxylic acid (S)-piperidin-3-ylamide;
2-Methanesulfonyl-4-ureido-thiazole-5-carboxylic acid (S)-piperidin-3-ylamide;
2-Phenyl-4-ureido-thiazole-5-carboxylic acid (S)-piperidin-3-ylamide;
2-Phenyl-5-ureido-oxazole-4-carboxylic acid (S)-piperidin-3-ylamide;
2-Methyl-5-ureido-oxazole-4-carboxylic acid (S)-piperidin-3-ylamide;
5-Ethynyl-2-ureido-thiophene-3-carboxylic acid (S)-piperidin-3-ylamide;
5-Prop-1-ynyl-2-ureido-thiophene-3-carboxylic acid (S)-piperidin-3-ylamide;
5-(3-Methoxy-prop-1-ynyl)-2-ureido-thiophene-3-carboxylic acid (S)-piperidin-3-ylamide;
5-Phenylethynyl-2-ureido-thiophene-3-carboxylic acid (S)-piperidin-3-ylamide.

In another embodiment the present invention provides a compound having formula (I) wherein one or more of the atoms is a radioisotope of the same element.

In another embodiment the present invention provides a compound of formula (I) or a pharmaceutically acceptable salt thereof for use in the treatment or prophylaxis of disorders associated with cancer.

In another embodiment the present invention provides a compound of formula (I) or a pharmaceutically acceptable salt thereof for the use in treatment or prophylaxis of neoplastic disease such as cervical cancer, cancer of the head and neck, carcinoma of the breast, ovary, lung(non small cell), pancreas, colon, prostate or other tissues, as well as leukemias and lymphomas, tumors of the central and peripheral nervous system, and other tumor types such as melanoma, fibrosarcoma and osteosarcoma.

In another embodiment the present invention provides a compound of formula (I) or a pharmaceutically acceptable salt thereof for use in the treatment or prophylaxis of proliferative diseases including autoimmune, inflammatory, neurological, and cardiovascular diseases.

In another embodiment the present invention provides a method of limiting cell proliferation in a human or animal comprising administering to said human or animal a therapeutically effective amount of a compound of formula (I) or a pharmaceutically acceptable salt thereof.

In a further embodiment the present invention provides a method of inhibiting CHK1 kinase comprising administering to an animal or human in need of said inhibiting a therapeutically effective amount of a compound of formula (I) or a pharmaceutically acceptable salt thereof.

In another embodiment the present invention provides a method of treatment of a human or animal suffering from cancer comprising administering to said human or animal a therapeutically effective amount of a compound of formula (I) or a pharmaceutically acceptable salt thereof.

In further embodiment the present invention provides a method of prophylaxis treatment of cancer comprising administering to a human or animal in need of such treatment a therapeutically effective amount of a compound of formula (I) or a pharmaceutically acceptable salt thereof.

In another embodiment the present invention provides a method of treatment of a human or animal suffering from a neoplastic disease such as cervical cancer , cancer of the head and neck, carcinoma of the breast, ovary, lung (non small cell), pancreas, colon, prostate or other tissues, as well as leukemias and lymphomas, tumors of the central and peripheral nervous system, and other tumor types such as melanomasarcomas including fibrosarcoma and osteosarcoma, malignant brain tumors, comprising administering to said human or animal a therapeutically effective amount of a compound of formula (I) or a pharmaceutically acceptable salt thereof.

In another embodiment the present invention provides a method of treatment of a human or animal suffering from a proliferative disease such as autoimmune, inflammatory, neurological, and cardiovascular diseases comprising administering to said human or animal a therapeutically effective amount of a compound of formula (I) or a pharmaceutically acceptable salt thereof.

One embodiment the of present invention provides a method of treating cancer by administering to a human or animal a compound of formula (I) or a pharmaceutically acceptable salt thereof and an anti-tumor agent.

One embodiment of the present invention provides a method of treating cancer by administering to a human or animal a compound of formula (I) or a pharmaceutically acceptable salt thereof and a DNA damaging agent.

One embodiment of the present invention provides a method for the treatment of infections associated with cancer comprising administering to a human or animal in need of such treatment a therapeutically effective amount of a compound of formula (I) or a pharmaceutically acceptable salt thereof.

A further embodiment of the present invention provides a method for the prophylaxis treatment of infections associated with cancer comprising administering to a human or animal in need of such treatment a therapeutically effective amount of a compound of formula (I) or a pharmaceutically acceptable salt thereof.

Another embodiment of the present invention provides a pharmaceutical composition comprising a compound of formula (I) or a pharmaceutically acceptable salt thereof together with at least one pharmaceutically acceptable carrier, diluent or excipent.

In another embodiment the present invention provides the use of a compound of formula (I) or a pharmaceutically acceptable salt thereof in the preparation of a medicament.

In another embodiment the present invention provides the use of a compound of formula (I) or a pharmaceutically acceptable salt thereof in the preparation of a medicament for the treatment or prophylaxis of cancer.

In another embodiment the present invention provides the use of a compound of formula (I) or a pharmaceutically acceptable salt thereof in the preparation of a medicament for the treatment or prophylaxis of neoplastic disease such as carcinoma of the breast, ovary, lung, colon, prostate or other tissues, as well as leukemias and lymphomas including CLL and CML, tumors of the central and peripheral nervous system, and other tumor types such as melanoma, fibrosarcoma and osteosarcoma.

In still another embodiment the present invention provides the use of a compound of formula (I) or a pharmaceutically acceptable salt thereof in the preparation of a medicament for the treatment or prophylaxis of proliferative diseases including autoimmune, inflammatory, neurological, and cardiovascular diseases.

In another embodiment the present invention provides the use of a compound of formula (I) or a pharmaceutically acceptable salt thereof in the preparation of a medicament for use in the inhibition of CHK1 kinase activity.

In another embodiment the present invention provides the use of a compound of formula (I) or a pharmaceutically acceptable salt thereof in the preparation of a medicament for use in limiting cell proliferation.

### Definitions

The definitions set forth in this section are intended to clarify terms used throughout this application. In this section, the definition applies to compounds of formula (I) and (II) and compounds of formula (1a) unless otherwise stated. The term "herein" means the entire application.

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names and rules on naming chemical structures.

The term "Cₘ₋ₙ" or "Cₘ₋ₙ group" used alone or as a prefix, refers to any group having m to n carbon atoms.

As used in this application, the term "optionally substituted," means that substitution is optional and therefore it is possible for the designated atom to be unsubstituted. In the event a substitution is desired then such substitution means that any number of hydrogens on the designated atom is replaced with a selection from the indicated group, provided that the normal valency of the designated atom is not exceeded, and that the substitution results in a stable compound. For example when a substituent is keto (i.e., =O), then 2 hydrogens on the atom are replaced. When a group is indicated to be "optionally substituted" or "substituted" unless otherwise expressly stated examples of suitable substituents include the following: halogen, nitro, amino, cyano , trifluoromethyl, methyl, ethyl, alkyl, alkenyl, alkynyl, haloalkyl, alkoxy, hydroxy, alkylhydroxy, carbonyl, keto, -CH(OH)CH₃, -CH₂NH-alkyl-OH, alkyl-(OH)CH₃, -Oalkyl, -OCOalkyl, -NHCHO, -N-(alkyl)-CHO, -NH-CO-amino, -N-(alkyl)-CO-amino, -NH-COalkyl, -N-(alkyl)-COalkyl, -carboxy, -amidino, -CO-amino, -CO-alkyl, - CO₂alkyl, mercapto, -Salkyl, -SO(alkyl), -SO₂(alkyl), -SO₂-amino, -alkylsulfonylamino, phenyl, cycloalkyl, heterocyclic and heteroaryl, -alkly-NH-cycloalkyl, -alkyl-NH-optionally substituted heterocyclyl, -alkyl-NH-alkyl-OH, -C(=O)OC(CH₃)₃, -N(CH₃)₂, -alkyl-NH-alkyl-optionally substituted heterocyclyl, alkyl-aryl, alkyl-polycyclyl, alkyl-amino, alkyl-hydroxy, -CH₂NH-alkyl-heterocyclyl, -CH₂NHCH2CH(CH₃)₂ If the group to be substituted is a ring, the optional substituents could also be selected from:vicinal -O(alkyl)O-, vicinal -OC(haloalkyl)O-, vicinal -CH₂O(alkyl)O-, vicinal -S(alkyl)S- and -O(alkyl)S-. Each of these substituents can, themselves, be further substituted. Suitable examples of such further substitution include any of the foregoing suitable substituents.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" general includes both saturated alkyl and unsaturated alkyl.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkylene" used alone or as suffix or prefix, refers to divalent straight or branched chain hydrocarbon radicals comprising 1 to about 12 carbon atoms, which serves to links two structures together.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms. When cycloalkyl contains more than one ring, the rings may be fused or unfused and include bicyclo radicals. Fused rings generally refer to at least two rings sharing two atoms therebetween.

The term "cycloalkenyl" used alone or as suffix or prefix, refers to a monovalent ring-containing hydrocarbon radical having at least one carbon-carbon double bond and comprising at least 3 up to about 12 carbon atoms. When cycloalkenyl contains more than one ring, the rings may be fused or unfused and include bicyclo radicals.

The term "aryl" used alone or as suffix or prefix, refers to a hydrocarbon radical having one or more polyunsaturated carbon rings having aromatic character, (e.g., 4n + 2 delocalized electrons) and comprising 6 up to about 14 carbon atoms, wherein the radical is located on a carbon of the aromatic ring. Exemplary aryl includes phenyl, naphthyl, and indenyl.

The term "alkoxy" used alone or as a suffix or prefix, refers to radicals of the general formula -O-R, wherein -R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "carbocyclyl" is intended to include both alicyclic and aromatic ring structures wherein the closed ring is made of carbon atoms. These may include fused or bridged polycyclic systems. Carbocyclyls may have from 3 to 10 carbon atoms in their ring structure, and often have 3, 4, 5, 6 and 7 carbon atoms in the ring structure. For example, "C₃₋₇ carbocyclyl" denotes such groups as cyclopropyl, cyclobutyl, cyclopentyl, cyclohexyl, cycloheptyl, cyclopentadiene or phenyl.

A "heterocyclyl" " is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)- and a ring sulphur atom may be optionally oxidised to form the S-oxides. Heterocyclyl may contain more than one ring. When a heterocyclyl contains more than one ring, the rings may be fused or unfused. Fused rings generally refer to at least two rings sharing two atoms therebetween. Heterocyclyl may have aromatic character or may not have aromatic character Examples of heterocyclyls include, but are not limited to, 1H-indazolyl, 2-pyrrolidonyl, 2H, 6H-1, 5,2-dithiazinyl, 2H-pyrrolyl, 3H-indolyl, 4-piperidonyl, 4aH-carbazolyl, 4H-quinolizinyl, 6H-1, 2,5-thiadiazinyl, acridinyl, azepanyl, azetidinyl, aziridinyl, azocinyl, benzimidazolyl, benzofuranyl, benzofuranyl, benzothiofuranyl, benzothiophenyl, , benzodioxolyl, benzoxazolyl, benzthiophenyl, benzthiazolyl, benzotriazolyl, benzotetrazolyl, benzisoxazolyl, benzthiazole, benzisothiazolyl, benzimidazolyls, benzimidazalonyl, carbazolyl, 4aH-carbazolyl, b-carbolinyl, chromanyl, chromenyl, cinnolinyl, decahydroquinolinyl, 2H,6H-1,5,2-dithiazinyl, dioxolanyl, furyl, 2,3-dihydrofuranyl, 2,5-dihydrofuranyl, dihydrofuro[2,3-b]tetrahydrofuranyl, furanyl, furazanyl, homopiperidinyl, imidazolyl, imidazolidinyl, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxiranyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, piperidinyl, pteridinyl, piperidonyl, 4-piperidonyl, purinyl, pyranyl, pyrrolidinyl, pyrrolinyl, pyrrolidinyl, pyrazinyl, pyrazolidinyl, pyrazolinyl, pyrazolyl, pyridazinyl, pyridooxazolyl, pyridoimidazolyl, pyridothiazolyl, pyridinyl, N-oxide-pyridinyl, pyridyl, pyrimidinyl, pyrrolidinyl, pyrrolinyl, pyrrolyl, pyridinyl, quinazolinyl, quinolinyl, 4H-quinolizinyl, quinoxalinyl, quinuclidinyl, carbolinyl, tetrahydrofuranyl, tetrahydroisoquinolinyl, thiophanyl, thiotetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, thiiranyl, triazinyl, 1,2,3-triazolyl, 1,2,4-triazolyl, 1,2,5-triazolyl, 1,3,4-triazolyl, and xanthenyl.

The terms "seven-membered", "six-membered" and "five-membered" used as prefix refers to groups having a ring that contains, respectively, seven, six, and five ring atoms.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "amine" or "amino" used alone or as a suffix or prefix, refers to radicals of the general formula -NRR', wherein R and R' are independently selected from hydrogen or a hydrocarbon radical.

The term halogen includes fluorine, chlorine, bromine and iodine. "Halogenated," used as a prefix of a group, means one or more hydrogens on the group are replaced with one or more halogens.

"RT" or "rt" means room temperature.

When any variable (e.g., R¹, R⁴, R^{a}, R^{e} etc.) occurs more than one time in any constituent or formula for a compound, its definition at each occurrence is independent of its definition at every other occurrence. Thus, for example, if a group is shown to be substituted with 0-3 R¹, then said group may optionally be substituted with 0, 1, 2 or 3 R¹ groups and R¹ at each occurrence is selected independently from the definition of R¹ Also, combinations of substituents and/or variables are permissible only if such combinations result in stable compounds.

A variety of compounds in the present invention may exist in particular geometric or stereoisomeric forms. The present invention takes into account all such compounds, including cis- and trans isomers, R- and S- enantiomers, diastereomers, (D)-isomers, (L)-isomers, the racemic mixtures thereof, and other mixtures thereof, as being covered within the scope of this invention. Additional asymmetric carbon atoms may be present in a substituent such as an alkyl group. All such isomers, as well as mixtures thereof, are intended to be included in this invention. The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. When required, separation of the racemic material can be achieved by methods known in the art. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

When a circle is shown within a ring structure, it indicates that the ring system is aromatic.

As used herein, the phrase "protecting group" means temporary substituents which protect a potentially reactive functional group from undesired chemical transformations. Examples of such protecting groups include esters of carboxylic acids, silyl ethers of alcohols, and acetals and ketals of aldehydes and ketones respectively. The field of protecting group chemistry has been reviewed (Greene, T.W.; Wuts, P.G.M. Protective Groups in Organic Synthesis, 3rd ed.; Wiley: New York, 1999).

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, maleic, tartaric, citric, ascorbic, palmitic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, the disclosure of which is hereby incorporated by reference.

"Prodrugs" are intended to include any covalently bonded carriers that release the active parent drug according to formula (I) in vivo when such prodrug is administered to a mammalian subject. Prodrugs of a compound of formula (I) are prepared by modifying functional groups present in the compound in such a way that the modifications are cleaved, either in routine manipulation or in vivo, to the parent compound. Prodrugs include compounds of formula (I) wherein a hydroxy, amino, or sulfhydryl group is bonded to any group that, when the prodrug or compound of formula (I) is administered to a mammalian subject, cleaves to form a free hydroxyl, free amino, or free sulfhydryl group, respectively. Examples of prodrugs include, but are not limited to, acetate, formate and benzoate derivatives of alcohol and amine functional groups in the compounds of formula (I), and the like.

"Stable compound" and "stable structure" are meant to indicate a compound that is sufficiently robust to survive isolation to a useful degree of purity from a reaction mixture, and formulation into an efficacious therapeutic agent.

### Combinations

The anti-cancer treatment defined herein may be applied as a sole therapy or may involve, in addition to the compound of the invention, conventional surgery and/or radiotherapy and/or chemotherapy. Such chemotherapy may include one or more of the following categories of antitumour agents:
(i) antiproliferative/antineoplastic drugs and combinations thereof, as used in medical oncology, such as alkylating agents or platinating (for example cis-platin, carboplatin, oxaliplatin, cyclophosphamide, nitrogen mustard, melphalan, chlorambucil, busulphan and nitrosoureas); antimetabolites (for example gemcitabine and fludarabine, as well as antifolates such as fluoropyrimidines like 5-fluorouracil and tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan, irinotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbbl antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of infection is an amount sufficient to symptomatically relieve in a warm-blooded animal, particularly a human the symptoms of infection, to slow the progression of infection, or to reduce in patients with symptoms of infection the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula (I) or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solution in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

Compounds of formula (I) have been shown to inhibit checkpoint kinase activity in vitro. Inhibitors of checkpoint kinase have been shown to allow cells to progress inappropriately to the metaphase of mitosis leading to apoptosis of effected cells, and to therefore have anti-proliferative effects. Therefore it is believed that the compounds of formula (I) and their pharmaceutically acceptable salts may be used for the treatment of neoplastic disease as described above. In addition, compounds of formula (I) and their pharmaceutically acceptable salts are also expected to be useful for the treatment of other proliferative diseases. It is expected that the compounds of formula (I) would most likely be used in combination with a broad range of DNA damaging agents but could also be used as a single agent.

Generally, the compounds of formula (I) have been identified in one or both assays described below as having an IC₅₀ or EC₅₀ value of 100 micromolar or less. For example, the compound of example 263 has an IC₅₀ value of 0.12 nM and the compound of example 97 has an IC₅₀ value of 0.14 nM.

Checkpoint Kinase 1 Assay: This in vitro assay measures the inhibition of CHK1 kinase by compounds. The kinase domain is expressed in baculovirus and purified by the GST tag. Purified protein and biotinylated peptide substrate (Cdc25C) is then used in a 384 well automated Scintillation Proximity Assay (SPA). Specifically, peptide, enzyme and reaction buffer are mixed and aliquoted into a 384 well plate containing dilution series of compounds and controls. Cold and hot ATP are then added to initiate the reaction. After 2 hours, a SPA bead slurry, CsC12 and EDTA are added to stop the reaction and capture the biotinylated peptide. Plates are then counted on a Topcount. Data is analyzed and IC₅₀s determined for individual compounds.

Abrogation Assay: This cellular assay measures the ability of CHK1 inhibitors to abrogate the DNA-damage induced G2/M checkpoint. Compounds active against the enzyme (< 2 uM) are tested in the cellular assay. Briefly HT29 cells (colon cancer cell line, p53 null) are plated in 96 well plates on day 1. The following day, cells are treated with camptothecin for 2 hours to induce DNA damage. After 2 hours, camptothecin is removed and cells are treated for an additional 18 hours with test compound and nocodazole, a spindle poison that traps in cells in mitosis that abrogate the checkpoint. Cells are then fixed with formaldehyde, stained for the presence of phosphohistone H3, a specific marker for mitosis and labeled with Hoechst dye so that cell number can be measured. Plates are scanned using the Mitotic Index protocol on the Array Scan (Cellomics). As a positive control for abrogation, 4 mM caffeine is used. Compounds are tested in a 12-point dose response in triplicate. Data is analyzed and EC₅₀s determined for individual compounds.

### Synthesis

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Such methods include, but are not limited to, those described below. All references cited herein are hereby incorporated in their entirety by reference.

The novel compounds of this invention may be prepared using the reactions and techniques described herein. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents, which are not compatible with the reaction conditions, will be readily apparent to one skilled in the art and alternate methods must then be used.

The starting materials for the examples contained herein are either commercially available or are readily prepared by standard methods from known materials. For example the following reactions are illustrations but not limitations of the preparation of some of the starting materials and examples used herein.

General procedures for synthesizing the compounds of the invention are as follows:

Compounds of Formula (I) can be synthesized from the general synthetic methods described below in Schemes 1-13. The first general method shown in both Scheme 1 and 1a utilize a Weinreb amide formation by reaction of either the trichloroacetyl-protected or free urea (generated by reaction of the protected urea with ammonia in methanol) with an amino aluminate organometallic complex. The urea can also be formed in one step by reaction of the unprotected amine with chlorosulfonyl isocyanate.

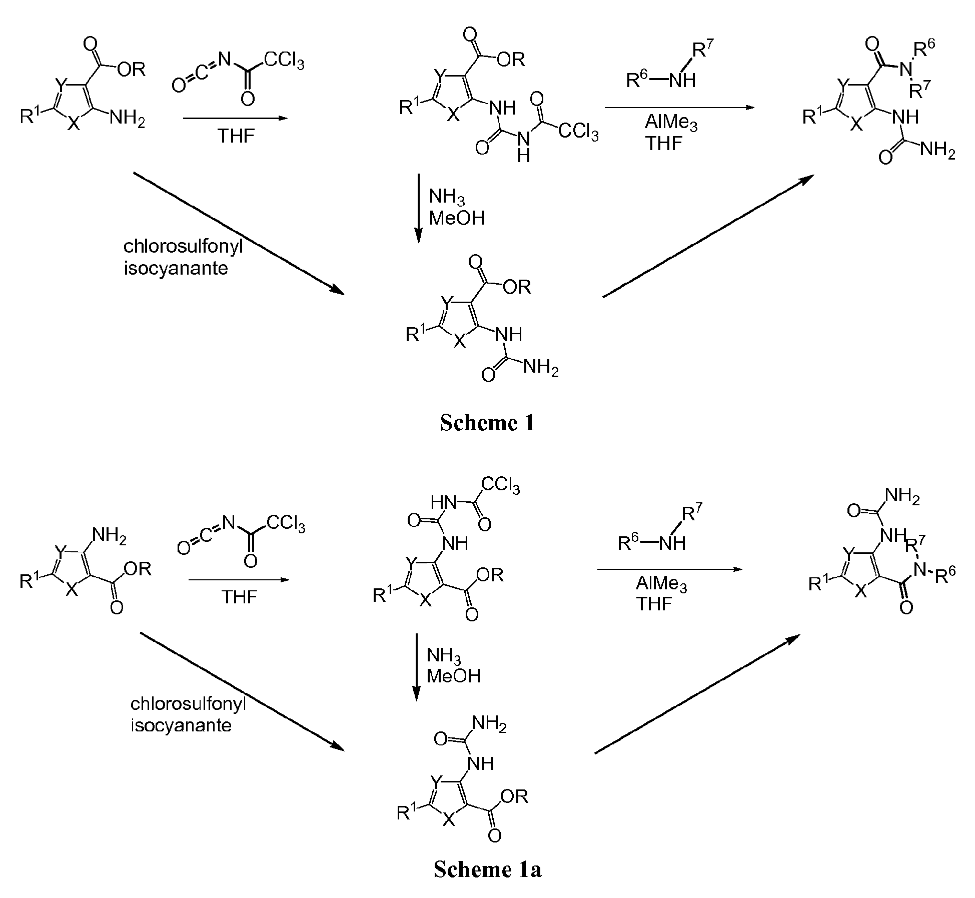

In the case of compounds of formula (I) where R³ is NHC(O)NHR⁴; R² is C(=O)R⁶R⁷; and X=S;where Scheme 1) the starting materials are either purchased commercially or produced by a Gewald reaction of cyanoacetates with various benzaldehydes and elemental sulfur under basic conditions shown below in Scheme 2. If not commercially available, the corresponding aldehydes can be synthesized by DIBAL reduction of readily available esters.

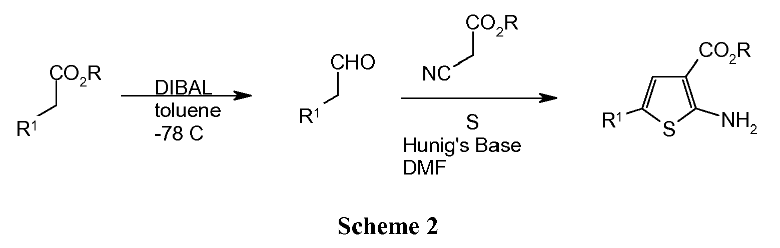

In the case of compounds of formula (I) where (R² is NHC(O)NHR⁴; R³ is C(=O)R⁶R⁷;and X=S;) the 3-amino thiophene ester (Scheme 1a) is either purchased commercially or produced by the synthesis outlined below in Scheme 3. Reaction of DMF and POCl₃ and hydroxylamine yields the acrylonitrile, which is cyclized to the abovementioned 3-amino thiophene ester by reaction with a thioglycolate.

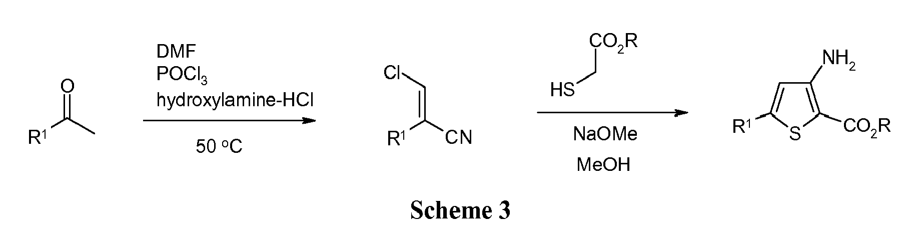

In the case where (X=S, Y=N) or (X=O, Y=N), the amino esters can be generated through the three-step sequence shown below (Scheme 4) involving reductive removal of water from the commercially available oxime, followed by acylation and finally cyclization to the 2-amino-oxazole by reaction of the nitrile with HCl in dioxane or to the corresponding 2-amino-thiazole using Lawesson's Reagent.

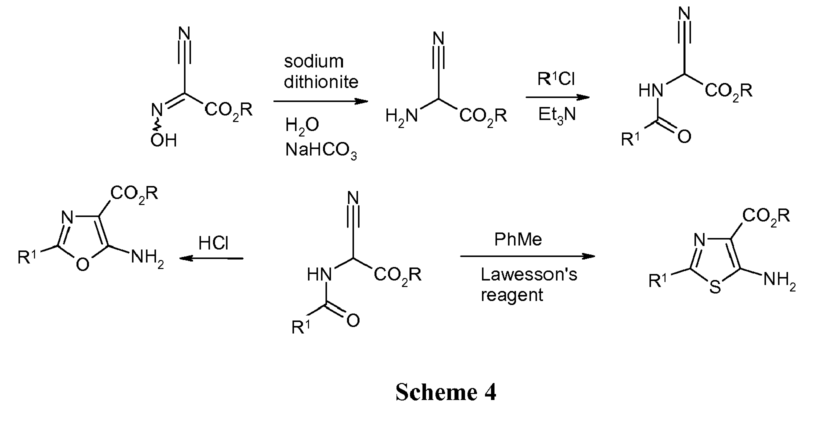

An alternate method for the generation of compounds of Formula (I) is described in Scheme 5 or 5a. This general route utilizes the same starting amino ester from Scheme 1 or 1a. The amide bond formation is executed from the reaction of the corresponding carboxylic acids (generated via hydrolysis of the ester in refluxing concentrated sodium hydroxide in methanol) with various amines. A variety of coupling agents can be used to effect this transformation including EDCI, DIC, BOP, and HATU under standard coupling methods very familiar to those practicing and trained in the art of organic synthesis. Generation of the final primary urea is then performed using a simple two-step reaction with trichloroacetylisocyanate followed by cleavage with ammonia in methanol. The urea can also be formed in one step by reaction of the amine with chlorosulfonyl isocyanate.

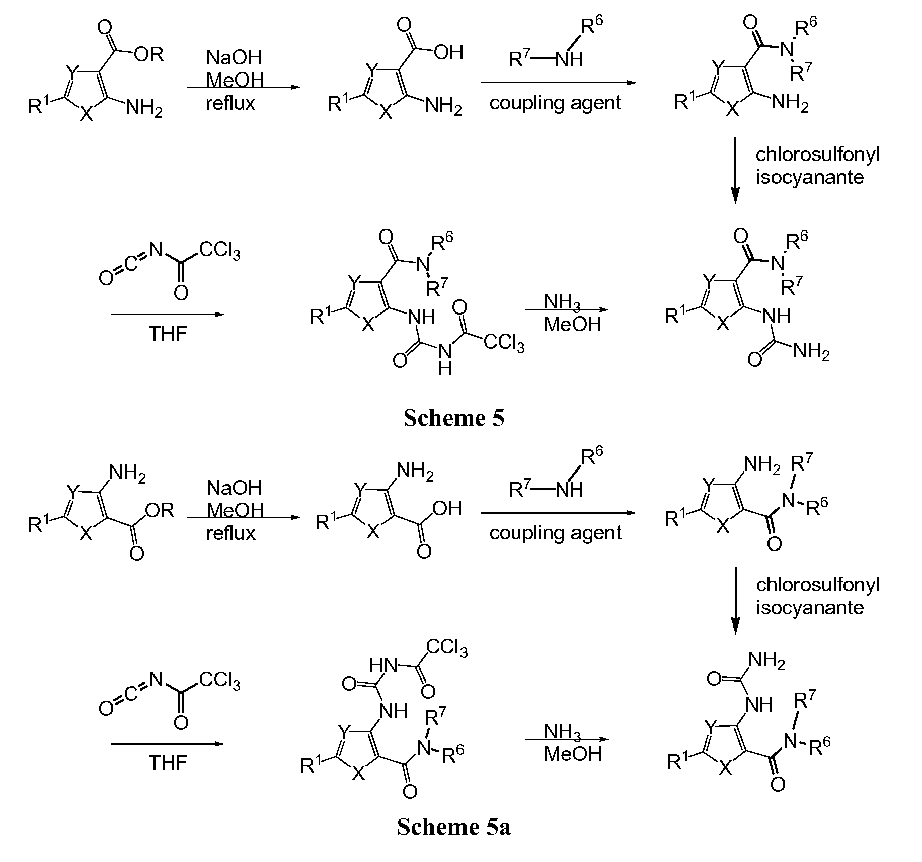

As shown in the following Schemes 6-9 and 6a-9a, the amino amides in Scheme 5 or 5a can be used as a common intermediate for the formation of various substituted ureas and N-substituted guanidiniums. Reaction with isocyanantes, acyl, or carbonyldiimidazole and amines, leads to the creation of various substituted ureas.

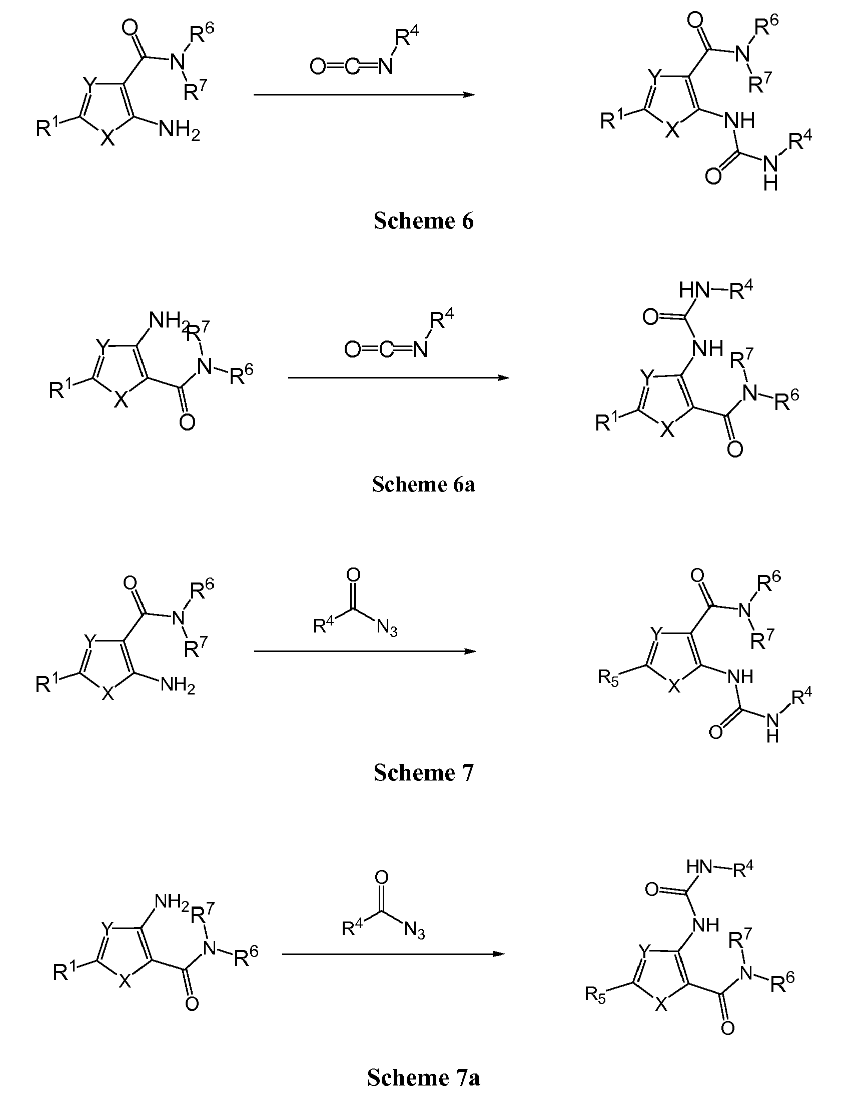

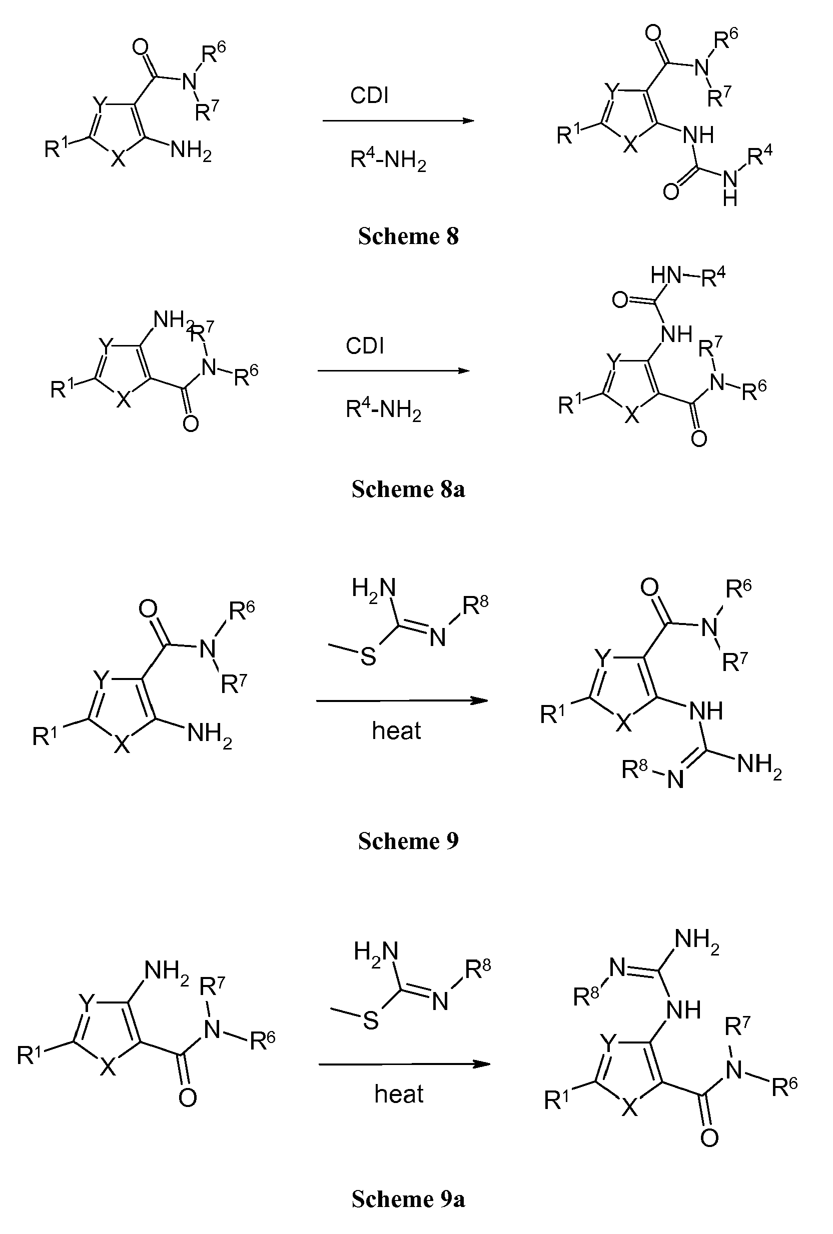

An additional general process presented in this invention involves the construction of general compounds with formula (I). The method, which employs a Suzuki Coupling of a 5-bromo heterocycle intermediate as its key transformation, is shown in Scheme 10 and Scheme 10a. Commercially available amino esters are protected as the trichloroacetyl urea, followed by selective bromination at the 5-position with bromine in acetic acid or N-bromosuccinimide. Removal of the protecting group with ammonia in methanol followed by Weinreb amidation yields the advanced bromoheterocycle intermediate. Standard Suzuki reaction conditions are then employed to finally generate the target compounds. If not commercially available, boronic acids are synthesized by normal methods. The Weinreb amidation and Suzuki coupling steps can also be done in alternate order as shown in Scheme 10 or 10a. Finally, the bromo ester and bromo urea can both be utilized as a substrate in other Pd-mediated transformations such as Stille Couplings with organotin reagents and Sonagashiri reactions with alkynes to produce compounds of Formula (I).

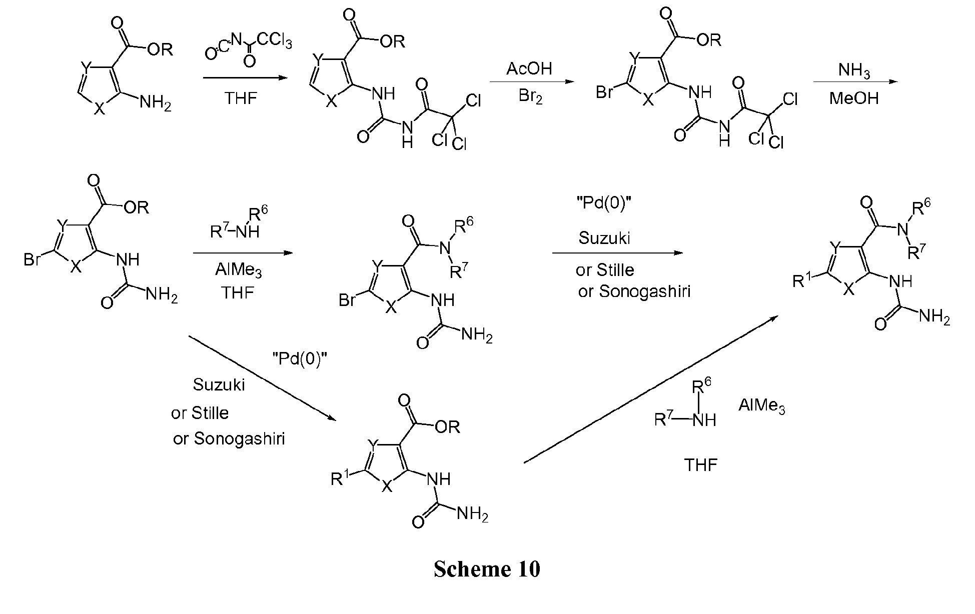

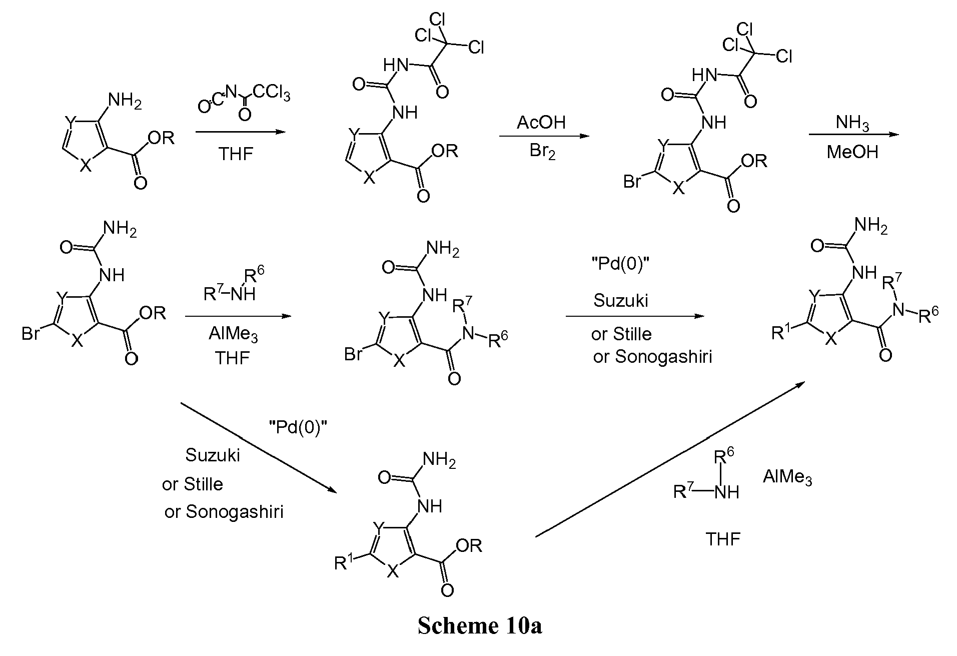

Another method for obtaining compounds of Formula (I) utilizes a "reverse" Suzuki Coupling as displayed in Scheme 11 or 11a. A boronate ester of the heterocyclic scaffold is first generated through a Pd-mediated transformation of the bromo ureido amide (Scheme 10 or 10a) that involves an *in situ* formation of the iodide and susbsequent boronation. This intermediate can be coupled to aryl halides or other reagents where L is a displaceable group under standard Pd coupling conditions. This invention allows for the synthesis of compounds of Formula (I) for which the corresponding aryl boronic acids or esters are not readily available for the penultimate transformation shown in Scheme 10 or 10a.

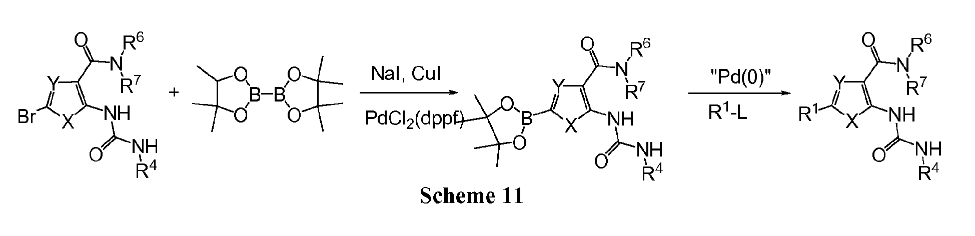

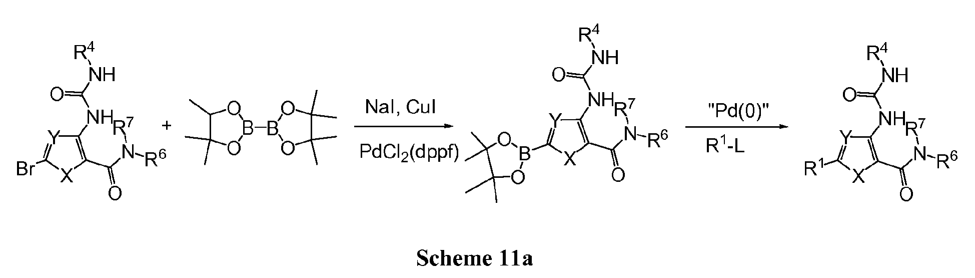

Sulfonamide compounds of Formula (I) can be formed using the following general procedure outlined in Scheme 12, below. Sulfonamide formation by reaction of a desired amine with commercially available sulfonyl chlorides, followed by selective deprotonation, subsequent azidation, and reduction yields the amino sulfonamide intermediate. Reaction as before with trichloroacetylisocyanate and ammonia to generate a primary urea followed by a Suzuki Pd-meditaed coupling gives the desired sulfonamide urea target molecules.

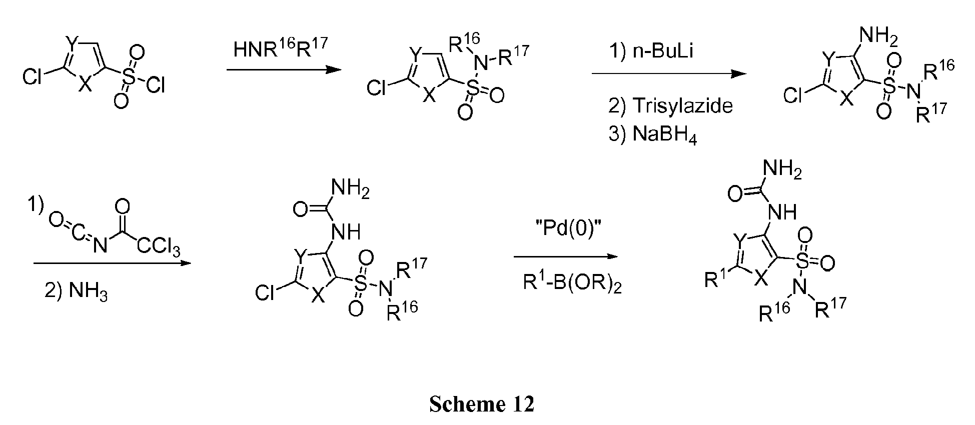

Substitution adjacent to X with a nitrogen heteroatom (R¹=NR¹¹R¹²) can be achieved using the procedure in Scheme 13 or 13a. This reaction can be accomplished by heating a bromo ureido amide (Scheme 10 or 10a) with a desired amine.

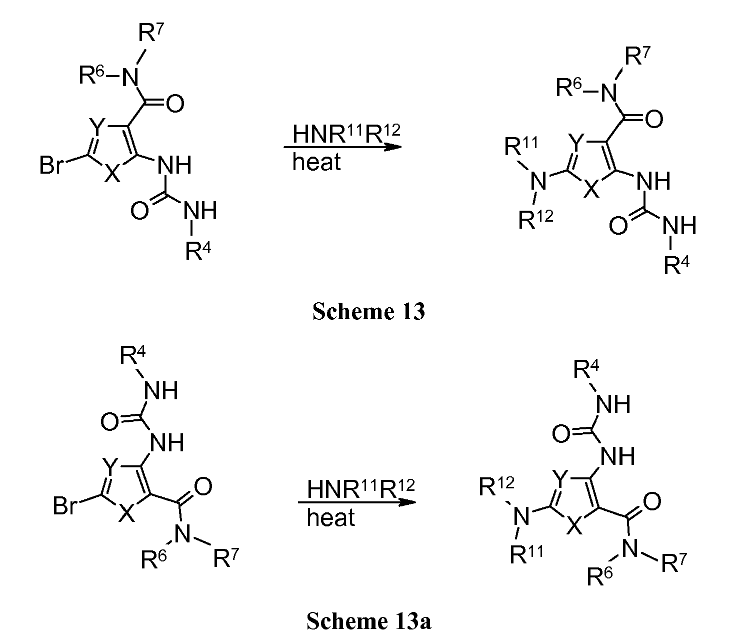

If the compound generated from any of the above-mentioned synthetic methods or Schemes 1-13 (and 1a-13a) has a protecting group present anywhere on the molecule standard synthetic methods of removal and deprotection can be utilized to generate final compounds. The general method employed for the deprotection of a *tert*-butoxycarbonyl carbamate to yield the free amine product is to use a strong anhydrous acid such as HCl or TFA. This gives products of Formula (I) as the corresponding hydrochloride or trifluoroacetate salt. Cleavages of methyl ethers to phenols or esters to acids are effected by reaction with the Lewis Acid boron tribromide (BBr₃) in methylene chloride. Cleavages of benzyloxycarbonyl carbamates or benzyl alcohols are effected by catalytic hydrogenation or the use of Lewis Acids such as BBr₃. These above methods are well known in the art of organic synthesis.

An additional embodiment of the present invention is directed to a process for the preparation of a compound of formula (I) or a pharmaceutically acceptable salt thereof, which comprises:
a. reacting a compound with formula (III) wherein A is thienyl and L is a displaceable group such as -OR, -CO₂alkyl or halogen wherein R is a hydrocarbon radical 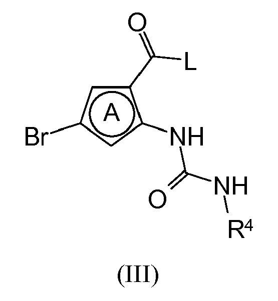 with an amine of formula (IV); 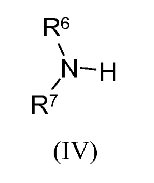 to yield a compound of formula (V) 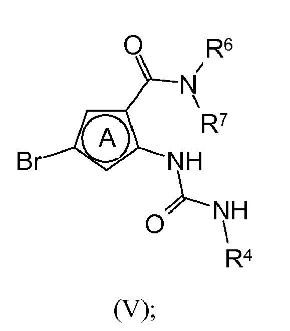
b. reacting a compound with formula (V) with a boronic acid or ester
   to form a compound of formula (I); and
c. optionally
   i) converting a compound of the formula **(I)** into another compound of the formula **(I);** and/or
   ii) forming a pharmaceutically acceptable salt thereof.

In another embodiment, the present invention is directed to a process for the preparation of a compound of formula (I) or a pharmaceutically acceptable salt thereof, which comprises:
a. reacting a compound of formula (VII) wherein A is thienyl and R is a hydrocarbon radical; 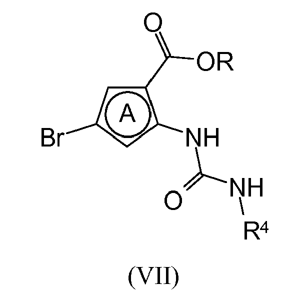 with a boronic acid or ester to form a compound of formula (VIII): 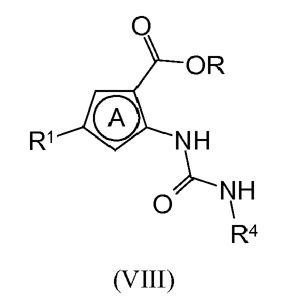
b. reacting a compound of formula (VIII) with an amine of formula (IV) 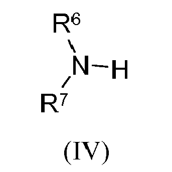 to form a compound of formula (I); and
c. optionally,
   i) converting a compound of the formula **(I)** into another compound of the formula (I) including removing any protecting groups; and/or
   ii) forming a pharmaceutically acceptable salt thereof.

In a still further embodiment, the present invention is directed toa process for the preparation of a compound of formula (I) or a pharmaceutically acceptable salt thereof, which comprises:
a. reacting a compound of formula (IX) wherein A is thienyl and R is a hydrocarbon radical; 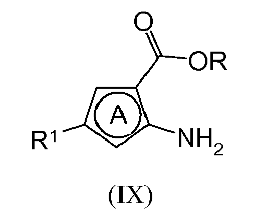 with a concentrated hydroxide base to form a compound of formula (X); 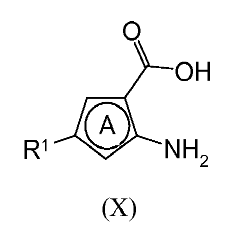
b. reacting the compound of formula (X) with an amine of formula (IV) 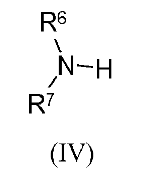 to form a compound of formula (XI) 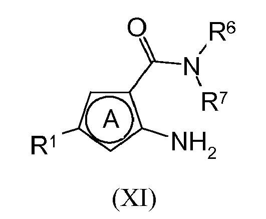
c. reacting the compound of formula (XI) with a compound selected from compounds of formulas (XII), (XIII) and a carbonylation reagent or (XIV); 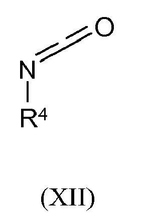 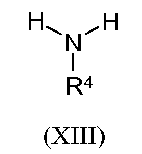 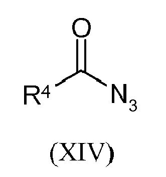 to form a compound of formula (I); and
d. optionally,
   i) converting a compound of the formula (I) into another compound of the formula (I), including removing any protecting groups; and/or
   ii) forming a pharmaceutically acceptable salt thereof.

In an additional embodiment, the present invention is directed to compounds of formula (XV), (XVI) and (XI) useful as intermediates in the production of compounds according to formula (I) 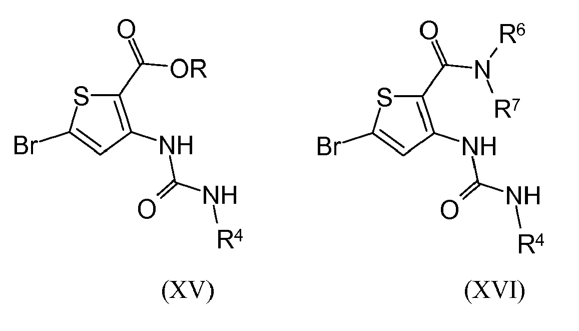 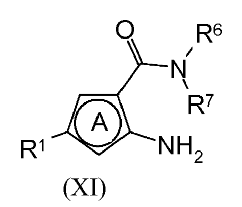 wherein R¹ is aryl and R⁴, R⁶ and R⁷ are as defined in formula (I), A is a thienyl ring and R is a hydrocarbon radical and provided that the compound of formula (XI) is not 3-Amino-5-(4-chloro-phenyl)-thiophene-2-carboxylic acid [(1R,2R)-2-(2,4-difluoro-phenyl)-2-hydroxy-1-methyl-3-[1,2,4]triazol-1-yl-propyl]-amide.

In a further embodiment, the present invention is directed to the use of compounds of formulas (XV), (XVI), (XI), or pharmaceutically acceptable salts or an *in vivo* hydrolysable precursor in the manufacture of a compound of formula (I).

The invention will now be further described with reference to the following illustrative examples in which, unless stated otherwise:
(i) temperatures are given in degrees Celsius (°C); operations are carried out at room temperature or ambient temperature, that is, in a range of 18-25 °C;
(ii) organic solutions were dried over anhydrous sodium sulfate; evaporation of organic solvent was carried out using a rotary evaporator under reduced pressure (4.5 - 30 mmHg) with a bath temperature of up to 60 °C;
(iii) chromatography means flash chromatography on silica gel; thin layer chromatography (TLC) was carried out on silica gel plates;
(iv) in general, the course of reactions was followed by TLC or liquid chromatography/mass spectroscopy (LC/MS) and reaction times are given for illustration only;
(v) final products have satisfactory proton nuclear magnetic resonance (NMR) spectra and/or mass spectra data;
(vi) yields are given for illustration only and are not necessarily those which can be obtained by diligent process development; preparations were repeated if more material was required;
(vii) when given, NMR data is in the form of delta values for major diagnostic protons, given in part per million (ppm) relative to tetramethylsilane (TMS) as an internal standard, determined at 300 MHz in d₆-DMSO unless otherwise stated;
(viii) chemical symbols have their usual meanings;
(ix) solvent ratio was given in volume:volume (v/v) terms;
(x) Rochelle's Salt is sodium potassium tartarate;
(xi) Hunig's Base is diisopropylethylamine (DIEA);
(xii) Purification of the compounds were carried out using one or more of the following methods:
a) flash chromatography on regular silica gel;
b) flash chromatography on silica gel using Isco Combiflash® separation system: RediSep normal phase flash column, flow rate, 30-40 ml/min;
c) flash chromatography on silica gel using Biotage® separation system;
c) Gilson semiprep HPLC separation system: YMC pack ODS-AQ column, 100x20mm, S 5µm 12 nm, water (0.1 % trifluoroacetic acid) and acetonitrile (0.1 % trifluoroacetic acid) as solvents, 20 min run; and
(xvi) the following abbreviations have been used:

| | |
|---|---|
| CIV | concentrated in vacuo; |
| DMF | dimethylformamide; |
| EtOAc | ethyl acetate; |
| ether | diethyl ether; |
| EtOH | ethanol; |
| THF | tetrahydrofuran; |
| MeOH | methanol; |
| DCM | dichloromethane; |
| TFA | trifluoracetic acid; and |
| TEA | triethylamine. |

### Example 1

### 5-Phenyl-2-ureido-thiophene-3-carboxylic acid (S)-azepan-3-ylamide

**methyl 2-amino-5-phenylthiophene-3-carboxylate.** To a solution of phenylacetaldehyde (12.7 mL, 100 mmol) in DMF (150 mL) was added cyanomethyl acetate (8.9 mL, 100 mmol) and sulfur (3.2 g, 100 mmmol), followed by diisopropylethylamine (Hunig's Base, 17.4 mL, 100 mmol). The resultant suspension immediately turned dark yellow to brown with an exotherm. The reaction mixture was stirred overnight at room temperature. The reaction was slowly added to water (-800 mL) while stirring. An off-white precipitate formed and was filtered after an additional 30 minutes of stirring. The resultant solid was purified by column chromatography (SiO₂, 10-20% EtOAc/ Hexanes) to yield 23.2g (100%) of the title compound as an off-white solid. ¹H NMR (d₆-DMSO, δ 7.5, br s, 2H; δ 7.45, m, 2H; δ 7.33, m, 2H; δ 7.24, s, 1H; δ 7.18, m, 1H; δ 3.73, s, 3H), LC/MS (APCI, ES, M+H=234).

**2-amino-5-phenylthiophene-3-carboxylic acid.** To a stirred solution of methyl 2-amino-5-phenylthiophene-3-carboxylate (13.0g, 55.7 mmol) in MeOH (400 mL) was added 6N NaOH (200mL) and water (100mL). The reaction was heated to reflux for 2h or until starting material was gone by TLC or LCMS. The solution was concentrated under vacuum to about half of the original volume. The pH of the resultant cloudy mixture was adjusted to 3-5 by the careful addition of 6N HCl (∼300 mL) while stirring. The gummy red precipitate was filtered and dried. Purification was achieved by triturating in boiling hexanes. The product (11.5g, 94%) was isolated in pure form by filtration after cooling to room temperature and drying in a vacuum oven overnight. ¹H NMR (d₆-DMSO, δ 12.0, br s, 1H; δ 7.43, m, 2H; δ 7.41, br s, 2H; δ 7.33, m, 2H; δ 7.22, s, 1H; δ 7.17, m, 1H), LC/MS (APCI, ES, M+H=220).

***tert*-butyl (3*S*)-3-aminoazepane-1-carboxylate.** (3*S*)-azepan-3-amine (5g; 43.8 mmol) was dissolved in 100 mL of anhydrous CH₂Cl₂ and cooled to -78 °C while stirring with a magnetic stirring bar. In another flask N-(tert-Butoxycarbonyloxy)succinimide [Boc-OSu] (9.7g; 45 mmol) was dissolved in 50 mL of anhydrous CH₂C1₂. To the stirred solution of the amine was added the solution of the succinimide over a period of 10-15 minutes so as to keep the reaction mixture at -78 °C while stirring. After the addition was complete, the reaction was allowed to warm to room temperature and then stirred for an additional 4h or until the reaction was complete by TLC (Ninhydrin; R_{f} 0.3; 0.1:1:10 NH₄OH, MeOH; CH₂Cl₂). The reaction mixture was washed with 50 mL of H₂O. The aqueous layer was brought to a pH >13 by the addition of 6N NaOH and extracted with CH₂C1₂ (3 x 100mL). The organic layer was dried over Na₂CO₃, filtered, and concentrated *in vacuo* to yield pure title compound as a viscous oil (5.1g, 54%). ¹H NMR (d₆-DMSO, d 3.4, m, 2H; d 2.89, m, 1H; d 2.71, m, 1H; d 2.54, m, 1H; d 1.54, m, 3H; d 1.34, m, 3H; d 1.27, s, 9H; d 1.12, m, 2H), LC/MS (APCI, ES, M+H=215).

***tert*-butyl (3*S*)-3-{[(2-amino-5-phenyl-3-thienyl)carbonyl]amino}azepane-1-carboxylate.** To a stirred solution of 2-amino-5-phenylthiophene-3-carboxylic acid in anhydrous DMF is added *tert*-butyl (3*S*)-3-aminoazepane-1-carboxylate (75mg, 0.34 mmol), 1-hydroxybenzotriazole (HOBt ,70 mg, 0.51 mmol), EDCI (71 mg, 0.34 mmol), and N-methylmorpholine (NMM, 0.15 ml, 1 mmol). The reaction mixture was stirred overnight at room temperature. The solution was diluted with water and EtOAc. The organic layer was separated and set aside. The remaining aqueous layer was extracted with EtOAc(2x) and then the combined organic extracts were pooled and washed with brine. The resultant EtOAc solution was dried over Na₂SO₄, filtered, and concentrated under vacuum to yield a brown solid. Purification was performed by column chromatography or MPLC (SiO₂, 20-30% EtOAc /hexanes) to give 100 mg (71 %) of the title compound as an off-white solid. ¹H NMR (d₆-DMSO, δ 7.65, s, 0.5H; δ 7.56, d, 0.5H; δ 7.55, s, 0.5H; δ 7.46, s, 2H; δ 7.44, d, 0.5H; δ 7.40, m, 2H; δ 7.34, t, 2H; δ 7.17, t, 1H; δ 4.10, m, 1H; δ 3.61, dq, 1H; δ 3.47, m, 1H; δ 3.16, m, 2H; δ 1.74, m, 3H; δ 1.56, m, 2H; δ 1.42, s, 4.5H; δ 1.38, s, 4.5H; δ 1.36, m, 1H), LC/MS (APCI, ES, M+H=416). [α]_{D}=-6.5°(25 °C, c=5.5, MeOH).

***tert*-butyl (3*S*)-3-({[5-phenyl-2-({[(trichloroacetyl)amino]carbonyl}amino)-3-thienyl]carbonyl}amino)azepane-1-carboxylate.** To a stirred solution of *tert*-butyl (3*S*)-3-{[(2-amino-5-phenyl-3-thienyl)carbonyl]amino}azepane-1-carboxylate dropwise (120 mg, 0.29 mmol) in anhydrous THF (3.0 mL) at room temperature was slowly added trichloroacetyl isocyanate (0.15 mL, 1.15 mmol)) dropwise over 5 min. After the addition was complete, the resulting cloudy solution was stirred for an additional 1h where after a precipitate formed. The desired product was obtained by concentration of the solvent under vacuum. The residue was diluted with MeOH and re-concentrated and dried under high vacuum. The product was used in the next step without purification. LC/MS (APCI, ES, M+H=603).

***tert*-butyl (3*S*)-3-[({2-[(aminocarbonyl)amino]-5-phenyl-3-thienyl}carbonyl)aminolazepane-1-carboxylate.** A solution of *tert*-butyl (3*S*)-3-({[5-phenyl-2-({[(trichloroacetyl)amino]carbonyl}amino)-3-thienyl]carbonyl}amino)azepane-1-carboxylate (0.29 mmol) in anhydrous MeOH (3.0 mL) was treated with a solution of NH₃ in MeOH (2.0 M, 0.3 mL, 0.58 mmol) at room temperature. The mixture was stirred for 1h at room temperature. Concentration of the reaction mixture under vacuum gave the desired product as white solid. Purification by column chromatography (SiO₂, 50% EtOAc/hexanes) gave the desired product as an off-white solid in good yield for the two step conversion (100mg, 76%). ¹H NMR (d₆-DMSO, δ 11.1, s, 1H; δ 7.99, d, 0.5H; δ 7.84, d, 0.5H; δ 7.82, s, 0.5H; δ 7.72, s, 1H; δ 7.54, m, 2H; δ 7.40, t, 2H; δ 7.25, t, 1H; δ 6.98, br s, 2H; δ 4.20, m, 0.5H; δ 4.12, m, 0.5H; δ 3.65, m, 1H; δ 3.48, m, 1H; δ 3.20, m, 3H; δ 1.76, m, 3H; δ 1.59, m, 2H; δ 1.42, s+m, 5.5H; δ 1.36, s, 4.5H), LC/MS (APCI, ES, M+H=459).

**5-Phenyl-2-ureido-thiophene-3-carboxylic acid (S)-azepan-3-ylamide.** To a stirred solution of *tert-butyl* (3*S*)-3-[({2-[(aminocarbonyl)amino]-5-phenyl-3-thienyl}carbonyl)amino]azepane-1-carboxylate (90 mg, 0.196 mmol) in 1, 4-dioxane (4.0 mL) was added 4.0N HCl in 1, 4-dioxane (4.0 mL, 16 mmoL). A precipitate forms shortly and the reaction is stirred for an additional 4h at room temperature. Due to the hygroscopic nature of the salt form, the solvent was removed under vacuum. The residue was dissolved in methanol and concentrated under vacuum (2x) to yield and off-white solid. Recrystallization from using 2-propanol gave 60 mg (80%) of the hydrochloride salt as a white solid. ¹H NMR (d₆-DMSO, δ 10.9, s, 1H; δ 9.57, br s, 1H; δ 9.28, br s, 1H; δ 8.44, d, 1H; δ 8.00, s, 1H; δ 7.56, d, 2H; δ 7.39, t, 2H; δ 7.24, t, 1H; δ 7.02, br s, 2H; δ 4.37, m, 1H; δ 3.30, m, 1H; δ 3.21, m, 2H; δ 3.08, m, 1H; δ 1.99, m, 1H; δ 1.84, m, 4H; δ 1.60, m, 1H), LC/MS (APCI, ES, M+H=359).

The following examples 2-7 were prepared in analogous fashion to Example 1 using the appropriate starting materials. MS in the table is M+H unless noted.

| **Ex. No.** | **Compound Name** | **MS** | **¹H NMR (300 MHz; d₆-DMSO; δ ppm) unless otherwise noted** |
|---|---|---|---|
| **2** | 5-(4-Chloro-phenyl)-3-ureido-thiophene-2-carboxylic acid (piperidin-2-ylmethyl)-amide | 393 | 1.14 - 1.61 (m, 2 H) 1.58 - 1.99 (m, 2 H) 2.66 - 2.96 (m, 2 H) 2.95 - 3.25 (m, 2 H) 3.35 (t, J=5.84 Hz, 2 H) 3.63 - 3.84 (m, 1 H) 6.54 - 6.73 (m, 1 H) 7.48 (d, 2 H) 7.59 (d, 2 H) 8.10 - 8.38 (m, 1 H) 9.86 (s, 1 H) |
| **3** | 5-(4-Chloro-phenyl)-3-ureido-thiophene-2-carboxylic acid (1-aza-bicyclo[2.2.2]oct-3-yl)-amide | 406 | 1.60 - 1.74 (m, 1 H) 1.75 - 1.94 (m, 2 H) 1.96 - 2.11 (m, 1 H) 2.11 - 2.25 (m, 1 H) 2.90 - 3.35 (m, 4 H) 3.61 (t, J=11.49 Hz, 1 H) 4.10 - 4.45 (m, 1 H) 6.96 (s, 1 H) 7.40 (d, 2 H) 7.52 (d, 2 H) 7.80 (s, 1 H) 8.22 (d, 1 H) |
| **4** | 5-(4-Chloro-phenyl)-3-ureido-thiophene-2-carboxylic acid [(3R,4S)-4-(4-fluoro-phenyl)-piperidin-3-yl]-amide | 474 | 1.72 - 1.93 (m, 2 H) 3.24 - 3.40 (m, 2 H) 3.65 - 3.81 (m, 2 H) 4.15 - 4.37 (m, 1 H) 4.61 - 4.80 (m, 1 H) 6.99 - 7.12 (m, 2 H) 7.24 (s, 2 H) 7.53 (d, *J*=7.00 Hz, 2 H) 7.65 (d, *J*=7.00 Hz, 2 H) 8.07 (d, *J*=9.61 Hz, 1 H) 8.16 (s, 1 H) |
| **5** | (R)-2-Amino-3-[(5-phenyl-3-ureido-thiophene-2-carbonyl)-amino]-propionic acid methyl ester | 363 | 2.44 (s, 2 H), 3.13 (d, 1 H), 3.28 (m, 2 H), 3.42 (m, 1 H), 3.53 (d, 1 H), 3.58 (s, 3 H), 6.62 (s, 2 H), 7.40 (m, 3 H), 7.58 (d, 2 H), 8.07 (t, 1 H), 8.21 (s, 1 H), 9.96 (s, 1 H) |
| **6** | 5-Phenyl-3-ureido-thiophene-2-carboxylic acid ((R)-2-amino-3-hydroxy-propyl)-amide | 335 | 2.93 (m, 1 H), 3.30 (m, 2 H), 3.39 (dd, 2 H), 4.71 (s, 2 H), 6.66 (s, 2 H), 7.44 (m, 3 H), 7.64 (d, 2 H), 8.00 (s, 1 H), 8.27 (s, 1 H), 10.04 (s, 1 H) |
| **7** | 5-Phenyl-3-ureido-thiophene-2-carboxylic acid ((S)-2-amino-3-hydroxy-propyl)-amide | 335 | 2.96 (m, 1 H), 3.15 (m, 1 H), 3.37 (m, 1 H), 4.02 (m, 1 H), 4.77 (s, 2 H), 6.58 (s, 2 H), 7.35 (m, 3 H), 7.55 (d, 2 H), 7.96 (s, 1 H), 8.19 (s, 1 H), 9.91 (s, 1 H) |

### Example 8

### [3-((S)-3-Amino-azepane-1-carbonyl)-5-pyridin-4-yl-thiophen-2-yl]-urea

**methyl 2-[(aminocarbonyl)amino]-5-pyridin-4-ylthiophene-3-carboxylate.** To a mixture of methyl 2-[(aminocarbonyl)amino]-5-bromothiophene-3-carboxylate [prepared as in Example 15] (0.28 g, 1.0 mmol), (Ph₃P)₄ (40 mg, 0.04 mmol), and 4-pyridyl-tributylstannane (0.47g, 1.2 mmol) in DMF (4.0 mL) was added Cul (40 mg, 0.20 mmol). The resultant heterogeneous mixture was heated to 80°C overnight in a sealed reaction vial while stirring. The reaction was cooled to room temperature, filtered, and concentrated *in vacuo.* The residue was purified by PrepLC (5-80% MeCN, H₂O 0.1% TFA) to give the product as a brown solid (140 mg, 50%). ¹H NMR (d₆-DMSO; 10.25 (s, 1H), 8.50 (br s, 2H), 7.80 (s, 1H), 7.60 (d, 2H), 7.30 (br s, 2H), 3.85 (s, 3H)). LCMS (APCI, M+H=278).

**benzyl (3*S*)-3-aminoazepane-1-carboxylate.** (3*S*)-azepan-3-amine (12.4 g; 108 mmol) was dissolved in 200 mL of anhydrous CH₂C1₂ and cooled to -78 °C while stirring with a magnetic stirring bar. In another flask N-(Benzyloxycarbonyloxy)succinimide [Cbz-OSu] (27.0 g; 108 mmol) was dissolved in 60 mL of anhydrous CH₂Cl₂. To the stirred solution of the amine was added the solution of the succinimide over a period of 10-15 minutes so as to keep the reaction mixture at -78 °C while stirring. After the addition was complete, the reaction was allowed to warm to room temperature and then stirred overnight or until the reaction was complete by TLC (Ninhydrin; R_{f} 0.25; 0.1:1:10 NH₄OH, MeOH; CH₂C1₂). The reaction was diluted with CH₂Cl₂, washed with saturated NaHCO₂). The organic layer was dried over Na₂CO₃, filtered, and concentrated *in vacuo* to yield crude product. To this residue was added 4.0 N HCl in dioxane (100 mL) and concentrated in vacuo. The hydrochloride salt was recrystallized from EtOAc to give the pure title compound as a white crystalline solid (12.5 g, 46%). ¹H NMR (d₆-DMSO; 8.30 (br s, 1H), 8.23 (br s, 2H), 7.35 (m, 5H), 5.10 (m, 2H), 3.83 (m, 1H), 3.48 (m, 1H), 3.28 (m, 3H), 1.87 (m, 1H), 1.75 (m, 2H), 1.56 (m, 2H), 1.33 (m, 1H)). LCMS (ES, M+H=249).

**benzyl (3*S*)-3-[(*tert*-butoxycarbonyl)aminolazepane-1-carboxylate.** To a stirred solution of benzyl (3*S*)-3-aminoazepane-1-carboxylate (12.4 g, 50 mmol) in 200 mL of CH₂Cl₂ was added 200 mL of saturated NaHCO₃. To this biphasic mixture was slowly added Boc₂O (11.0 g, 50 mmol). (dissolved in 50 mL of CH₂C1₂). The reaction was stirred for an additional 4h at room temperature. The organic layer was separated, dried over Na₂SO₄, filtered and concentrated to yield crude product (18.0 g) as a dark yellow residue. Recrystallization from hexanes gave pure product (12. 6g, 72%) as a crystalline solid. ¹H NMR (d₆-DMSO; 7.35 (m, 5H), 6.80 (m, 1H), 5.07 (m, 2H), 3.61 (m, 3H), 3.08 (m, 2H), 1.65 (m, 3H), 1.52 (m, 2H), 1.38 (m, 9H), 1.30 (m, 1H)). LCMS (ES, M+H=349).

***tert*-butyl (3*S*)-azepan-3-ylcarbamate.** To a solution of benzyl (3S)-3-[(*tert-*butoxycarbonyl)amino]azepane-1-carboxylate (17.4 g, 50 mmol) in MeOH (200 mL) was carefully added Pd(OH)₂ under a positive nitrogen atmosphere. To the reaction vessel was affixed a balloon of hydrogen and the resultant system was stirred overnight at room temperature, filtered through a pad of Celite, and concentrated in vacuo to yield the product (10.5 g, 98%) which was used in the next step without purification. ¹H NMR (d₆-DMSO; 6.61 (d, 1H), 3.41 (m, 2H), 2.83 (m, 1H), 2.69 (m, 1H), 2.46 (m, 1H), 1.68 (m, 2H), 1.54 (m, 2H), 1.44 (m, 2H), 1.38 (m, 1H), 1.37 (s, 9H)). LCMS (APCI, M+H=215).

***tert*-butyl [(3*S*)-1-({12-[(aminocarbonyl)amino]-5-pyridin-4-yl-3-thienyl}carbonyl)azepan-3-yl]carbamate.** To a solution of methyl 2-[(aminocarbonyl)amino]-5-pyridin-4-ylthiophene-3-carboxylate (140 mg, 0.50 mmol) in anhydrous THF (4 mL) was added a solution of [Me₂Al-*tert*-butyl (3*S*)-azepan-3-ylcarbamate] (3 equiv) in THF (4.75 mL) (which was preformed by the addition of Me₃Al (2.0M in hexanes, 0.75 mL, 1.5 mmol) to a solution of *tert*-butyl (3*S*)-azepan-3-ylcarbamate (320 mg, 1.5 mmol) in 4 mL of THF at -78°C followed by warming to room temperature and stirring for an additional 30 min) The resulting light orange solution was stirred overnight at room temperature. The reaction mixture was cooled with ice and a 10% aqueous solution of Rochelle's salt was added slowly to quench the reaction. The resulting biphasic solution was warmed to room temperature and stirred for an additional 1h. The mixture was diluted with EtOAc and H₂O, the aqueous layer was extracted with EtOAc (3x) and the combined organic extracts were washed with H₂O, brine and dried (Na₂SO₄). Evaporation gave a pale orange solid. Purification by PrepLC (5-95% MeCN, H₂O 0.1 % TFA) gave 60 mg (21 %) of the title compound as a yellow solid. LCMS (APCI, M+H=460).

**[3-((S)-3-Amino-azepane-1-carbonyl)-5-pyridin-4-yl-thiophen-2-yl]-urea** (N-(3-{[(3S)-3-aminoazepan-1-yl]carbonyl}-5-pyridin-4-yl-2-thienyl)urea). To a stirred solution of *tert*-butyl [(3*S*)-1-({2-[(aminocarbonyl)amino]-5-pyridin-4-yl-3-thienyl}carbonyl)azepan-3-yl]carbamate (60 mg, 0.13 mmol) in 1 mL of MeOH was added 4.0N HCl in 1, 4-dioxane (1 mL, 4 mmol). The solvent was removed under vacuum and the residue was redissolved in methanol and concentrated under vacuum (2x) to yield the title compound as an off-white solid. (45 mg, 80%). ¹H NMR (d₆-DMSO); 10.41 (s, 1H), 8.65 (d, 2H), 8.28 (m, 3H), 8.14 (s, 1H), 8.09 (d, 2H), 7.23 (br s, 1H), 4.04 (m, 1H), 2.98 (m, 1H), 2.82 (m, 2H), 2.03 (m, 1H), 1.75 (m, 3H), 1.44 (m, 2H), 1.22 (m, 1H). LCMS (APCI, M+H=360).

The following examples **9-11** were made in an analogous fashion to Example 8 using the appropriate starting materials.

| **Example No.** | **Compound Name** | **MS (M+H unless noted)** |
|---|---|---|
| 9 | [3-((S)-3-Amino-azepane-1-carbonyl)-5-pyridin0-3-yl-thiophen-2-yl]-urea | 360 |
| 10 | [3-((S)-3-Amino-azepane-1-carbonyl)-5-pyrazin-2-yl-thiophen-2-yl]-urea | 361 |
| 11 | [3-((S)-3-Amino-azepane-1-carbonyl)-5-pyrimidin-2-yl-thiophen-2-yl]-urea | 361 |

### Example 12

### 5-Ethynyl-2-ureido-thiophene-3-carboxylic acid (S)-azepan-3-ylamide

***tert*-butyl (3S)-3-[({2-[(aminocarbonyl)amino]-5-bromo-3-thienyl}carbonyl)amino]azepane-1-carboxylate.** To a solution of methyl 2-[(aminocarbonyl)amino]-5-bromothiophene-3-carboxylate [prepared according to Example 15] (1.0 g, 3.6 mmol) in anhydrous THF (20 mL) was added via cannula a solution of [Me₃Al and *tert*-butyl (3*S*)-3-aminoazepane-1-carboxylate] in THF (preformed by the careful addition of Me₃Al (2.0M in hexanes, 9 mL, 18 mmol) to a solution of *tert*-buty1 (3*S*)-3-aminoazepane-1-carboxylate (3.85g, 18 mmol) in 10 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as an off- white solid. LC/MS (ES, M+H=462).

**2-[(aminocarbonyl)amino]-*N*-[(3*S*)-azepan-3-yl]-5-[(trimethylsilyl)ethynyl]thiophene-3-carboxamide.** To a solution of *tert*-butyl (3*S*)-3-[({2-[(aminocarbonyl)amino]-5-bromo-3-thienyl}carbonyl)amino]azepane-1-carboxylate (142 mg, 0.3 mmol) in dry DMF (1.5 mL), Pd(PPh₃)₄ (10 mg), CuI (12 mg) were loaded in a microwave tube and the resulting mixture was purged with nitrogen for 10 mins. Triethylamine (130 µL, 0.92 mmol) and trimethylsilyl acetylyne (65 µL, 0.46 mmol) were added and the resulting dark mixture was heated to 140°C for 1200 seconds in a SMITH PERSONAL CHEMISTRY® microwave. The reaction mixture was cooled to rt and partitioned between EtOAc and H₂O, the aqueous layer was extracted with EtOAc (3x) and the combined organic extracts were washed with H₂O, brine and dried (MgSO₄). Evaporation gave a dark brown oil. The product was used in the next step without any further purification. LC/MS (ES, M+H=379).

**5-Ethynyl-2-ureido-thiophene-3-carboxylic acid (S)-azepan-3-ylamide.** To a solution of 2-[(aminocarbonyl)amino]-*N*-[(3*S*)-azepan-3-yl]-5*-*[(trimethylsilyl)ethynyl]thiophene-3-carboxamide (0.3 mmol) in dry THF (10 mL) was added a solution of TBAF (5mL, 1M in THF) and the resulting orange mixture was stirred for 2 h at rt. The reaction mixture was partitioned between EtOAc and H₂O, the aqueous layer was extracted with EtOAc (3x) and the combined organic extracts were washed with H₂O brine and dried (MgSO₄). Evaporation gave brown oil. The desired compound was isolated after purification by Gilson-HPLC as pale brown solid (7 mg, 8% over two steps). LC/MS (ES, M+H=305).

### Example 13

### 5-(3-Fluoro-phenyl)-3-ureido-thiophene-2-carboxylic acid (S)-piperidin-3-ylamide

**methyl 3-({[(trichloroacetyl)amino]carbonyl}amino)thiophene-2-carboxylate.** To a stirred solution of methyl 3-aminothiophene-2-carboxylate (17.0g, 108 mmol) in anhydrous THF (216 mL) was added trichloroacetyl isocyanate (12.9 mL, 108 mmol) slowly over a period of 20 min. After the addition was complete, a precipitate formed and the reaction stirred for an additional 1h at room temperature. The desired product was obtained by filtration to give the title compound (99%) as an off-white solid. The product was used in the next step without any further purification. LC/MS (ES, M+H=345).

**methyl 5-bromo-3-({[(trichloroacetyl)amino[carbony}amino)thioohene-2-carboxylate**. To a solution of methyl 3-({[(trichloroacetyl)amino]carbonyl}amino)thiophene-2-carboxylate (10.11 g, 29.38 mmol) in glacial acetic acid (146 mL) at 0°C was added slowly a solution of bromine (3 equiv) in glacial acetic acid (38mL). The resulting cloudy solution was to 70°C for 3 h whereupon the mixture was cooled to rt. The desired product (white solid, 12 g, 97%) was collected by filtration and the filtrate was washed with ether and dried under vacuum. LC/MS (ES, M+H=424).

**methyl 3-[(aminocarbonyl)amino]-5-bromothiophene-2-carboxylate.** A solution of methyl 5-bromo-3-({[(trichloroacetyl)amino]carbonyl}amino)thiophene-2-carboxylate (12g, 28.3 mmol) in dry MeOH (60 mL) was purged with gaseous NH₃ at 0°C until the solution became clear. The mixture was stirred for 20 mins at rt and evaporation gave the desired product (7.8 g, 99%) as white solid. ¹H NMR (300 MHz, DMSO-d6) δ ppm 9.35 (s, 1H), 8.10 (S, 1H), 6.90 br s, 2H), 3.82 (s, 3 H); LC/MS (ES, M+H=280).

***tert*-butyl (3*S*)-3-[({3-[(aminocarbonyl)amino]-5-bromo-2-thienyl}carbonyl)-amino]piperidine-1-carboxylate.** To a solution of methyl 3-[(aminocarbonyl)amino]-5-bromothiophene-2-carboxylate (1.6 g, 5.7 mmol) in dry THF (30 mL) was added a solution of [Me₃Al/ *tert*-butyl (3*S*)-3-aminopiperidine-1-carboxylate] in THF (18 mL) (which was preformed by the addition of Me₃Al (17.2 mL, 34.4 mmol) to a solution of *tert*-butyl (3*S*)-3-aminopiperidine-1-carboxylate (3.7 g, 17.2 mmol) in THF at -78°C and the resulting yellow solution was stirred at this temperature for 15 mins) and the resulting deep yellow solution was warmed to rt and stirred overnight at this temperature. The reaction mixture was cooled with ice and saturated solution of Rochelle's salt was added to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale orange solid. Purification by Gilson (5%H₂O→95%H₂O-MeCN) gave the desired product (1.0 g, 40%) as off-white solid. ¹H NMR (300 MHz, DMSO-d6; 10.06 (s, 1H), 8.02 (s, 1H), 7.89 (d, 1H), 6.71 (brs, 2H), 4.04 (m, 1H), 3.72 (m, 2H), 2.74 (m, 2H), 1.81 (m, 1H), 1.68 (m, 1H), 1.52 (m, 1H), 1.37 (s, 9H), 1.34 (m, 1H); LC/MS (ES, M+H=448).

### tert-Butyl (3S)-3-({[3-[(aminocarbonyl)amino]-5-(3-fluorophenyl)-2-thienyl]carbonyl}amino)piperidine-1-carboxylate

### (Route A)

*tert*-butyl (3*S*)-3-[({3-[(aminocarbonyl)amino]-5-bromo-2-thienyl}carbonyl)amino]piperidine-1-carboxylate (1,000 mg, 2.24 mmol), 3-fluorobenzeneboronic acid (470.2 mg, 3.36 mmol) and cesium carbonate (2,919.7 mg, 8.96 mmol) were dissolved in a mixture of water (3 mL) and 1,4-dioxane (20 mL). The solution was degassed under low vacuum and nitrogen. Palladium (0) tetrakis triphenylphosphine was added (259 mg, 0.22 mmol) to the solution and the colorless reaction mixture was heated at 75 °C with stirring for 1 hour. The aqueous layer was separated and ethyl acetate was added (20 mL) and the resulting solution was dried over MgSO₄ anhydrous. After solvent evaporation the residual solid was subjected to flash chromatography on silica gel (40 g) with a 2-5% methanol in methylene chloride over 30 min gradient. The main fraction contained traces of triphenylphosphine oxide and was resubjected to flash chromatography under the same conditions to afford a light yellow solid (916 mg, 88 %). MS ES+ 463 (M+1); ¹H NMR (CDC1₃, 300 MHz) δ 10.35 (s, 1H), 8.30 (s, 1H), 7.43-7.28 (m, 3H), 7.05 (m, 1H), 5.91 (brs, 1H), 4.79 (brs, 2H), 4.05 (m, 1H), 3.53-3.55 (m, 2H), 3.36-3.29(m, 2H), 1.89-1.49 (m, 13H).

### (Route B)

**(2*E,Z*)-3-chloro-3-(3-fluorophenyl)acrylonitrile.** POCl₃ (13.049 mL, 140 mmol) was added to DMF (21.7 mL, 280 mmol) with stirring and cooling while keeping the temperature below 40 °C. After the addition was completed 3-fluoroacetophenone (8.587 mL, 70 mmol) was added slowly. The reaction mixture was heated at 50 °C and hydroxylamine chloride was added in portions (19.457 g, 280 mmol). After each addition the temperature was allowed to rise and evolution of gas to cease. When the addition was finished the internal temperature of the reaction mixture reached 140 °C. The mixture was allowed to stir and soon solidified. Water/ice was added slowly and the solid that separated was filtered off, was washed with water on the filter (6 x 50 mL) and the solid was taken in ethyl acetate (60 mL), the solution was dried over MgSO₄ anhydrous and solvent was evaporated to afford a brown oil (12 g). The oil was subjected to flash chromatography on silica gel (120 g) with a 15-25 % ethyl acetate in hexanes over 20 min gradient. The early eluting fraction was collected and was evaporated to dryness. The residue was recrystalized from hot petrol ether (150 mL) to afford an off-white solid (3.334 g, 26 %). MS ES+ 182 (M+1); ¹H NMR (CDCl₃, 300 MHz) δ 7.44-7.17 (m, 1H), 7.24-7.17 (m, 1H), 6.04 (s, 1H).

**Methyl 3-amino-5-(3-fluorophenyl)thiophene-2-carboxylate.** Sodium methoxide 25% by weight (3.429 mL, 15 mmol) was dissolved in methanol (6 mL) and methyl thioglycolate (1.341 mL, 15 mmol) was added with stirring. To the brown solution (2*E,Z*)-3-chloro-3-(3-fluorophenyl)acrylonitrile (2.724 g, 15 mmol) was added in small portions and the resulting cream-like mass was heated at 76 °C under reflux for 20 min. Ethyl acetate (60 mL) was added to the cold suspension and the organic was washed with water (2 x 50 mL) followed by a dilute solution of bleach (50 mL) and saturated aqueous ammonium chloride (50 mL). The organic was dried over MgSO₄ and solvent evaporated to afford a brown solid. The solid was subjected to flash chromatography on silica gel (120 g) with 20% v/v ethyl acetate in hexanes over 25 min. The first fraction was the desired product that was obtained after evaporation of solvents as an off-white solid (2.282 g, 60.5%). MS ES- 250 (M-1); ¹H NMR (CDCl₃, 300 MHz) δ 7.36-7.24 (m, 3H), 7.07-7.06 (m, 1H), 6.76 (s, 1H), 5.48 (brs, 2H), 3.84 (s, 3H).

**Methyl3-[(aminocarbonyl)amino]-5-(3-fluorophenyl)thiophene-2-carboxylate.** Methyl 3-amino-5-(3-fluorophenyl)thiophene-2-carboxylate (2.517 g, 10 mmol) was dissolved in dry THF and was cooled to -78 °C. Trichloroacetylisocyanate (1.311 mL, 11 mmol) was added with stirring and the reaction was allowed to reach room temperature overnight. Methanol (10 mL) was added slowly and the solvents were evaporated under reduced pressure. The white residue was suspended in methanol (200 mL) and 7M ammonia in methanol was added (3 mL, 21 mmol). After stirring at room temperature for one hour the solvents were evaporated to dryness to afford a white powder. The white powder was suspended in hot methanol (10 mL) and allowed to cool to room temperature. The solid was filtered and dried in the air to afford a cream-colored powder (2.861 g, 97%). MS ES+ 295 (M+1); ¹H NMR (DMSO-d₆, 300 MHz) δ 9.24 (s, 1H), 8.33 (s, 1H), 7.54-7.50 (m, 3H), 7.31-7.24 (m, 1H), 6.86 (brs, 2H), 3.84 (s, 3H).

***tert*-Butyl (3*S*)-3-({[3-[(aminocarbonyl)amino]-5-(3-fluorophenyl)-2-thienyl]-carbonyl}amino)piperidine-1-carboxylate.** Methyl 3-[(aminocarbonyl)amino]-5-(3-fluorophenyl)thiophene-2-carboxylate (1.177 g, 4 mmol) was dissolved in THF (20 mL) and cooled to -78 °C. A solution of *tert*-butyl (3*S*)-3-aminopiperidine-1-carboxylate (3.096 mL, 9.6 mmol) in THF (14 mL) was cooled to -78 °C and trimethylaluminum 2M in hexanes (5 mL, 10 mmol) was added. An aliquot of the aluminate (20 mL, 10 mmol) was added to the previously prepared solution of ester in THF and the solution was heated at reflux for 4 hours. The cold reaction mixture was washed with 20% aqueous Rochelle's salt. The organic layer was separated and the aqueous layer was extracted with ethyl acetate (2 x 20 mL). The combined organic was dried and solvents were evaporated under reduced pressure. The residue was dissolved in ethyl acetate and was washed with aqueous 1M KHSO₄ (2x 20 mL) and brine (2x 20 mL). After drying over MgSO₄ and solvent evaporation the residue was subjected to flash chromatography on silica gel (120g) with a 50-70% ethyl acetate in hexanes over 45 min gradient. The late eluting fraction was the desired product, *tert*-Butyl (3*S*)-3-({[3-[(aminocarbonyl)amino]-5-(3-fluorophenyl)-2-thienyl]carbonyl}amino)piperidine-1-carboxylate, that was isolated as a glassy solid (724 mg, 39%). MS ES+ 463 (M+1); ¹H NMR (DMSO-d₆, 300 MHz) δ 10.02 (s, 1H), 8.29 (s, 1H), 7.96 (brs, 1H), 7.55-7.42 (m, 3H), 7.24 (t, 1H), 6.67 (brs, 1H), 3.75 (brs, 3H), 2.74-2.72 (m, 2H), 1.83-1.54 (m, 3H), 1.32 (s, 9+2H).

**5-(3-Fluoro-phenyl)-3-ureido-thiophene-2-carboxylic acid (S)-piperidin-3-ylamide.** *tert*-Butyl (3*S*)-3-({[3-[(aminocarbonyl)amino]-5-(3-fluorophenyl)-2-thienyl]carbonyl}amino)piperidine-1-carboxylate (916 mg, 1.98 mmol) was dissolved in methanol (7 mL) and 4M HCl in dioxane (10 mL, 40 mmol) was added while cooling at 0 °C and the solution was stirred for 3hours. The solvent was evaporated under reduced pressure and the residue was taken in methanol (20 mL) and evaporated to dryness. This procedure was repeated 5 times to yield a white powder. The solid was dissolved in water (10 mL) and filtered and solvent was evaporated by lyophilisation over 48 hours. Obtained a light yellow solid (669 mg 84%). MS ES+ 363 (M+1). ¹H NMR (DMSO-d₆, 300 MHz) δ 9.95 (s, 1H), 9.23 (brs, 2H), 8.28 (s, 1H), 8.21 (d, 1H), 7.55-7.42 (m, 3H), 7.24 (m, 1H), 4.19 (m, 1H), 3.95 (brs, 6H, water), 3.27-3.14 (m, 2H), 2.90-2.82 (m, 2H), 1.89-1.55 (m, 4H).

### Example 14

### 5-Benzofblthiophen-2-yl-3-ureido-thiophene-2-carboxylic acid (S)-piperidin-3-ylamide

A flask was loaded with *tert*-butyl (3*S*)-3-[({3-[(aminocarbonyl)amino]-5-bromo-2-thienyl}carbonyl)amino]piperidine-1-carboxylate [prepared as in Example 13] (100 mg, 0.25 mmol), 1-benzothien-2-ylboronic acid (44.5 mg, 0.375 mmol), Cs₂CO₃ (285 mg, 0.875mmol) and Pd(PPh₃)₄ (2.0 mg, 5% mmol) and was purged with nitrogen for 10 mins. Dioxane and H₂O were added and the resulting mixture was heated to 90°C for 2 h. The mixture was allowed to cool to rt and the mixture was filtered and the filtrate was washed with EtOAc. The organic layer was washed with H₂O, brine and dried (MgSO₄). Evaporation gave a yellow oil which was treated at 0°C with a solution of HCl in dioxane (4M, 5 mL). The resulting cloudy solution was stirred at rt for 30 mins whereupon the solvent was evaporated to afford a pale orange solid. Purification by Gilson (5%H₂O→95%H₂O-MeCN) gave the desired product. 1H NMR (300 MHz, DMSO-D6) δ ppm 1.46 - 1.64 (m, 2 H) 1.74 - 1.90 (m, 2 H) 2.68 - 2.93 (m, 2 H) 3.09 - 3.32 (m, 2 H) 3.97 - 4.13 (m, 1 H) 6.67 (s, 2 H) 7.33 - 7.38 (m, 2 H) 7.70 (s, 1 H) 7.77 - 7.87 (m, 1 H) 7.88 - 7.98 (m, 1 H) 8.09 (d, *J*=7.72 Hz, 1 H) 8.21 (s, 1 H) 8.62 (s, 2 H) 9.91 (s, 1 H); LC/MS (ES, M+H=401).

### Example 15

### 5-Phenyl-2-ureido-thiophene-3-carboxylic acid (S)-piperidin-3-ylamide

### tert-butyl (3S)-3-[({2-[(aminocarbonyl)amino]-5-ohenyl-3-thienyl}carbonyl)amino]piperidine-1-carboxylate.

### (Method A)

**methyl 5-phenyl-2-({[(trichloroacetyl)amino]carbonyl}amino)thiophene-3-carboxylate.** To a stirred solution of methyl 2-amino-5-phenylthiophene-3-carboxylate[prepared as in Example 1] (5.0 g, 21.4 mmol), in anhydrous THF (140 mL) at 0°C was slowly added trichloroacetyl isocyanate (2 equiv.) dropwise over 10 min. After the addition was complete, the resulting cloudy solution was stirred for an additional 1h at room temperature where after 30 min a precipitate formed. The desired product was obtained by filtration and drying under vacuum to give a light yellow solid (7.7 g, 86%). ¹H NMR (d₆-DMSO δ 12.55, br s, 1H; δ 12.41, s, 1H; δ 7.73, m, 1H; δ 7.71, m, 1H; δ 7.64, s, 1H; δ 7.47, t, 2H; δ 7.37, t, 1H; δ 3.95, s, 3H), LC/MS (APCI, ES, M+H=421).

To a solution of methyl 5-phenyl-2-({[(trichloroacetyl)amino]carbonyl}amino)thiophene-3-carboxylate (15.0 g, 35.6 mmol) in anhydrous THF (200 mL) was added a solution of [Me₂Al-3-(S)-3-Amino-piperidine-1-carboxylic acid tert-butyl ester] (4 equiv) in THF (200 mL) (which was preformed by the addition of Me₃Al (2.0M in hexanes, 71 mL, 142 mmol) to a solution of (S)-3-Amino-piperidine-1-carboxylic acid tert-butyl ester (28.5 g, 142 mmol) in 200 mL of THF at -78°C followed by warming to room temperature and stirring for an additional 30 min) The resulting light orange solution was stirred overnight at room temperature. The reaction mixture was cooled with ice and a 10% aqueous solution of Rochelle's salt was added slowly to quench the reaction. The resulting biphasic solution was warmed to room temperature and stirred for an additional 1h. The mixture was diluted with EtOAc and H₂O, the aqueous layer was extracted with EtOAc (3x) and the combined organic extracts were washed with H₂O brine and dried (Na₂SO₄). Evaporation gave a pale orange solid. Purification by FLASH Biotage MPLC (SiO₂, 50-70% EtOAc/hexanes) gave 10.9 g (69%) of a light yellow solid. ¹H NMR (CDCl₃, 300 MHz) δ 11.26 (s, 1H), 7.55 (d, 2H), 7.34 (t, 2H), 7.24 (s, 1H), 7.05 (s, 1H), 6.37 (brs, 1H), 5.43 (brs, 2H), 4.03 (brs, 1H), 3.64-3.33 (m, 4H), 1.86-1.26 (m, 13H). LC/MS (APCI, ES, M+H=445).

### (Method B)

**methyl 2-({[(trichloroacetyl)amino]carbonyl}amino)thiophene-3-carboxylate.** To a mixture of 2-amino thiophene-3-carboxylic acid methyl ester (42.0 g, 1 equiv) in tetrahydrofuran (8 vol.) was added dropwise trichloroacetyl isocyanate (1.05 equiv) in tetrahydrofuran (2 vol.) at -40°C; keeping the temp < 0°C (Caution!! exothermic). The resulting cloudy solution was warmed to 0°C and stirred for 3 h at this temperature. The product (an off-white yellowish solid) was obtained by filtration and washing with *iso*-hexane. The filtrate was concentrate to recovered more product (90.0 g, 84%). ¹H NMR (d₆-DMSO; 7.20 (d, 1H), 7.10 (d, 1H), 3.90 (s, 3H), 3.35 (br s, 2H)). LCMS (ES, M+H=345).

**methyl 5-bromo-2-({[(trichloroacetyl)amino]carbony}amino)thiophene-3-carboxylate.** To a solution of methyl 2-({[(trichloroacetyl)amino]carbonyl}amino)thiophene-3-carboxylate (50.0 g, 1 equiv) in glacial acetic acid (18 vol.) at 0°C was slowly added a solution of bromine (3 equiv) in glacial acetic acid (2 vol.). The resulting cloudy solution was stirred at room temperature overnight. The desired product (white solid) was collected by filtration and the filtrate washed with ether and dried under vacuum (48.0 g, 78%). ¹H NMR (d₆-DMSO; 7.30 (s, 1H), 3.85 (s, 3H), 3.35 (br s, 2H)). LCMS (ES, M+H=425).

**methyl 2-[(aminocarbonyl)amino]-5-bromothiophene-3-carboxylate.** methyl 5-bromo-2-({[(trichloroacetyl)amino]carbonyl}amino)thiophene-3-carboxylate (75.0 g, ) was treated with saturated methanol-ammonia (10 vol.) at room temperature under nitrogen atmosphere. The reaction was stirred for 3 hrs at RT and a mixture diethyl ether/ *iso*-hexane (1:1, 10 vol.) was added. This mixture was then filtered and washed with *iso*-hexane to afford the desired product as white solid. The filtrate was concentrated to recover more product (49.0 g, 98%). ¹H NMR (d₆-DMSO; 10.20 (br s, 1H), 7.2 (br s, 2H), 7.10 (s, 1H), 3.80 (s, 3H)). LC/MS (ES, M+H=280).

***tert*-butyl(3*S*)-3-[({2-[(aminocarbonyl)amino]-5-bromo-3-thienyl}carbonyl)amino] piperidine-1-carboxylate.** To a solution Boc-3-(S)-aminopiperidine (2 equiv) in tetrahydrofuran (10 vol.) was added trimethyl aluminium(2.0M in hexanes 2.2 equiv.) drop wise at -40°C (keeping the temp <-5°C). The mixture was stirred for 15 minutes and slowly warmed to room temperature (∼25°C). To this mixture was then added a suspension of methyl 2-[(aminocarbonyl)amino]-5-bromothiophene-3-carboxylate (28.0 g, 1 equiv) in tetrahydrofuran (12 vol.) at room temperature. The reaction mixture was stirred overnight (the suspension goes into a supernatant solution), cooled to -78°C and a solution of Rochelle's salt (10%) in water (40 vol.) added slowly. The mixture was allowed to warm and stirred for 1hr at room temperature then extracted with ethyl acetate (20 vol. x 3). The combined organic extracts were washed with water (20 vol. x 1), brine (10 vol. x 2), dried (magnesium sulfate) and concentrated to afford a dark solid. Column chromatography (ethyl acetate/ *iso*-hexanes, 50:50) gave a pale brown solid (32.0 g, 70%). ¹H NMR (d₆-DMSO, δ 10.9, s, 1H; δ 9.48, br s, 1H; δ 9.31, br s, 1H; δ 8.48, d, 1H; δ 8.10, s, 1H; δ 7.57, d, 2H; δ 7.38, t, 2H; δ 7.23, t, 1H; δ 7.01, br s, 2H; δ 4.26, m, 1H; δ 3.29, m, 1H; δ 3.11, m, 1H; δ 2.94, m, 2H; δ 1.91, m, 2H; δ 1.69, m, 2H), LC/MS (APCI, ES, M+H=345).

***tert*-butyl (3*S*)-3-[({2-[(aminocarbonyl)amino]-5-phenyl-3-thienyl}carbonyl)-amino]piperidine-1-carboxylate.** To a mixture of *tert*-butyl (3S)-3-[({2-[(aminocarbonyl) amino]-5-bromothien-3-yl}carbonyl) amino]piperidine-1-carboxylate (5.7 g, 1.0 equiv.), phenylboronic acid (1.5 equiv.) and caesium carbonate (3.0 equiv.) in dioxane-water (4:1, 40 vol.) was added Pd(PPh₃)₄ (0.1 equiv.) at room temperature. The mixture was heated to 90°C for 2-3hrs (LCMS shows no starting material) to cooled to room temperature and extracted in ethyl acetate (20 vol. x 3), dried (magnesium sulphate), filtered through celite and concentrated dryness. The residue was purified by column chromatography (ethyl acetate/ iso-hexanes, 30:70) to give a dark solid. Trituration in diethyl ether/ iso-hexane (1:1) gave the desired product as an off white solid (4.6 g, 81%). ¹H NMR (CDCl₃, 300 MHz) δ 11.26 (s, 1H), 7.55 (d, 2H), 7.34 (t, 2H), 7.24 (s, 1H), 7.05 (s, 1H), 6.37 (brs, 1H), 5.43 (brs, 2H), 4.03 (brs, 1H), 3.64-3.33 (m, 4H), 1.86-1.26 (m, 13H). LC/MS (ES, M+H=445).

**5-Phenyl-2-ureido-thiophene-3-carboxylic acid (S)-piperidin-3-ylamide.** To a stirred solution of *tert*-butyl (3*S*)-3-[({2-[(aminocarbonyl)amino]-5-phenyl-3-thienyl}carbonyl)amino]piperidine-1-carboxylate (10.9 g, 24.5 mmol) in 50 mL of 1, 4-dioxane was added 4.0N HCl in 1, 4-dioxane (50 mL, 200 mmol). A precipitate forms shortly and the reaction is stirred for an additional 4h at room temperature. Due to the hygroscopic nature of the salt form, the solvent was removed under vacuum. The residue was dissolved in methanol and concentrated under vacuum (2x) to yield and off-white solid. Recrystallization was performed using 2-propanol to yield the product as a light grey powder (7.5 g, 81%). ¹HNMR (d₆-DMSO, δ 10.9, s, 1H; δ 9.48, br s, 1H; δ 9.31, br s, 1H; δ 8.48, d, 1H; δ 8.10, s, 1H; δ 7.57, d, 2H; δ 7.38, t, 2H; δ 7.23, t, 1H; δ 7.01, br s, 2H; δ 4.26, m, 1H; δ 3.29, m, 1H; δ 3.11, m, 1H; δ 2.94, m, 2H; δ 1.91, m, 2H; δ 1.69, m, 2H), LC/MS (APCI, ES, M+H=345).

The following examples **16-173** are synthesized in an analogous manner to that of Example 15 using the appropriate starting materials. MS in Table is M+H unless noted.

| **Ex. No.** | **Compound Name** | **MS** | **¹H NMR (300 MHz; d₆-DMSO;** δ **ppm) unless otherwise noted** |
|---|---|---|---|
| 16 | 5-Pyridin-4-yl-2-ureido-thiophene-3-carboxylic acid (S)-piperidin-3-ylamide | 346 | 11.33 (s, 1H), 9.02 (br s, 2H), 8.89 (d, 2H), 8.62 (s, 1H), 8.51 (d, 1H), 8.08 (d, 2H), 7.48 (br s, 2H), 4.34 (m, 1H), 3.50 (dd, 2H), 3.06 (dd, 2H), 2.15 (dd, 2H), 1.83 (m, 2H) |
| 17 | 5-(4-Cyanomethyl-phenyl)-2-ureido-thiophene-3-carboxylic acid (S)-piperidin-3-ylamide | 384 | 10.93 (s, 1H), 9.23 (s, 1H), 9.10 (s, 1H), 8.35 (d, 1H), 8.02 (s, 1H), 7.70 (d, 1H), 7.58 (d, 2H), 7.46 (d, 1H), 7.36 (d, 2H), 7.04 (br, 2H), 4.24 (s, 1H), 4.05 (s, 2H), 3.29 (d, 1H), 3.14 (d, 1H), 2.92 (m, 2H), 1.91 (d, 2H), 1.66 (m, 2H). |
| 18 | 5-(3-Dimethylcarbamoyl-phenyl)-2-ureido-thiophene-3-carboxylic acid (S)-piperidin-3-ylamide | 416 | 10.95 (s, 1H), 9.40 (s, 1H), 9.28 (s, 1H), 8.41 (d, 1H), 8.14 (s, 1H), 7.62 (d, 1H), 7.58 (s, 1H), 7.45 (t, 1H), 7.24 (d, 1H), 7.07 (br, 2H), 4.26 (s, 1H), 3.29 (d, 1H), 3.14 (d, 1H), 2.97 (d, 8H), 1.90 (d, 2H), 1.70 (m, 2H). |
| 19 | 5-[3-(2-Methoxy-ethylcarbamoyl)-phenyl]-2-ureido-thiophene-3-carboxylic acid (S)-piperidin-3-ylamide | 446 | 10.93 (s, 1H), 9.19 (brs, 2H), 8.69 (brs, 1H), 8.341 (d, 1H), 8.05 (d, 2H), 7.70 (dd, 2H), 7.46 (t, 1H), 7.05 (brs, 2H), 4.42 (brs, 4H), 4.23 (brm, 1H), 3.48-3.42 (m, 4H), 3.26 (s, 3H), 3.19-2.90 (m, 4H), 1.92-1.62 (m, 4H). |
| 20 | 5-(3-Cyanomethyl-phenyl)-2-ureido-thiophene-3-carboxylic acid (S)-piperidin-3-ylamide | 384 | 10.96 (s, 1H), 9.29 (s, 1H), 9.18 (s, 1H), 8.41 (t, 1H), 8.02 (d, 1H), 7.58 (d, 1H), 7.52 (s, 1H), 7.46 (t, 1H), 7.25 (d, 1H), 7.06 (br, 2H), 4.23 (s, 1H), 4.09 (s, 2H), 3.29 (d, 1H), 3.14 (d, 1H), 2.93 (m, 2H), 1.91 (d, 2H), 1.67 (m, 2H). |
| 21 | 5-(3-Carbamoyl-phenyl)-2-ureido-thiophene-3-carboxylic acid (S)-piperidin-3-ylamide | 388 | 1.69 (m, 2 H), 1.93 (d, 2 H), 2.90 (m, 2 H), 3.18 (m, 2 H), 3.33 (d, 1 H), 4.21 (m, 1 H), 7.07 (s, 2 H), 7.47 (t, 2 H), 7.72 (dd, 2 H), 8.03 (s, 1 H), 8.11 (d, 2 H), 8.33 (d, 1 H), 9.08 (s, 2 H), 10.94 (s, 1 H) |
| 22 | 5-(4-Carbamoyl-phenyl)-2-ureido-thiophene-3-carboxylic acid (S)-piperidin-3-ylamide | 388 | 10.96 (s, 1H), 9.29 (s, 1H), 9.11 (s, 1H), 8.40 (d, 1H), 8.16 (s, 1H), 7.99 (s, 1H), 7.89 (t, 3H), 7.65 (d, 1H), 7.45 (m, 2H), 7.36 (s, 1H), 7.10 (br, 2H), 4.25 (s, 1H), 3.30 (d, 1H), 3.14 (d, 1H), 2.97 (m, 2H), 1.93 (d, 2H), 1.70 (m, 2H). |
| 23 | 5-(4-Cyano-phenyl)-2-ureido-thiophene-3-carboxylic acid (S)-piperidin-3-ylamide | 370 | 1.67 (m, 2 H), 1.93 (d, 2 H), 2.93 (t, 2 H), 3.17 (d, 1 H), 3.31 (d, 1 H), 4.20 (s, 1 H), 7.14 (s, 2 H), 7.72 (d, 2 H), 7.84 (d, 2 H), 8.22 (s, 1 H), 8.37 (d, 1 H), 9.03 (s, 2 H), 10.98 (s, 1 H) |
| 24 | 5-(3,5-Difluoro-phenyl)-2-ureido-thiophene-3-carboxylic acid (S)-piperidin-3-ylamide | 381 | 10.95 (s, 1H), 9.10 (s, 1H), 8.98 (s, 1H), 8.32 (d, 1H), 8.16 (s, 1H), 7.26 (d, 2H), 7.12 (t, 2H), 4.22 (s, 1H), 3.28 (d, 1H), 3.17 (d, 1H), 2.95 (m, 2H), 1.91 (d, 2H), 1.65 (m, 2H). |
| 25 | 5-Pyridin-3-yl-2-ureido-thiophene-3-carboxylic acid (S)-piperidin-3-ylamide | 346 | 10.76 (s, 1H), 8.68 (s, 1H), 8.58 (br s, 2H), 8.34 (d, 1H), 7.93 (dd, 2H),7.76 (s, 1H), 7.40 (dd, 1H), 6.94 (br s, 2H), 3.97 (m, 1H), 3.10 (dd, 2H), 2.70 (dd, 2H), 1.78 (dd, 2H), 1.48 (m, 2H) |
| 26 | 5-(4-Methanesulfonyl-phenyl)-2-ureido-thiophene-3-carboxylic acid (S)-piperidin-3-ylamide | 423 | 1.70 (m, 2 H), 1.93 (m, 2 H), 2.95 (m, 2 H), 3.15 (d, 1 H), 3.24 (s, 3 H), 3.30 (d, 1 H), 4.23 (m, 1 H), 7.14 (s, 2 H), 7.60 (m, 2 H), 7.81 (d, 2 H), 7.93 (d, 2 H), 8.29 (s, 1 H), 8.47 (d, 1 H), 9.12 (s, 1 H), 9.26 (s, 1 H), 11.02 (s, 1 H) |
| 27 | 5-(3-Amino-phenyl)-2-ureido-thiophene-3-carboxylic acid (S)-piperidin-3-ylamide | 360 | 1.70 (m, 2 H), 1.92 (d, 2 H), 2.94 (m, 2 H), 3.15 (s, 1 H), 3.31 (s, 1 H), 4.24 (m, 1 H), 7.08 (s/d, 3 H), 7.35 (s, 1 H), 7.43 (t, 1 H), 7.50 (d, 1 H), 8.08 (s, 1 H), 8.50 (s, 1 H), 9.19 (s, 1 H), 9.35 (s, 1 H), 10.96 (s, 1 H) |
| 28 | 5-[4-((S)-Piperidin-3-ylcarbamoyl)-5-ureido-thiophen-2-yl]-1H-pyrrole-2-carboxylic acid ethyl ester | 406 | 12.06 (s, 1H), 10.79 (s, 1H), 9.01 (brs, 1H), 8.15 (d, 1H), 7.70 (s, 1H), 7.03 (brs, 2H), 6.79 (m, 1H), 6.30 m, 1H), 4.26-4.19 (m, 3H), 3.40-2.84 (m, 4H), 1.92-1.60 (m, 4H), 1.27 (t, 3H). |
| 29 | 5-(3-Acetylamino-phenyl)-2-ureido-thiophene-3-carboxylic acid (S)-piperidin-3-ylamide | 402 | 1.69 (m, 2 H), 1.92 (d, 2 H), 2.91 (m, 2 H), 3.17 (d, 1 H), 3.31 (d, 1 H), 4.20 (m, 1 H), 6.93 (s, 1 H), 7.06 (s, 2 H), 7.21 (s, 1 H), 7.31 (m, 3H), 7.86 (m, 1 H), 7.98 (s, 1 H), 8.36 (q, 1 H), 9.08 (s, 2 H), 10.91 (s, 1 H) |
| 30 | 5-(4-Fluoro-phenyl)-2-ureido-thiophene-3-carboxylic acid (S)-piperidin-3-ylamide | 363 | 1.68 (m, 2 H), 1.92 (d, 2 H), 2.92 (m, 2 H), 3.17 (d, 1 H), 3.30 (d, 1 H), 4.21 (s, 1 H), 7.03 (s, 2 H) 7.25 (t, 2 H) 7.59 (t, 2 H), 7.92 (s, 1 H), 8.31 (d, 1 H), 9.07 (d, 2 H), 10.90 (s, 1 H) |
| 31 | 5-(3-Hydroxymethyl-phenyl)-2-ureido-thiophene-3-carboxylic acid (S)-azepan-3-ylamide | 389 | 10.90 (s, 1H), 9.06 (s, 1H), 8.89 (s, 1H), 8.22 (d, 1H), 7.82 (s, 1H), 7.56 (d, 1H), 7.44 (d, 1H), 7.35 (t, 1H), 7.20 (d, 1H), 7.05 (s, 2H), 5.28 (br, 1H), 4.54 (s, 2H), 4.29 (s, 1H), 3.18 (m, 4H), 1.99 (m, 1H), 1.85 (m, 4H), 1.59 (m, 1H). |
| 32 | 5-(3-Cyano-phenyl)-2-ureido-thiophene-3-carboxylic acid (S)-azepan-3-ylamide | 384 | 10.93 (s, 1H), 9.23 (s, 1H), 9.04 (s, 1H), 8.27 (d, 1H), 8.07 (s, 1H), 7.98 (s, 1H), 7.83 (d, 2H), 7.69 (d, 2H), 7.60 (t, 1H), 7.11 (s, 1H), 4.33 (s, 1H), 3.16 (m, 4H), 2.01 (m, 1H), 1.84 (m, 4H), 1.60 (m, 1H). |
| 33 | 5-(3,4-Dimethoxy-phenyl)-2-ureido-thiophene-3-carboxylic acid (S)-piperidin-3-ylamide | 405 | 10.89 (s, 1H), 9.41 (s, 1H), 9.11 (s, 1H), 8.42 (d, 1H), 7.99 (s, 1H), 7.19 (s, 1H), 7.05 (d, 1H), 6.98 (d, 2H), 4.25 (s, 1H), 3.84 (s, 3H), 3.77 (s, 3H), 3.28 (d, 1H), 3.11 (d, 1H), 2.98 (m, 2H), 1.92 (d, 2H), 1.69 (m, 2H). |
| 34 | 5-(4-Chloro-phenyl)-2-ureido-thiophene-3-carboxylic acid (S)-piperidin-3-ylamide | 379 | 10.92 (s, 1H), 9.12 (d, 2H), 8.34 (d,1H), 8.02 (s, 1H), 7.57 (d, 2H), 7.45 (d, 2H), 7.06 (s, 2H), 4.21 (s, 1H), 3.31 (d, 1H), 3.16 (d, 1H), 2.93 (t, 2H), 1.92 (d, 2H), 1.67 (m, 2H) |
| 35 | 5-(2-Fluoro-phenyl)-2-ureido-thiophene-3-carboxylic acid (S)-piperidin-3-ylamide | 363 | 10.98 (s, 1H), 9.29 (s, 1H), 9.20 (s, 1H), 8.43 (d, 1H), 8.06 (s, 1H), 7.77 (m, 2H), 7.29 (m, 3H), 7.08 (br, 2H), 4.25 (s, 1H), 3.29 (d, 1H), 3.14 (d, 1H), 2.92 (m, 2H), 1.93 (d, 2H), 1.67 (m, 2H). |
| 36 | 5-(4-Methylcarbamoyl-phenyl)-2-ureido-thiophene-3-carboxylic acid (S)-piperidin-3-ylamide | 402 | 10.95 (s, 1H), 9.13 (s, 1H), 9.03 (s, 1H), 8.46 (d, 1H), 8.35 (d, 1H), 8.10 (s, 1H), 7.88 (d, 2H), 7.62 (d, 2H), 7.46 (d, 1H), 7.08 (br, 2H), 4.23 (s, 1 H), 3.28 (d, 1H), 3.17 (d, 1H), 2.91 (m, 2H), 2.78 (s, 3H), 1.94 (d, 2H), 1.70 (m, 2H). |
| 37 | 5-(4-Dimethylcarbamoyl-phenyl)-2-ureido-thiophene-3-carboxylic acid (S)-piperidin-3-ylamide | 416 | 1.68 (m, 2 H), 1.92 (d, 2 H), 2.94 (s/s, 8 H), 3.17 (d, 1 H), 3.30 (m, 1 H), 4.20 (m, 1 H), 7.08 (s, 2 H), 7.45 (d, 2 H), 7.61 (d, d H), 8.04 (s, 1 H), 8.33 (d, 1 H), 8.98 (s, 1 H), 9.08 (s, 1 H), 10.95 (s, 1 H) |
| 38 | 5-(4-Cyclohexylcarbamoyl-phenyl)-2-ureido-thiophene-3-carboxylic acid (S)-piperidin-3-ylamide | | 1.12 (t, 1 H), 1.30 (q, 4 H), 1.64 (s, 2 H), 1.75 (s, 5 H), 1.93 (s, 2 H), 2.92 (m, 2 H), 3.17 (d, 1 H), 3.29 (d, 1 H), 3.74 (s, 1 H), 4.20 (s, 1 H), 7.09 (s, 2 H), 7.61 (d, 2 H), 7.87 (d, 2 H), 8.07 (s, 1 H), 8.21 (d, 1 H), 8.31 (d, 1 H), 8.97 (s, 1 H), 9.09 (s, 1 H), 10.93 (s, 1 H) |
| 39 | 5-(3-Cyano-phenyl)-2-ureido-thiophene-3-carboxylic acid (S)-piperidin-3-ylamide | 370 | 1.68 (m, 2 H), 1.93 (d, 2 H), 2.91 (m, 2 H), 3.18 (d, 2 H), 4.18 (s, 1 H), 7.12 (s, 2 H), 7.61 (t, 1 H), 7.71 (d, 1 H), 7.83 (d, 2 H), 7.98 (s, 1 H), 8.11 (s, 1 H), 8.28 (d, 1 H), 9.01 (s, 2 H), 10.94 (s, 1 H) |
| 40 | 5-(2,3,4-Trifluoro-phenyl)-2-ureido-thiophene-3-carboxylic acid (S)-piperidin-3-ylamide | 399 | 10.97 (s, 1H), 8.94 (brs, 2H), 8.32 (d, 1H), 7.95 (s, 1H), 7.34-7.53 (m, 23H), 7.08 (brs, 2H), 4.17 (m, 1H), 3.70-3.45 (m, 1H), 3.16 (brs, 1H), 2.88 (brm, 2H), 1.89 (m, 2H), 1.63 (m, 2H). |
| 41 | 5-(3-Fluoro-phenyl)-2-ureido-thiophene-3-carboxylic acid (S)-piperidin-3-ylamide | 363 | 1.67 (m, 2H), 1.93 (d, 2H), 2.93 (m, 2H), 3.18 (d, 1H), 3.30 (m, 1H), 4.19 (s, 1H), 7.08 (t, 2 H), 7.40 (m, 3 H), 8.03 (s, 1 H), 8.27 (d, 2 H), 8.98 (d, 2 H), 10.94 (s, 1 H) |
| 42 | 5-[4-(Morpholine-4-carbonyl)-phenyl]-2-ureido-thiophene-3-carboxylic acid (S)-piperidin-3-ylamide | 458 | 1.68 (m, 2 H), 1.92 (d, 2 H), 2.93 (m, 2 H), 3.16 (d, 2 H), 3.57 (m, 8 H), 4.21 (m, 1 H), 7.08 (s, 2 H), 7.45 (d, 2 H), 7.63 (d, 2 H), 8.05 (s, 1 H), 8.33 (d, 1 H), 8.97 (s, 1 H), 9.08 (s, 1 H), 10.94 (s, 1 H) |
| 43 | 5-(2,3-Difluoro-phenyl)-2-ureido-thiophene-3-carboxylic acid (S)-piperidin-3-ylamide | 381 | 11.01 (s, 1H), 9.22 (s, 1H), 9.14 (s, 1H), 8.46 (d, 1H), 8.10 (s, 1H), 7.81 (t, 1H), 7.29 (m, 2H), 7.11 (br, 2H), 4.20 (s, 1H), 3.30 (d, 1H), 3.14 (d, 1H), 2.92 (m, 2H), 1.90 (d, 2H), 1.69 (m, 2H). |
| 44 | 5-p-Tolyl-2-ureido-thiophene-3-carboxylic acid (S)-piperidin-3-ylamide | 359 | 1.68 (m, 2 H), 1.92 (d, 2 H), 2.91 (m, 2 H), 3.17 (d, 1 H), 3.31 (d, 1 H), 4.20 (s, 1 H), 7.01 (s, 2 H), 7.20 (d, 2 H). 7.45 (d, 2 H), 7.87 (s, 1 H), 8.27 (d, 1 H), 9.04 (s, 1 H), 9.15 (s, 1 H), 10.88 (s, 1 H) |
| 45 | 5-(2,3-Difluoro-phenyl)-2-ureido-thiophene-3-carboxylic acid (S)-azepan-3-ylamide | 395 | 11.01 (s, 1H), 9.52 (s, 1H), 9.23 (s, 1H), 8.51 (d, 1H), 8.08 (s, 1H), 7.52 (t, 1H), 7.29 (m, 2H), 7.07 (br, 2H), 3.16 (m, 4H), 1.98 (m, 1H), 1.83 (m, 4H), 1.57 (m, 1H). |
| 46 | 5-(3-Methanesulfonylamino-phenyl)-2-ureido-thiophene-3-carboxylic acid (S)-piperidin-3-ylamide | 438 | 1.67 (m, 2 H), 1.92 (d, 2 H), 2.90 (m, 2 H), 3.02 (s, 3 H), 3.18 (d, 1 H), 3.30 (d, 1 H), 4.20 (s, 1 H), 7.09 (s, 2 H), 7.39 (m, 3 H), 7.90 (s, 1 H), 8.33 (d, 1 H), 9.01 (s, 2 H), 9.84 (s, 1 H), 10.97 (s, 1 H) |
| 47 | 5-(3-Chloro-phenyl)-2-ureido-thiophene-3-carboxylic acid (S)-piperidin-3-ylamide | 379 | 10.94 (s, 1H), 9.10 (s, 1H), 8.30 (d, 1H), 8.06 (s, 1H), 7.61 (s, 1H), 7.49 (d, 1H), 7.42 (t, 1H), 7.30 (d, 1H), 7.09 (s, 2H), 4.21 (s, 1H), 3.32 (d, 1H), 3.17 (d, 1H), 2.92 (t, 2H), 1.93 (d, 2H), 1.68 (m, 2H). |
| 48 | 5-[4-(Piperidine-1-carbonyl)-phenyl]-2-ureido-thiophene-3-carboxylic acid (S)-piperidin-3-ylamide | 456 | 10.95 (s, 1H), 9.29 (br s, 1H), 9.12 (br s, 1H), 8.41 (d, 1H), 8.11 (s, 1H), 7.60 (d, 2H), 7.39 (d, 2H), 7.07 (br s, 1H), 4.48 (br s, 1H), 4.25 (m, 1H), 3.60 (m, 1H), 3.31 (m, 2H), 3.14 (m, 1H), 2.94 (dd, 2H), 1.91 (m, 2H), 1.65 (m, 5H), 1.52 (m, 5H) |
| 49 | 5-[4-(Pyrrolidine-1-carbonyl)-phenyl]-2-ureido-thiophene-3-carboxylic acid (S)-piperidin-3-ylamide | 442 | 1.67 (m, 2 H), 1.86 (m, 4 H), 2.93 (m, 2 H), 3.16 (d, 1 H), 3.28 (d, 1 H), 3.45 (m, 4 H), 4.22 (m, 1 H), 7.07 (s, 2 H), 7.59 (dd, 4 H), 8.10 (s, 1 H), 8.39 (d, 1 H), 9.06 (s, 1 H), 9.20 (s, 1 H), 10.97 (s, 1 H) |
| 50 | 5-(2-Fluoro-4-methyl-phenyl)-2-ureido-thiophene-3-carboxylic acid (S)-piperidin-3-ylamide | 377 | 10.92 (s, 1H), 9.02 (brs, 2H), 8.30 (d, 1H), 7.89 (s, 1H), 7.57 (t, 1H), 7.15-7.05 (m, 4H), 4.18 (m, 1H), 3.29-2.87 (m, 4H), 2.31 (s, 3H), 1.92-1.53 (m, 4H). |
| 51 | 5-(4-Fluoro-2-hydroxy-phenyl)-2-ureido-thiophene-3-carboxylic acid (S)-piperidin-3-ylamide | 379 | 10.84 (s, 1H), 10.71 (s, 1H), 9.30 (d, 2H), 8.32 (d, 1H), 7.88 (s, 1H), 7.60 (td, 1H), 6.80 (brs, 2H), 6.77 (dd, 1H), 6.69 (td, 1H), 4.20 (m, 1H), 3.30 (dd, 2H), 2.96-2.86 (m, 2H), 1.90 (brs, 2H), 1.69-1.61 (m, 2H). |
| 52 | 5-(4-Fluoro-phenyl)-2-ureido-thiophene-3-carboxylic acid (S)-azepan-3-ylamide | 377 | 10.88 (s, 1H), 8.90 (br s, 2H), 8.13 (d, 1H), 7.71 (s, 1H), 7.57 (dd, 2H), 7.26 (t, 2H), 7.05 (br s, 2H), 4.27 (m, 1H), 3.35 (m, 1H), 3.18 (m, 3H), 2.01 (m, 1H), 1.82 (m, 4H), 1.57 (m, 1H) |
| 53 | 5-p-Tolyl-2-ureido-thiophene-3-carboxylic acid (S)-azepan-3-ylamide | 373 | 10.88 (s, 1H), 9.24 (s, 1H), 9.02 (s, 1H), 8.24 (d, 1H), 7.81 (s, 1H), 7.45 (d, 2H), 7.22 (d, 2H), 7.01 (br, 2H), 4.33 (s, 1H), 3.16 (m, 4H), 2.31 (s, 3H), 1.97 (m, 1H), 1.83 (m, 4H), 1.68 (m, 1H). |
| 54 | 5-(3-Acetylamino-phenyl)-2-ureido-thiophene-3-carboxylic acid (S)-azepan-3-ylamide | 416 | 10.92 (s, 1H), 10.05 (s, 1H), 9.28 (s, 1H), 9.09 (s, 1H), 8.32 (d, 1H), 7.84 (d, 2H), 7.41 (m, 2H), 7.32 (m, 2H), 7.04 (s, 2H), 4.33 (s, 1H), 3.16 (m, 4H), 2.06 (s, 3H), 1.99 (m, 1H), 1.84 (m, 4H), 1.60 (m, 1H). |
| 55 | 5-(2,4-Difluoro-phenyl)-2-ureido-thiophene-3-carboxylic acid (S)-piperidin-3-ylamide | 381 | 10.97 (s, 1H), 9.30 (s, 1H), 9.18 (s, 1H), 8.45 (d, 1H), 8.02 (s, 1H), 7.78 (q, 1H), 7.38 (t, 1H), 7.18 (t, 1H), 7.06 (br, 2H), 4.23 (s, 1H), 3.29 (d, 1H), 3.14 (d, 1H), 2.92 (m, 2H), 1.90 (d, 2H), 1.69 (m, 2H). |
| 56 | 5-(4-Amino-phenyl)-2-ureido-thiophene-3-carboxylic acid (S)-piperidin-3-ylamide | 360 | 1.68 (m, 2 H), 1.91 (d, 2 H), 2.92 (m, 2 H), 3.17 (d, 1 H), 3.31 (d, 1 H), 4.20 (s, 1 H), 7.07 (m, 4 H), 7.51 (d, 2 H), 7.89 (s, 1 H), 8.32 (d, 1 H), 8.99 (s, 1 H), 9.15 (s, 1 H), 10.88 (s, 1 H) |
| 57 | 5-(3-Chloro-phenyl)-2-ureido-thiophene-3-carboxylic acid (S)-azepan-3-ylamide | 393 | 10.86 (s, 1H), 8.79 (br s, 2H), 8.05 (d, 1H), 7.80 (s, 1H), 7.54 (m, 1H), 7.41 (m, 2H), 7.26 (m, 1H), 7.03 (br s, 2H), 4.21 (m, 1H), 3.30 (m, 1H), 3.12 (m, 3H), 1.94 (m, 1H), 1.75 (m, 4H), 1.52 (m, 1H) |
| 58 | 5-(4-Chloro-phenyl)-2-ureido-thiophene-3-carboxylic acid (S)-azepan-3-ylamide | 393 | 11.05 (s, 1H), 9.02 (br s, 2H), 8.29 (d, 1H), 7.95 (s, 1H), 7.71 (d, 2H), 7.62 (d, 2H), 7.22 (br s, 2H), 4.43 (m, 1H), 3.50 (m, 1H), 3.33 (m, 3H), 2.16 (m, 1H), 1.98 (m, 4H), 1.73 (m, 1H) |
| 59 | 5-(3-Nitro-phenyl)-2-ureido-thiophene-3-carboxylic acid (S)-azepan-3-ylamide | 404 | 10.97 (s, 1H), 9.03 (s, 1H), 8.92 (s, 1H), 8.34 (s, 1H), 8.26 (d, 1H), 8.07 (s, 2H), 7.95 (d, 1H), 7.72 (t, 1H), 7.12 (s, 2H), 4.31 (s, 1H), 3.16 (m, 4H), 2.04 (m, 1H), 1.84 (m, 4H), 1.57 (m, 1H). |
| 60 | 5-(3-Methanesulfonyl-phenyl)-2-ureido-thiophene-3-carboxylic acid (S)-piperidin-3-ylamide | 423 | 1.71 (m, 2 H), 1.92 (m, 2 H), 2.94 (m, 2 H), 3.15 (s, 1 H), 3.40 (s, 4 H), 4.24 (m, 1 H), 7.12 (s, 2 H), 7.68 (t, 1 H), 7.78 (d, 1 H), 7.89 (d, 1 H), 8.05 (s, 1 H), 8.22 (s, 1 H), 8.47 (d, 1 H), 9.26 (d, 2 H), 10.99 (s, 1 H) |
| 61 | 5-(3-Fluoro-4-methyl-phenyl)-2-ureido-thiophene-3-carboxylic acid (S)-piperidin-3-ylamide | 377 | 10.88 (s, 1H), 9.00 (brs, 2H), 8.21 (d, 1H), 7.93 (s, 1H), 7.24-7.31 (m, 3H), 7.05 (brs, 2H), 4.16 (m, 1H), 3.14 (m, 2H), 2.91 (m, 2H), 2.22 (s, 3H), 1.90 (d, 2H), 1.63 (m, 2H). |
| 62 | 5-(3-Dimethylamino-phenyl)-2-ureido-thiophene-3-carboxylic acid (S)-piperidin-3-ylamide | 388 | 1.70 (m, 2 H), 1.91 (m, 2 H), 2.97 (m, 2 H), 3.11 (m, 8 H), 3.29 (s, 1 H), 4.23 (m, 1 H), 7.12 (s, 2 H), 7.36 (s, 2 H), 8.24 (s, 1 H), 8.53 (d, 1 H), 9.21 (s, 1 H), 9.46 (s, 1 H), 10.94 (s, 1 H) |
| 63 | 5-(3,5-Difluoro-phenyl)-2-ureido-thiophene-3-carboxylic acid (S)-azepan-3-ylamide | 395 | 10.92 (s, 1H), 9.08 (s, 1H), 8.93 (s, 1H), 8.22 (d, 1H), 8.04 (s, 1H), 7.25 (d, 2H), 7.13 (t, 2H), 4.29 (s, 1H), 3.16 (m, 4H), 1.98 (m, 1H), 1.84 (m, 4H), 1.60 (m, 1H). |
| 64 | 5-(2-Amino-phenyl)-2-ureido-thiophene-3-carboxylic acid (S)-piperidin-3-ylamide | 360 | 10.83 (s, 1H), 9.44 (s, 1H), 9.26 (s, 1H), 8.44 (d, 1H), 8.23 (s, 1H), 7.54 (m, 2H), 7.42 (d, 2H), 4.19 (s, 1H), 3.28 (d, 1H), 3.15 (d, 1H), 2.91 (m, 2H), 1.94 (d, 2H), 1.74 (m, 2H). |
| 65 | 5-[4-Fluoro-3-(piperidine-1-carbonyl)-phenyl]-2-ureido-thiophene-3-carboxylic acid (S)-piperidin-3-ylamide | 474 | 11.02 (s, 1H), 9.26 (br s, 1H), 9.17 (br s, 1H), 8.38 (d, 1H), 8.10 (s, 1H), 7.72 (m, 1H), 7.63 (m, 1H), 7.44 (t, 1H), 7.18 (br s, 2H), 4.27 (m, 1H), 3.74 (m, 2H), 3.42 (m, 1H), 3.34 (m, 2H), 3.27 (m, 1H), 3.02 (m, 2H), 2.05 (m, 2H), 1.72 (m, 6H), 1.58 (m, 2H) |
| 66 | 5-(4-Chloro-3-fluoro-phenyl)-2-ureido-thiophene-3-carboxylic acid (S)-piperidin-3-ylamide | 397 | 10.99 (s, 1H), 9.12 (brd, 2H), 8.39 (d, 1H), 8.07 (s, 1H), 7.66 (t, 1H), 7.47 (t, 1H), 7.26 (t, 1H), 7.10 (brs, 2H), 4.19 (m, 1H), 3.32-2.88 (m, 4H), 1.92-1.60 (m, 1H). |
| 67 | 5-(3-Fluoro-phenyl)-2-ureido-thiophene-3-carboxylic acid (S)-azepan-3-ylamide | 377 | 10.91 (s, 1H), 8.90 (br s, 2H), 8.12 (d, 1H), 7.85 (s, 1H), 7.44 (m, 1H), 7.36 (m, 2H), 7.10 (m, 1H), 7.10 (br s, 2H), 4.28 (m, 1H), 3.36 (m, 1H), 3.18 (m, 3H), 2.02 (m, 1H), 1.81 (m, 4H), 1.58 (m, 1H) |
| 68 | 5-(4-Chloro-3,5-difluoro-phenyl)-2-ureido-thiophene-3-carboxylic acid (S)-piperidin-3-ylamide | 417 | 10.89 (s, 1H), 9.19 (2 brs, 2H), 8.36 (d, 1H), 8.23 (s, 1H), 7.43 (d, 2h), 7.07 (brs, 2H), 4.18 (m, 1H), 3.22-2.88 (m, 4H), 1.85-1.57 (m, 4H). |
| 69 | 5-(4-Trifluoromethyl-phenyl)-2-ureido-thiophene-3-carboxylic acid (S)-piperidin-3-ylamide | 413 | 1.69 (m, 2 H), 1.93 (d, 2 H), 2.93 (m, 2 H), 3.18 (d, 1 H). 3.30 (m, 1 H), 4.21 (s, 1 H), 7.11 (s, 2 H), 7.76 (s, 4 H), 8.16 (s, 1 H), 8.37 (d, 1 H), 9.04 (d, 2 H), 10.98 (s, 1 H) |
| 70 | 5-(2,4-Dimethoxy-phenyl)-2-ureido-thiophene-3-carboxylic acid (S)-piperidin-3-ylamide | 405 | 10.86 (s, 1H), 9.27 (s, 1H), 9.14 (s, 1H), 8.27 (d, 1H), 7.79 (s, 1H), 7.54 (d, 1H), 6.91 (br, 2H), 6.66 (s, 1H), 6.59 (d, 1H), 4.22 (s, 1H), 3.87 (s, 3H), 3.80 (s, 3H), 3.29 (d, 1H), 3.14 (d, 1H), 2.91 (m, 2H), 1.93 (d, 2H), 1.69 (m, 2H). |
| 71 | 5-(3-Cyano-4-fluoro-phenyl)-2-ureido-thiophene-3-carboxylic acid (S)-piperidin-3-ylamide | 388 | 10.92 (s, 1H), 9.10 (s, 1H), 8.99 (s, 1H), 8.30 (d, 1H), 8.07 (s, 1 H), 8.04 (s, 1H), 7.94 (t, 1H), 7.58 (t, 2H), 7.11 (s, 2H), 4.21 (s, 1H), 3.28 (d, 1H), 3.17 (d, 1H), 2.89 (m, 2H), 1.94 (d, 2H), 1.69 (m, 2H). |
| 72 | 5-(3-Chloro-4-fluoro-phenyl)-2-ureido-thiophene-3-carboxylic acid (S)-azepan-3-ylamide | 411 | 10.90 (s, 1H), 9.20 (s, 1H), 9.02 (s, 1H), 8.26 (d, 1H), 7.93 (d, 1H), 7.73 (m, 2H), 7.44 (m, 2H), 7.12 (br, 2H).4.31 (s, 1H), 3.16 (m, 4H), 2.00 (m, 1H), 1.82 (m, 4H), 1.60 (m, 1H). |
| 73 | 5-(3,4-Dichloro-phenyl)-2-ureido-thiophene-3-carboxylic acid (S)-piperidin-3-ylamide | 413 | 10.96 (s, 1H), 9.30 (s, 1H), 9.19 (s, 1H), 8.43 (d, 1H), 8.18 (s, 1H), 7.79 (s, 1H), 7.63 (d, 1H), 7.51 (d, 1H), 7.10 (br, 2H), 4.24 (s, 1H), 3.29 (d, 1H), 3.13 (d, 1H), 2.94 (m, 2H), 1.92 (d, 2H), 1.70 (m, 2H). |
| 74 | 5-(4-Trifluoromethyl-phenyl)-2-ureido-thiophene-3-carboxylic acid (S)-azepan-3-ylamide | 427 | 10.95 (s, 1H), 9.03 (s, 1H), 8.90 (s, 1H), 8.38 (s, 2H), 8.27 (d, 1H), 8.04 (s, 1H), 7.98 (d, 2H), 7.76 (s, 2H), 7.69 (d, 2H), 7.12 (s, 2H), 4.31 (s, 1H), 3.16 (m, 4H), 1.98 (m, 1H), 1.84 (m, 4H), 1.57 (m, 1H). |
| 75 | 5-(1H-Pyrrol-3-yl)-2-ureido-thiophene-3-carboxylic acid (S)-azepan-3-ylamide | 348 | 10.92 (s, 1H), 10.78 (s, 1H), 8.85 (br s, 2H), 8.01 (d, 1H), 7.22 (s, 1H), 6.95 (s, 2H), 6.90 (br s, 2H), 6.23 (s, 1H), 4.27 (m, 1H), 3.34 (m, 1H), 3.18 (m, 3H), 1.96 (m, 1H), 1.82 (m, 4H), 1.58 (m, 1H) |
| 76 | 5-(3-Trifluoromethyl-phenyl)-2-ureido-thiophene-3-carboxylic acid (S)-azepan-3-ylamide | 427 | 10.95 (s, 1H), 9.19 (s, 1H), 9.00 (s, 1H), 8.41 (s, 1H), 8.30 (d, 1H), 8.07 (m, 2H), 7.60 (m, 2H), 7.11 (s, 2H), 4.32 (s, 1H), 3.16 (m, 4H), 1.99 (m, 1H), 1.84 (m, 4H), 1.60 (m, 1H). |
| 77 | 5-(2,4-Difluoro-phenyl)-2-ureido-thiophene-3-carboxylic acid (S)-azepan-3-ylamide | 395 | 10.94 (s, 1H), 9.02 (s, 1H), 8.87 (s, 1H), 8.25 (d, 1H), 7.83 (s, 1H), 7.71 (m, 1H), 7.39 (t, 1H), 7.20 (t, 1H), 7.06 (s, 2H), 4.28 (s, 1H), 3.17 (m, 4H), 1.97 (m, 1H), 1.81 (m, 4H), 1.58 (m, 1H). |
| 78 | 5-(4-Cyclopropylcarbamoyl-phenyl)-2-ureido-thiophene-3-carboxylic acid (S)-piperidin-3-ylamide | 428 | 0.58 (m, 2 H), 0.68 (m, 2 H), 1.70 (m, 2 H), 1.92 (m, 2 H), 2.95 (m, 2 H), 3.15 (d, 1 H), 3.31 (d, 1 H), 4.25 (m, 1 H), 7.09 (s, 1 H), 7.61 (d/m, 3 H), 7.86 (d, 2 H), 8.19 (s, 1 H), 8.46 (m, 2 H), 9.19 (s, 1 H), 9.37 (s, 1 H), 10.97 (s, 1 H) |
| 79 | 5-(3-Trifluoromethyl-phenyl)-2-ureido-thiophene-3-carboxylic acid (S)-piperidin-3-ylamide | 413 | 1.62 (m, 2 H), 1.86 (d, 2 H), 2.85 (m, 2 H), 3.11 (d, 1 H), 3.30 (m, 1 H), 4.14 (s, 1 H), 7.03 (s, 2 H) 7.55 (m, 2 H), 7.76 (m, 2 H), 8.04 (s, 1 H), 8.26 (d, 1 H), 9.00 (d, 2 H), 10.89 (s, 1 H) |
| 80 | 5-(2-Chloro-phenyl)-2-ureido-thiophene-3-carboxylic acid (S)-piperidin-3-ylamide | 379 | 10.96 (s, 1H), 9.21 (s, 1H), 9.14 (s, 1H), 8.36 (d, 1H), 7.83 (s, 1H), 7.62 (d, 1H), 7.54 (d, 1H), 7.36 (m, 2H), 7.05 (br, 2H), 4.22 (s, 1H), 3.28 (d, 1H), 3.17 (d, 1H), 2.89 (m, 2H), 1.89 (d, 2H), 1.64 (m, 2H). |
| 81 | 5-[3-(Piperidine-1-carbonyl)-phenyl]-2-ureido-thiophene-3-carboxylic acid (S)-piperidin-3-ylamide | 456 | 1.43 (m, 5 H), 1.59 (m, 7 H), 2.40 (m, 2 H), 2.82 (d, 1 H), 3.03 (d, 1 H), 3.31 (s, 1 H), 3.60 (s, 1 H), 3.82 (m, 1 H), 7.01 (s, 2 H), 7.20 (d, 1 H), 7.44 (t, 1 H), 7.56 (m, 2 H), 7.86 (d, 1 H), 7.92 (s, 1 H) |
| 82 | 5-(3-Fluoro-4-methoxy-phenyl)-2-ureido-thiophene-3-carboxylic acid (S)-azepan-3-ylamide | 407 | 10.87 (s, 1H), 9.41 (s, 1H), 9.14 (s, 1H), 8.33 (d, 1H), 7.87 (s, 1H), 7.80 (m, 1H), 7.41 (d, 1H), 7.29 (d, 1H), 7.19 (t, 1H), 7.02 (s, 2H), 4.33 (s, 1H), 3.85 (s, 3H), 3.17 (m, 4H), 1.97 (m, 1H), 1.84 (m, 4H), 1.57 (m, 1H). |
| 83 | 5-(4-Dimethylamino-phenyl)-2-ureido-thiophene-3-carboxylic acid (S)-piperidin-3-ylamide | 388 | 1.68 (m, 2 H), 1.91 (d, 2 H), 2.93 (s/m, 8 H), 3.18 (d, 1 H), 3.31 (d, 1 H), 4.19 (m, 1 H), 6.95 (m, 3 H), 7.45 (d, 2 H), 7.72 (s, 1 H), 8.23 (d, 1 H), 8.95 (s, 1 H), 9.06 (s, 1 H), 10.87 (s, 1 H) |
| 84 | 5-(4-Methanesulfonylamino-phenyl)-2-ureido-thiophene-3-carboxylic acid (S)-piperidin-3-ylamide | 438 | 1.69 (m, 2 H), 1.91 (m, 2 H), 2.96 (t,s, 5 H), 3.14 (d, 1 H), 3.30 (d, 1 H), 4.23 (m, 1 H), 7.03 (s, 1 H), 7.23 (d, 2 H), 7.54 (d, 2 H), 7.96 (s, 1 H), 8.40 (d, 1 H), 9.12 (s, 1 H), 9.32 (s, 1 H), 9.85 (s, 1 H), 10.91 (s, 1 H) |
| 85 | 5-(3,4,5-Trifluoro-phenyl)-2-ureido-thiophene-3-carboxylic acid (S)-piperidin-3-ylamide | 399 | 10.92 (s, 1H), 9.09 (s, 1H), 8.98 (s, 1H), 8.49 (s, 1H), 8.29 (d, 1H), 8.11 (s, 1H), 7.59 (t, 1H), 7.47 (t, 2H), 7.11 (s, 2H), 4.19 (s, 1H), 3.28 (d, 1H), 3.17 (d, 1H), 2.95 (m, 2H), 1.93 (d, 2H), 1.65 (m, 2H). |
| 86 | 5-(4-Benzyloxy-3-fluoro-phenyl)-2-ureido-thiophene-3-carboxylic acid (S)-piperidin-3-ylamide | 469 | 10.86 (s, 1H), 9.09 (brs, 1H), 8.98 (brs, 1H), 8.22 (d, 1H), 7.87 (s, 1H), 7.47-7.24 (m, 8H), 7.02 (brs, 2H), 5.19 (s, 2H), 4.17 (m, 1H), 3.20-2.92 (m, 4H), 1.91-1.60 (m, 4H). |
| 87 | 5-(3,5-Dichloro-phenyl)-2-ureido-thiophene-3-carboxylic acid (S)-azepan-3-ylamide | 427 | 10.97 (s, 1H), 9.62 (s, 1H), 9.41 (s, 1H), 8.49 (d, 1H), 8.23 (s, 1H), 7.57 (s, 2H), 7.46 (s, 1H), 4.36 (s, 1H), 3.16 (m, 4H), 1.98 (m, 1H), 1.87 (m, 4H), 1.62 (m, 1H). |
| 88 | 5-(2,4-Dimethoxy-phenyl)-2-ureido-thiophene-3-carboxylic acid (S)-azepan-3-ylamide | 419 | 10.82 (s, 1H), 9.07 (s, 1H), 8.90 (s, 1H), 8.17 (d, 1H), 7.65 (s, 1H), 7.51 (d, 1H), 7.13 (t, 2H), 6.91 (s, 2H), 6.67 (s, 1H), 6.62 (d, 1H), 4.29 (s, 1H), 3.87 (s, 3H), 3.80 (s, 3H), 3.16 (m, 4H), 1.98 (m, 1H), 1.82 (m, 4H), 1.60 (m, 1H). |
| 89 | 5-(3,4-Difluoro-phenyl)-2-ureido-thiophene-3-carboxylic acid (S)-azepan- 3-ylamide | 395 | 10.91 (s, 1H), 9.45 (s, 1H), 9.19 (s, 1H), 8.40 (d, 1H), 8.03 (s, 1H), 7.60 (m, 1H), 7.49 (t, 1H), 7.37 (d, 1H), 7.09 (s, 2H), 4.36 (s, 1H), 3.17 (m, 4H), 1.98 (m, 1H), 1.86 (m, 4H), 1.60 (m, 1H). |
| 90 | 5-(3,4-Dichloro-phenyl)-2-ureido-thiophene-3-carboxylic acid (S)-azepan-3-ylamide | 427 | 10.95 (s, 1H), 8.97 (s, 1H), 8.86 (s, 1H), 8.39 (s, 2H), 8.17 (d, 1H), 7.94 (d, 2H), 7.75 (m, 2H), 7.62 (m, 2H), 7.52 (d, 1H), 7.11 (br, 2H), 4.27 (s, 1H), 3.16 (m, 4H), 1.97 (m, 1H), 1.83 (m, 4H), 1.59 (m, 1H). |
| 91 | 5-(2,4-Dichloro-phenyl)-2-ureido-thiophene-3-carboxylic acid (S)-piperidin-3-ylamide | 413 | 10.95 (s, 1H), 9.00 (s, 2H), 8.31 (d, 1H), 7.82 (s, 1H), 7.74 (s, 1H), 7.62 (d, 1H), 7.52 (d, 1H), 7.08 (br, 2H), 4.19 (s, 1H), 3.28 (d, 1H), 3.17 (d, 1H), 2.86 (m, 2H), 1.93 (d, 2H), 1.68 (m, 2H). |
| 92 | 5-(3,5-Difluoro-4-methoxy-phenyl)-2-ureido-thiophene-3-carboxylic acid (S)-piperidin-3-ylamide | 411 | 10.95 (s, 1H), 9.36 (s, 1H), 9.19 (s, 1H), 8.47 (d, 1H), 8.28 (s, 1H), 7.46 (d, 1H), 7.23 (t, 1H), 7.03 (br, 2H), 4.24 (s, 1H), 3.81 (s, 3H), 3.29 (d, 1H), 3.13 (d, 1H), 2.95 (m, 2H), 1.92 (d, 2H), 1.70 (m, 2H). |
| 93 | 5-(4-Benzyloxy-2-fluoro-phenyl)-2-ureido-thiophene-3-carboxylic acid (S)-piperidin-3-ylamide | 469 | 10.90 (s, 1H), 9.08 (brs, 1H), 8.99 (brs, 1H), 7.81 (s, 1H), 7.59 (t, 1H), 7.47-7.34 (m, 5H), 7.05 (d, 1H), 6.93 (d, 1H), 6.90-7.00 (brs, 2H), 5.14 (s, 2H), 4.17 (m, 1H), 3.44 (d, 2H), 2.87 (brs, 2H), 1.80-1.60 (m, 4H). |
| 94 | 5-(5-Chloro-2-methoxy- phenyl)-2-ureido-thiophene-3-carboxylic acid (S)-azepan-3-ylamide | 423 | 10.87 (s, 1H), 9.00 (s, 1H), 8.85 (s, 1H), 8.18 (d, 1H), 7.90 (s, 1H), 7.66 (s, 1H), 7.42 (m, 1H), 7.28 (m, 1H), 7.16 (d, 1H), 6.98 (m, 2H), 4.27 (s, 1H), 3.90 (s, 3H), 3.16 (m, 4H), 1.98 (m, 1H), 1.85 (m, 4H), 1.57 (m, 1H). |
| 95 | 5-(2,5-Dimethoxy-phenyl)-2-ureido-thiophene-3-carboxylic acid (S)-azepan-3-ylamide | 419 | 10.87 (s, 1H), 9.37 (s, 1H), 9.04 (s, 1H), 8.35 (d, 1H), 7.98 (s, 1H), 7.26 (s, 1H), 7.11 (s, 1H), 7.04 (d, 2H), 6.93 (d, 2H), 6.85 (d, 2H), 4.33 (s, 1H), 3.82 (s, 3H), 3.70 (s, 3H), 3.18 (m, 4H), 2.03 (m, 1H), 1.83 (m, 4H), 1.59 (m, 1H). |
| 96 | 5-(4-Amino-3-fluoro-phenyl)-2-ureido-thiophene-3-carboxylic acid (S)-piperidin-3-ylamide | 378 | 10.85 (s, 1H), 9.18 (s, 1H), 9.04 (s, 1H), 8.26 (d, 1H), 7.79 (s, 1H), 7.26 (d, 1H), 7.13 (d, 1H), 7.00 (br, 2H), 6.90 (t, 1H), 4.19 (s, 1H), 3.29 (d, 1H), 3.17 (d, 1H), 2.94 (m, 2H), 1.92 (d, 2H), 1.68 (m, 2H). |
| 97 | 5-(5-Fluoro-2-methoxy-phenyl)-2-ureido- thiophene-3-carboxylic acid (S)-azepan-3-ylamide | 407 | 10.87 (s, 1H), 9.31 (s, 1H), 9.07 (s, 1H), 8.29 (d, 1H), 8.00 (s, 1H), 7.51 (d, 1H), 7.10 (m, 3H), 6.96 (m, 1H), 4.34 (s, 1H), 3.88 (s, 3H), 3.17 (m, 4H), 2.95 (m, 1H), 1.91 (m, 4H), 1.65 (m, 1H). |
| 98 | 5-(3-Isopropyl-phenyl)-2-ureido-thiophene-3-carboxylic acid (S)-azepan-3-ylamide | 401 | 10.91 (s, 1H), 9.19 (s, 1H), 9.04 (s, 1H), 8.29 (d, 1H), 8.00 (s, 1H), 7.87 (s, 1H), 7.67 (s, 1H), 7.57 (d, 1H), 7.29 (m, 3H), 7.04 (s, 2H), 4.31 (s, 1H), 3.19 (m, 4H), 2.89 (m, 1H), 2.00 (m, 1H), 1.84 (m, 4H), 1.56 (m, 1H), 1.22 (q, 6H). |
| 99 | 5-(2,3,4-Trimethoxy-phenyl)-2-ureido-thiophene-3-carboxylic acid (S)-piperidin-3-ylamide | 435 | 10.04 (s, 1H), 9.37 (s, 1H), 9.28 (s, 1H), 8.06 (d, 1H), 7.81 (d, 1H), 7.06 (d, 1H), 6.90 (d, 2H), 4.22 (s, 1H), 3.84 (s, 3H), 3.78 (s, 3H), 3.68 (s, 3H), 3.32 (d, 1H), 3.17 (d, 1H), 2.83 (m, 2H), 1.89 (d, 2H), 1.64 (m, 2H). |
| 100 | 5-(5-Chloro-2-methoxy-phenyl)-2-ureido-thiophene-3-carboxylic acid (S)-piperidin-3-ylamide | 409 | 10.91 (s, 1H), 9.21 (s, 1H), 9.19 (s, 1H), 8.34 (d, 1H), 8.04 (s, 1H), 7.72 (s, 1H), 7.30 (d, 1H), 7.12 (d, 1H), 6.98 (br, 2H), 4.21 (s, 1H), 3.90 (s, 3H), 3.29 (d, 1H), 3.15 (d, 1H), 2.91 (m, 2H), 1.91 (d, 2H), 1.68 (m, 2H). |
| 101 | 5-(2-Benzyloxy-4-fluoro-phenyl)-2-ureido-thiophene-3-carboxylic acid (S)-piperidin-3-ylamide | 469 | 10.78 (s, 1H), 8.72 (brs, 2H), 8.03 (d, 1H), 7.64-7.48 (m, 5H), 7.41-7.28 (m, 3H), 7.13 (dd, 1H), 6.95 (brs, 2H), 6.88 (dt, 1H), 5.28 (s, 2H), 4.11 (m, 1H), 3.34-3.24 (m,1H), 2.83 (m, 2H), 1.90-1.58 (m, 5H). |
| 102 | 5-(2,3,5-Trichloro-phenyl)- 2-ureido-thiophene-3-carboxylic acid (S)-azepan-3-ylamide | 463 | 10.97 (s, 1H), 9.15 (s, 1H), 8.97 (s, 1H), 8.29 (d, 1H), 7.86 (d, 2H), 7.67 (s, 1H), 7.59 (m, 1H), 7.10 (s, 2H), 4.31 (s, 1H), 3.16 (m, 4H), 1.99 (m, 1H), 1.82 (m, 4H), 1.59 (m, 1H). |
| 103 | 5-(4-Fluoro-3-hydroxymethyl-phenyl)-2-ureido-thiophene-3-carboxylic acid (S)-piperidin-3-ylamide | 393 | 10.90 (s, 1H), 9.15 (m, 2H), 8.33 (d, 1H), 7.88 (s, 1H), 7.63 (m, 1H), 7.50 (m, 1H), 7.18 (t, 1H), 7.04 (brs, 2H), 5.40 (brs, 1H), 4.56 (s, 2H), 4.18 (brs, 1H), 3.28 (m, 2H), 2.91 (m, 2H), 1.92-1.60 (m,4H). |
| 104 | 5-(4-Cyano-phenyl)-3-ureido-thiophene-2-carboxylic acid (S)-piperidin-3-ylamide | 370 | 9.82 (s, 1H), 8.98(brs, 1H), 8.32(s, 1H), 8.19(d, 1H), 7.82(d, 2H), 7.76 (d, 2H), 6.62(brs, 1H), 4.18 (m, 1H), 3.18 (m,2H), 2.78 (m, 2H), 1.89-1.42 (m, 4H). |
| 105 | 5-(3-Carbamoyl-phenyl)-3-ureido-thiophene-2-carboxylic acid (S)-piperidin-3-ylamide | 388 | 9.89 (s, 1H), 9.00(brs, 2H), 8.28 (s, 1H), 8.15(m, 3H), 7.51(m, 2H), 6.68(brs, 1H), 4.18 (m, 1H), 3.18 (m,2H), 2.78 (m, 2H), 1.87-1.50(m, 4H). |
| 106 | 5-(4-Carbamoyl-phenyl)-3-ureido-thiophene-2-carboxylic acid (S)-piperidin-3-ylamide | 388 | 9.89 (s, 1H), 9.18(brs, 2H), 8.28 (s, 1H), 8.19(d, 1H), 7.92(d, 2H), 7.68(d, 2H), 7.42(brs, 2H), 6.62(brs, 1H), 4.18 (m, 1H), 3.18 (m,2H), 2.78 (m,2H), 1.87-1.50(m, 4H). |
| 107 | 5-[4-(Piperidine-1-carbonyl)-phenyl]-3-ureido-thiophene-2-carboxylic acid (S)-piperidin-3-ylamide | 456 | 9.89 (s, 1H), 9.18(brs, 2H), 8.18 (s, 1H), 8.09(d, 1H), 7.56(d, 2H), 7.39(d, 2H), 6.62(brs, 1H), 4.18 (m, 1H), 3.18 (m,6H), 2.78 (m,2H), 1.80(m, 2H),1.65-1.28(m, 8H). |
| 108 | 5-(3-Aminomethyl-phenyl)-3-ureido-thiophene-2-carboxylic acid (S)-piperidin-3-ylamide | 374 | 1.54 - 1.74 (m, 2 H) 1.81 - 1.93 (m, 2 H) 2.73 - 2.97 (m, 2 H) 3.19 - 3.35 (m, 2 H) 3.40 - 3.49 (m, 1 H) 4.10 - 4.15 (m, 2 H) 6.69 (s, 1 H) 7.08 - 7.29 (m, 1 H) 7.38 - 7.65 (m, 3 H) 8.13 (d, J=7.72 Hz, 1 H) 8.26 (s, 2 H) 8.34 (s, 1 H) 8.85 (s, 2 H) 9.96 (s, 1 H) |
| 109 | 5-(3-Dimethylcarbamoyl-phenyl)-3-ureido-thiophene-2-carboxylic acid (S)-piperidin-3-ylamide | 416 | 9.89 (s, 1H), 8.98(brs, 2H), 8.20 (s, 1H), 8.09(d, 1H), 7.60(d, 1H), 7.52(s, 1H), 7.48(t, 1H), 7.35(d, 1H), 6.62(brs, 1H), 4.18 (m, 1H), 3.18 (m,2H), 2.78 (m,8H), 1.89-1.42 (m, 4H). |
| 110 | 5-(3,4-Difluoro-phenyl)-3-ureido-thiophene-2-carboxylic acid (S)-piperidin-3-ylamide | 381 | 1.46 - 1.65 (m, 2 H) 1.75 - 1.90 (m, 2 H) 2.69 - 2.89 (m, 2 H) 3.12 - 3.27 (m, 2 H) 3.95 - 4.15 (m, 1 H) 6.63 (s, 1 H) 7.36 - 7.56 (m, 2 H) 7.66 (ddd, J=11.63, 7.68, 1.98 Hz, 1 H) 8.07 (d, J=7.54 Hz, 1 H) 8.19 (s, 1 H) 8.64 (s, 2 H) 9.86 (s, 1 H) |
| 111 | 5-(4-Fluoro-phenyl)-3-ureido-thiophene-2-carboxylic acid (S)-piperidin-3-ylamide | 363 | 1.53 - 1.68 (m, *J*=9.61 Hz, 2 H) 1.88 (s, 2 H) 2.67 - 3.00 (m, 2 H) 3.07 - 3.48 (m, 2 H) 4.05 - 4.28 (m, 1 H) 6.68 (s, 2 H) 7.19 - 7.44 (m, 2 H) 7.56 - 7.76 (m, 2 H) 8.08 (d, *J*=7.54 Hz, 1 H) 8.22 (s, 1 H) 8.73 (s, 2 H) 9.94 (s, 1 H) |
| 112 | 5-(4-Cyanomethyl-phenyl)- 3-ureido-thiophene-2-carboxylic acid (S)-piperidin-3-ylamide | 384 | 9.89 (s, 1H), 9.18(brs, 2H), 8.22 (s, 1H), 8.15(d, 1H), 7.61(d, 2H), 7.39(d, 2H), 6.68(brs, 1H), 4.18 (m, 1H), 4.06(s, 2H), 3.18 (m,2H), 2.78 (m, 2H), 1.87-1.50(m, 4H). |
| 113 | 5-Furan-3-yl-3-ureido-thiophene-2-carboxylic acid (S)-piperidin-3-ylamide | 335 | 1.39 - 1.60 (m, 1 H) 1.66 - 2.01 (m, 3 H) 2.62 - 2.98 (m, 2 H) 3.07 - 3.34 (m, 2 H) 3.96 - 4.13 (m, 1 H) 6.59 (s, 1 H) 6.74 (dd, *J*=1.79, 0.85 Hz, 1 H) 6.91 (dd, *J=*1.88, 0.94 Hz, 1 H) 7.02 (s, 1 H) 7.13 (s, 1 H) 7.93 (d, *J*=7.72 Hz, 1 H) 7.97 (s, 1 H) 9.88 (s, 1H) |
| 114 | 5-(3-Chloro-phenyl)-3-ureido-thiophene-2-carboxylic acid (S)-piperidin-3-ylamide | 379 | 1.44 - 1.71 (m, 2 H) 1.72 - 1.91 (m, 2 H) 2.70 - 2.87 (m, 2 H) 3.08 - 3.28 (m, 2 H) 3.92 - 4.29 (m, 1 H) 6.65 (s, 1 H) 7.62 (t, *J*=7.91 Hz, 1 H) 7.81 (d, *J*=7.54 Hz, 1 H) 7.88 (d, *J*=7.54 Hz, 1 H) 8.05 (s, 1 H) 8.07 - 8.14 (m, *J*=7.72 Hz, 1 H) 8.30 (s, 1 H) 8.58 (s, 2 H) 9.85 (s, 1 H) |
| 115 | 5-(3-Cyano-phenyl)-3-ureido-thiophene-2-carboxylic acid (S)-piperidin-3-ylamide | 370 | 1.43 - 1.66 (m, 2 H) 1.76 - 1.87 (m, 2 H) 2.62 - 2.88 (m, 2 H) 3.20 (dd, *J*=24.96, 11.96 Hz, 4 H) 3.91 - 4.25 (m, 1 H) 6.64 (s, 1 H) 7.40 - 7.47 (m, 2 H) 7.54 (s, 1 H) 7.56 - 7.67 (m, 1 H) 8.06 (d, *J*=7.72 Hz, 2 H) 8.43 - 8.76 (m, 2 H) 9.86 (s, 1 H) |
| 116 | 5-(4-Methoxy-phenyl)-3-ureido-thiophene-2-carboxylic acid (S)-piperidin-3-ylamide | 375 | 1.47 - 1.73 (m, 2 H) 1.80 - 1.98 (m, 2 H) 2.73 - 2.99 (m, 2 H) 3.18 - 3.34 (m, 2 H) 3.74 - 3.87 (m, 3 H) 3.81 (s, 3 H) 4.03 - 4.26 (m, 1 H) 6.66 (dd, 1 H) 7.04 (d, *J*=7.00 Hz, 2 H) 7.57 (d, *J*=7.00 Hz, 2 H) 7.73 (d, *J*=8.67 Hz, 1 H) 8.01 (d, *J*=7.72 Hz, 1 H) 8.15 (s, 1 H) 8.67 (s, 2 H) 9.96 (s, 1 H) |
| 117 | 5-(3-Piperidin-1-yl-phenyl)-3-ureido-thiophene-2-carboxylic acid (S)-piperidin-3-ylamide | 428 | 9.92 (s, 1H), 8.92(brs, 2H), 8.24(s, 1H), 8.18(d, 1H), 7.65-7.20(m, 4H), 6.62(brs, 1H), 4.18 (m, 1H), 3.18 (m,6H), 2.78 (m, 2H), 1.87-1.42(m, 10H). |
| 118 | 5-(4-Methylcarbamoylphenyl)-3-ureido-thiophene-2-carboxylic acid (S)-piperidin-3-ylamide | 402 | 9.89 (s, 1H), 8.95(brs, 2H), 8.50(q, 1H), 8.25 (s, 1H), 8.12(d, 1H), 7.82(d, 2H), 7.62(d, 2H), 6.62(brs, 1H), 4.18 (m, 1H), 3.18 (m,2H), 2.78 (m, 2H), 2.72(d, 3H),1.89-1.42 (m, 4H). |
| 119 | 5-(3,4-Dimethoxy-phenyl)-3-ureido-thiophene-2-carboxylic acid (S)-piperidin-3-ylamide | 405 | 9.98 (s, 1H), 9.12 (brs, 2H), 8.10 (s, 1H), 8.01(d, 1H), 7.15 (d, 1H), 7.08 (s, 1H), 7.02(d, 1H), 6.62(brs, 1H), 4.12 (m, 1H), 3.85(s, 3H), 3.82(s, 3H),3.18 (m,2H), 2.75 (m, 2H), 1.89-1.42 (m, 4H). |
| 120 | 5-(3-Methanesulfonyl-phenyl)-3-ureido-thiophene-2-carboxylic acid (S)-piperidin-3-ylamide | 423 | 9.95(s, 1H), 9.12(brs, 2H), 8.28 (s, 1H), 8.15(d, 1H), 8.08(d, 1H), 7.90(d, 1H), 7.57(t, 1H), 6.68(brs, 1H), 4.18 (m, 1H), 3.18 (m,5H), 2.78 (m, 2H), 1.87-1.50(m, 4H). |
| 121 | 5-(3-Piperidin-1-ylmethyl-phenyl)-3-ureido-thiophene-2-carboxylic acid (S)-piperidin-3-ylamide | 442 | 1.22 - 2.08 (m, 4 H) 2.79 - 3.07 (m, 6 H) 3.13 - 3.70 (m, 2 H) 4.03 - 4.94 (m, 3 H) 7.28 (s, 1 H) 7.45 (s, 1 H) 7.48 -7.61 (m, 2 H) 7.68 (d, *J*=7.35 Hz, 1 H) 7.91 (d, *J*=8.00 Hz, 1 H) 8.15 (d, *J*=7.72 Hz, 1 H) 8.36 (s, 1 H) |
| 122 | 5-(4-Dimethylcarbamoyl-phenyl)-3-ureido-thiophene-2-carboxylic acid (S)-piperidin-3-ylamide | 416 | 9.89 (s, 1H), 9.18(brs, 2H), 8.28 (s, 1H), 8.19(d, 1H), 7.62(d, 2H), 7.48(d, 2H), 4.18 (m, 1H), 3.18 (m,2H), 2.78 (m,8H), 1.87-1.50(m, 4H). |
| 123 | 5-(3-Hydroxy-phenyl)-3-ureido-thiophene-2-carboxylic acid (S)-piperidin-3-ylamide | 361 | 9.96 (s, 1H), 9.18 (brs, 2H), 8.18 (s, 1H), 8.10(d, 1H), 7.24(t, 1H), 7.03 (m, 2H), 6.78(d, 1H), 6.62(brs, 1H), 4.12 (m, 1H), 3.18 (m,2H), 2.78 (m, 2H), 1.89-1.42 (m, 4H). |
| 124 | 5-(3-Amino-phenyl)-3-ureido-thiophene-2-carboxylic acid (S)-piperidin-3-ylamide | 360 | 9.89 (s, 1H), 8.98(brs, 2H), 8.16(s, 1H), 8.09(d, 1H), 7.36-7.22(m, 3H), 6.98(d, 1H), 6.62(brs, 1H), 4.18 (m, 1H), 3.18 (m,2H), 2.78 (m, 2H), 1.87-1.42(m, 4H). |
| 125 | 5-(3-Hydroxymethyl-phenyl)-3-ureido-thiophene-2-carboxylic acid (S)-piperidin-3-ylamide | 375 | 9.89 (s, 1H), 8.95(brs, 2H), 8.20 (s, 1H), 8.05(d, 1H), 7.65(brs, 1H), 7.60(s, 1H), 7.48(d, 1H), 7.38(t, 1H), 7.22(d, 1H), 6.62(brs, 1H), 4.50(s, 2H), 4.18 (m, 1H), 3.18 (m,2H), 2.78 (m, 2H), 1.89-1.42 (m, 4H). |
| 126 | 5-(2,3-Difluoro-phenyl)-3-ureido-thiophene-2-carboxylic acid (S)-piperidin-3-ylamide | 381 | 9.89 (s, 1H), 9.02 (brs, 2H), 8.35 (s, 1H), 8.20(d, 1H), 7.7.68-7.20(m, 3H), 6.68(brs, 1H), 4.15 (m, 1H), 3.18 (m,2H), 2.80 (m, 2H), 1.89-1.50 (m, 4H). |
| 127 | 5-(3-Chloro-4-fluoro-phenyl)-3-ureido-thiophene-2-carboxylic acid (S)-piperidin-3-ylamide | 397 | 1.55 - 1.74 (m, 2 H) 1.80 - 1.98 (m, 2 H) 2.76 - 2.97 (m, 2 H) 3.18 - 3.33 (m, 2 H) 3.99 - 4.27 (m, 1 H) 6.53 - 6.89 (m, 1 H) 7.52 (t, *J*=8.85 Hz, 1 H) 7.64 (ddd, *J*=8.62, 4.57, 2.26 Hz, 1 H) 7.83 (dd, *J*=6.97, 2.26 Hz, 1 H) 8.12 (d, *J*=7.72 Hz, 1 H) 8.26 (s, 1 H) 8.75 (s, 2 H) 9.92 (s, 1 H) |
| 128 | 5-(4-Cyanomethyl-phenyl)-3-ureido-thiophene-2-carboxylic acid (S)-azepan-3-ylamide | 398 | 1.06 - 1.97 (m, 6 H) 3.00 - 3.35 (m, 4 H) 4.10 (s, 2 H) 4.23 - 4.41 (m, 1 H) 7.46 (d, 2 H) 7.67 (d, J=8.29 Hz, 2 H) 7.81 (d, 1 H) 8.07 (d, 1 H) 8.27 (s, 1 H) 8.69 (s, 1 H) |
| 129 | 5-(2,4-Difluoro-phenyl)-3-ureido-thiophene-2-carboxylic acid (S)-piperidin-3-ylamide | 381 | 9.89 (s, 1H), 8.90(brs, 2H), 8.32 (s, 1H), 8.17(d, 1H), 7.78(q, 1H), 7.48 (t, 1H), 7.22(t, 1H), 6.68(brs, 1H), 4.18 (m, 1H), 3.18 (m,2H), 2.78 (m, 2H), 1.89-1.42 (m, 4H). |
| 130 | 5-(3-Piperazin-1-ylmethyl-phenyl)-3-ureido-thiophene-2-carboxylic acid (S)-piperidin-3-ylamide | 443 | 1.45 - 1.67 (m, 2 H) 1.71 - 1.91 (m, 2 H) 2.64 - 3.01 (m, 2 H) 3.06 - 3.35 (m, 2 H) 3.70 - 4.69 (m, 5 H) 7.02 - 7.14 (m, 1 H) 7.20 - 7.78 (m, 5 H) 8.05 (d, *J*=7.72 Hz, 1 H) |
| 131 | 5-[4-(2-Dimethylamino-ethylcarbamoyl)-phenyl]-3-ureido-thiophene-2-carboxylic acid (S)-piperidin-3-ylamide | 459 | 9.96 (s, 1H), 9.05 (brs, 2H), 8.91 (t, 1H), 8.36 (s, 1H), 8.21 (d, 1H), 8.02 (d, 2H), 7.77 (d, 2H), 6.73 (brs, 1H), 4.18 (m, 1H), 3.65 (m, 2H), 3.27 (m, 4H), 2.83 (m, 8H), 1.87 (m, 2H), 1.66 (m, 2H). |
| 132 | 5-(4-Methoxymethyl-phenyl)-3-ureido-thiophene-2-carboxylic acid (S)-piperidin-3-ylamide | 389 | 9.89 (s, 1H), 8.88(brs, 2H), 8.20 (s, 1H), 8.09(d, 1H), 7.50(m, 2H), 7.32(m, 1H), 7.28(d, 1H), 6.62(brs, 1H), 4.40(s, 2H),4.18 (m, 1H), 3.25(s, 3H), 3.18 (m,2H), 2.78 (m, 2H),1.89-1.42 (m, 4H). |
| 133 | 5-(3-Methanesulfonylamino-phenyl)-3-ureido-thiophene-2-carboxylic acid (S)-piperidin-3-ylamide | 438 | 9.89 (s, 1H), 8.82(brs, 2H), 8.16(s, 1H), 8.09(d, 1H), 7.42(s, 1H), 7.32(m, 2H), 7.12(d, 1H), 6.62(brs, 1H), 4.18 (m, 1H), 3.18 (m,2H), 2.95(s, 3H),2.78 (m, 2H), 1.87-1.42(m, 4H). |
| 134 | 5-(4-Dimethylcarbamoyl-phenyl)-3-ureido-thiophene-2-carboxylic acid (S)-azepan-3-ylamide | 430 | 1.14 - 2.16 (m, 6 H) 2.80 - 3.03 (m, 7 H) 3.12 - 3.58 (m, 2 H) 4.01 - 4.31 (m, 1 H) 4.41 - 4.68 (m, 1 H) 7.50 (d, 2 H) 7.69 (d, J=8.29 Hz, 1 H) 7.83 (d, 1 H) 8.13 (d, 1 H) 8.32 (s, 1 H) 8.70 (s, 1 H) |
| 135 | 3-[5-((S)-Piperidin-3-ylcarbamoyl)-4-ureido-thiophen-2-yl]-benzoic acid | 389 | 9.89 (s, 1H), 9.04(brs, 2H), 8.35 (s, 1H), 8.12(m, 2H), 7.82(m, 2H), 7.52(t, 1H), 6.62(brs, 1H), 4.18 (m, 1H), 3.18 (m,2H), 2.78 (m, 2H), 1.89-1.42 (m, 4H). |
| 136 | 5-(3-Amino-phenyl)-3-ureido-thiophene-2-carboxylic acid (S)-azepan-3-ylamide | 374 | 1.19 - 2.06 (m, 6 H) 2.95 - 3.45 (m, 4 H) 4.21 (s, 1 H) 6.53 - 7.37 (m, 4 H) 8.01 (s, 1 H) 8.69 (s, 1 H) 9.92 (s, 1 H) |
| 137 | 5-(3-Acetylamino-phenyl)-3-ureido-thiophene-2-carboxylic acid (S)-piperidin-3-ylamide | 402 | 10.08(s, 1H), 9.89 (s, 1H), 8.88(brs, 2H), 8.20 (s, 1H), 8.09(d, 1H), 8.04(s, 1H), 7.42(d, 1H), 7.30(t, 1H), 7.22(d, 1H), 6.62(brs, 1H), 4.18 (m, 1H), 3.18 (m,2H), 2.78 (m, 2H), ,2.04(s, 3H)1.89-1.42 (m, 4H). |
| 138 | 5-(2-Fluoro-phenyl)-3-ureido-thiophene-2-carboxylic acid (S)-piperidin-3-ylamide | 363 | 1.47 - 1.75 (m, 1 H) 1.74 - 2.21 (m, 3 H) 2.77 - 3.05 (m, 1 H) 3.11 - 3.36 (m, 2 H) 3.79 - 4.58 (m, *J*=78.93 Hz, 2 H) 6.69 (s, 1 H) 7.10 (s, 1 H) 7.22 - 7.57 (m, 3 H) 7.70 - 7.86 (m, 1 H) 7.84 - 8.02 (m, 1 H) 8.15 (d, *J*=7.54 Hz, 1 H) 8.66 (s, 2 H) 9.95 (s, 1 H) |
| 139 | 5-[4-(Acetylamino-methyl)-phenyl]-3-ureido-thiophene-2-carboxylic acid (S)-piperidin-3-ylamide | 416 | 9.97 (s, 1H), 8.77 (brs, 2H), 8.42 (t, 1H), 8.24 (s, 1H), 8.09 (d, 1H), 7.61 (d, 2H), 7.33 (d, 2H), 6.68 (brs, 1H), 4.26 (d, 2H), 4.13 (m, 1H), 3.25 (m, 2H), 2.84 (m, 2H), 1.87 (m, 5H), 1.62 (m, 2H). |
| 140 | 5-[3-(Methanesulfonylamino-methyl)-phenyl]-3-ureido-thiophene-2-carboxylic acid (S)-piperidin-3-ylamide | 452 | 9.98 (s, 1H), 8.84 (brs, 2H), 8.30 (s, 1H), 8.14 (d, 1H), 7.66 (d, 2H), 7.55 (d, 1H), 7.46 (t, 1H), 7.38 (d, 1H), 6.70 (brs, 2H), 4.23 (d, 2H), 4.15 (d, 1H), 3.19 (m, 2H), 2.91 (s,3H), 2.85 (d, 2H), 1.87 (m, 2H), 1.63 (m, 2H). |
| 141 | 5-[3-(Acetylamino-methyl)-phenyl]-3-ureido-thiophene-2-carboxylic acid (S)-piperidin-3-ylamide | 416 | 9.98 (s, 1H), 8.97 (brs, 2H), 8.47 (t, 1H), 8.13 (d, 1H), 7.53 (d, 2H), 7.43 (t, 1H), 7.30 (d, 1H), 6.64 (brs, 1H), 4.29 (d, 2H), 4.14 (m, 1H), 3.27 (m, 2H), 2.84 (m,3H), 1.89 (s, 3H), 1.87 (m, 2H), 1.63 (m, 2H). |
| 142 | 5-[4-(Methanesulfonylamino-methyl)-phenyl]-3-ureido-thiophene-2-carboxylic acid (S)-piperidin-3-ylamide | 452 | 9.97 (s, 1H), 8.80 (brs, 2H), 8.26 (s, 1H), 8.10 (d, 1H), 7.62 (d, 3H), 7.46 (d, 2H), 6.71 (brs, 2H), 4.20 (m, 3H), 3.17 (m, 2H), 2.91 (s,3H), 2.82 (d, 2H), 1.87 (m, 2H), 1.62 (m, 2H). |
| 143 | 5-(3-Methoxy-phenyl)-3-ureido-thiophene-2-carboxylic acid (S)-piperidin-3-ylamide | 375 | 9.89 (s, 1H), 8.92 (brs, 2H), 8.15 (s, 1H), 8.04(d, 1H), 7.28 (d, 1H), 7.20 (d, 1H), 7.02(s, 1H), 6.89(t, 1H), 6.62(brs, 1H), 4.12 (m, 1H), 3.82(s, 3H), 3.18 (m,2H), 2.75 (m, 2H), 1.89-1.42 (m, 4H). |
| 144 | 5-[3-(3-Amino-pyrrolidin-1-ylmethyl)-phenyl]-3-ureido-thiophene-2-carboxylic acid (S)-piperidin-3-ylamide | 443 | 1.42 - 2.23 (m, 11 H) 2.62 - 3.28 (m, 10 H) 3.28 - 3.83 (m, 10 H) 4.02 - 4.24 (m, 2 H) 4.38 (s, 3 H) 7.21 - 7.71 (m, 4 H) 7.75 - 7.93 (m, 2 H) 7.97 - 8.50 (m, 8 H) 9.90 (s, 1 H) 10.64 (s, 1 H) |
| 145 | 5-[4-(Propane-2-sulfonyl)-phenyl]-3-ureido-thiophene-2-carboxylic acid (S)-piperidin-3-ylamide | 451 | 9.94 (s, 1H), 8.71 (brs, 2H), 8.44 (s, 1H), 8.22 (d, 1H), 7.93 (s, 3H), 7.71 (s, 2H), 6.72 (brs, 1H), 4.14 (m, 1H), 3.70 (m,1H), 3.17 (m, 2H), 2.87 (m, 2H), 1.89 (m, 2H), 1.66 (m, 2H), 1.20 (d, 6H). |
| 146 | 5-(2-Chloro-phenyl)-3-ureido-thiophene-2-carboxylic acid (S)-piperidin-3-ylamide | 379 | 1.48 - 1.65 (m, 1 H) 1.72 - 2.04 (m, 3 H) 2.67 - 2.99 (m, 2 H) 2.96 - 3.46 (m, 2 H) 3.89 - 4.25 (m, 1 H) 7.03 (s, 1 H) 7.18 (s, 1 H) 7.33 - 7.47 (m, 2 H) 7.52 - 7.65 (m, 2 H) 8.06 (d, *J*=7.72 Hz, 1 H) 8.16 (s, 1 H) 8.44 - 8.70 (m, 2 H) 8.83 -9.16 (m, 1 H) 9.88 (s, 1 H) |
| 147 | 5-(4-Isobutyl-phenyl)-3-ureido-thiophene-2- carboxylic acid (S)-piperidin-3-ylamide | 401 | 0.80 (s, 3 H) 0.82 (s, 3 H) 1.08 - 1.33 (m, 3 H) 1.45 - 1.70 (m, 1 H) 1.68 - 1.91 (m, 2 H) 2.69 - 2.88 (m, 2 H) 3.08 -3.29 (m, 2 H) 4.03 - 4.12 (m, 2 H) 7.20 (d, *J*=7.91 Hz, 2 H) 7.49 (d, *J*=8.10 Hz, 2 H) 7.57 - 7.68 (m, 1 H) 8.00 (d, *J*=7.72 Hz, 1 H) 8.16 (s, 1 H) |
| 148 | 5-(3-Phenyl-isoxazol-5-yl)-3-ureido-thiophene-2-carboxylic acid (S)-piperidin-3-ylamide | 412 | 1.53 - 1.81 (m, 2 H) 1.81 - 1.92 (m, 2 H) 2.72 - 2.98 (m, 2 H) 3.24 - 3.54 (m, 2 H) 4.16 - 4.44 (m, 1 H) 7.48 - 7.59 (m, 3 H) 7.72 - 7.72 (m, 1 H) 7.82 - 8.05 (m, 2 H) 8.42 (d, *J*=7.54 Hz, 1 H) 8.48 (s, 1 H) 9.24 (s, 2 H) |
| 149 | 5-(3-Hydroxymethyl-phenyl)-3-ureido-thiophene-2-carboxylic acid (S)-azepan-3-ylamide | 389 | 1.05 - 2.00 (m, 6 H) 3.10 - 3.39 (m, 4 H) 4.19 (s, 2 H) 4.33 - 4.41 (m, 1 H) 7.50 (d, 2 H) 7.60 (d, 2 H) 7.81 (d, 1 H) 8.07 (d, 1 H) 8.27 (s, 1 H) 8.69 (s, 1 H) |
| 150 | 5-(4-Dimethylamino-phenyl)-3-ureido-thiophene-2-carboxylic acid (S)-piperidin-3-ylamide | 388 | 9.92 (s, 1H), 8.92(brs, 2H), 8.01(s, 1H), 7.88(d, 1H), 7.39(d, 2H), 6.72 (d, 2H), 6.62(brs, 1H), 4.18 (m, 1H), 3.18 (m,2H), 2.90(s, 6H),2.78 (m, 2H), 1.87-1.42(m, 4H). |
| 151 | 5-(4-Dimethylamino-phenyl)-3-ureido-thiophene-2-carboxylic acid (S)-azepan-3-ylamide | 402 | 1.32 - 2.03 (m, 6 H) 2.39 (s, 6 H) 2.93 - 3.17 (m, 3 H) 3.78 - 4.34 (m, 2 H) 6.97 - 7.50 (m, 5 H) 8.37 (s, 1 H) |
| 152 | 5-[4-(4-Methyl-piperidine-1-carbonyl)-phenyl]-3-ureido-thiophene-2-carboxylic acid (S)-piperidin-3-ylamide | 470 | 9.97 (s, 1H), 8.88 (brs, 2H), 8.32 (s, 1H), 8.19 (d, 1H), 7.68 (d, 2H), 7.49 (d, 2H), 6.70 (brs, 1H), 4.44 (m, 1H), 4.15 (m, 1H), 3.50-2.98 (m, 6H), 2.82 (m, 2H), 1.87 (m, 2H), 1.66 (m, 4H), 1.10 (m, 2H), 0.92 (d, 3H). |
| 153 | 5-(3-Carbamoyl-phenyl)-3-ureido-thiophene-2-carboxylic acid (S)-azepan-3-ylamide | 402 | 1.40 - 1.87 (m, 6 H) 3.00 - 3.40 (m, 4 H) 4.12 - 4.30 (m, 1 H) 6.51 - 6.75 (m, 1 H) 7.39 - 7.56 (m, 2 H) 7.68 (d, *J*=8.10 Hz, 1 H) 7.80 (d, *J*=7.72 Hz, 1 H) 8.03 (d, *J*=7.54 Hz, 1 H) 8.09 - 8.18 (m, 1 H) 8.63 (s, 1 H) |
| 154 | 5-(3,4-Dichloro-phenyl)-3-ureido-thiophene-2-carboxylic acid (S)-piperidin-3-ylamide | 414 | 9.82 (s, 1H), 9.12 (s, 1H), 8.98(brs, 1H), 8.22 (s, 1H), 8.17(d, 1H), 7.78(s, 1H), 7.70 (d, 1H), 7.50(s, 1H), 6.62(brs, 1H), 4.18 (m, 1H), 3.18 (m,2H), 2.78 (m, 2H), 1.89-1.42 (m, 4H). |
| 155 | 5-(2,4-Dimethoxy-phenyl)-3-ureido-thiophene-2-carboxylic acid (S)-piperidin-3-ylamide | 405 | 9.89 (s, 1H), 8.98 (brs, 2H), 8.15 (s, 1H), 7.90(d, 1H), 7.52 (d, 1H), 6.62 (s, 1H), 6.58(d, 1H), 6.50(brs, 1H), 4.08 (m, 1H), 3.82(s, 3H), 3.73(s, 3H), 3.18 (m,2H), 2.75 (m, 2H), 1.89-1.42 (m, 4H). |
| 156 | 5-(2,3,4-Trimethoxy-phenyl)-3-ureido-thiophene-2-carboxylic acid (S)-azepan-3-ylamide | 449 | 1.47 - 2.02 (m, 6 H) 3.06 - 3.34 (m, 4 H) 3.80 (s, 3 H) 3.83 - 3.85 (m, 6 H) 4.17 - 4.35 (m, 1 H) 6.94 (d, J=8.67 Hz, 2 H) 7.40 (d, J=8.85 Hz, 2 H) 7.97 (d, 1 H) 8.25 (s, 1 H) 8.68 (s, 1 H) |
| 157 | 5-(3-Pyrrolidin-1-yl-phenyl)-3-ureido-thiophene-2-carboxylic acid (S)-piperidin-3-ylamide | 414 | 9.97 (s, 1H), 9.18(brs, 2H), 8.18 (s, 1H), 8.09(d, 1H), 7.20(t, 1H), 6.97(d, 1H), 6.69(s, 1H), 6.60(d, 1H), 4.18 (m, 1H), 3.18 (m,6H), 2.78 (m,2H), 1.89-1.42 (m, 8H). |
| 158 | 5-(2,3,4-Trimethoxy-phenyl)-3-ureido-thiophene-2-carboxylic acid (S)-piperidin-3-ylamide | 435 | 9.89 (s, 1H), 9.32 (brs, 2H), 8.20 (s, 1H), 8.02(d, 1H), 7.38(d, 1H), 6.88(d, 1H), 6.62(brs, 1H), 4.12 (m, 1H), 3.80(s, 6H), 3.78(s, 3H), 3.18 (m,2H), 2.75 (m, 2H), 1.89-1.42 (m, 4H). |
| 159 | 5-(3-Acetylamino-phenyl)-3-ureido-thiophene-2-carboxylic acid (S)-azepan-3-ylamide | 416 | 1.30 - 1.85 (m, 6 H) 2.99 - 3.42 (m, 4 H) 4.04 - 4.33 (m, 1 H) 6.51 - 6.75 (m, 1 H) 7.39 - 7.56 (m, 2 H) 7.71 (d, *J*=8.10 Hz, 1 H) 7.83 (d, *J*=7.72 Hz, 1 H) 8.03 (d, *J*=7.54 Hz, 1 H) 8.09 - 8.18 (m, 1 H) 8.63 (s, 1 H) |
| 160 | 5-(3,5-Difluoro-phenyl)-3-ureido-thiophene-2-carboxylic acid (S)-piperidin-3-ylamide | 381 | 9.89 (s, 1H), 9.19(brs, 2H), 8.32 (s, 1H), 8.30(d, 1H), 7.28(m, 3H), 6.62(brs, 1H), 4.18 (m, 1H), 3.18 (m,2H), 2.78 (m, 2H), 1.89-1.42 (m, 4H). |
| 161 | 5-(4-tert-Butyl-phenyl)-3-ureido-thiophene-2-carboxylic acid (S)-azepan-3-ylamide | 414 | 1.26-1.33 (m, 6 H) 1.36 - 1.38 (m, 3 H) 1.44 - 2.07 (m, 6 H) 2.98 - 3.34 (m, 2 H) 3.47 - 3.72 (m, 1 H) 4.19 - 4.31 (m, 1 H) 4.32 - 4.51 (m, 1 H) 7.76 (s, 1 H) 7.83 - 7.89 (m, 1 H) 7.91 (s, 1 H) |
| 162 | 5-(1H-Indol-2-yl)-3-ureido-thiophene-2-carboxylic acid (S)-azepan-3-ylamide | 398 | 1.24 - 2.31 (m, 6 H) 2.84 - 4.29 (m, 4 H) 4.39 - 4.81 (m, 1 H) 7.30 (s, 2 H) 7.42 (s, 1 H) 7.52 (d, 1 H) 7.68 (d, 1 H) 7.88 - 7.99 (m, 2 H) 8.18 (d, 1 H) 8.26 (s, 1 H) |
| 163 | 5-(3-Chloro-phenyl)-3-ureido-thiophene-2-carboxylic acid (S)-azepan-3-ylamide | 394 | 1.24 - 2.02 (m, 6 H) 3.59 (s, 4 H) 3.79 - 3.97 (m, 1 H) 7.42 - 7.53 (m, 2 H) 7.60 (d, *J*=8.48 Hz, 1 H) 7.68 (d, *J*=8.48 Hz, 2 H) 7.92 - 7.98 (m, 1 H) 8.06 (s, 1 H) |
| 164 | 5-(2,5-Dimethoxy-phenyl)-3-ureido-thiophene-2-carboxylic acid (S)-piperidin-3-ylamide | 405 | 9.89 (s, 1H), 8.92(brs, 2H), 8.28 (s, 1H), 8.01(d, 1H),7.12 (m, 2H), 6.98(d, 1H), 6.62(brs, 1H), 4.12 (m, 1H), 3.85(s, 3H), 3.73(s, 3H), 3.22 (m,2H), 2.82 (m, 2H), 1.959-1.56 (m, 4H). |
| 165 | 5-(3,5-Difluoro-phenyl)-3-ureido-thiophene-2-carboxylic acid (S)-azepan-3-ylamide | 395 | 1.12 - 2.03 (m, 6 H) 2.72 - 3.19 (m, 3 H) 3.57 - 3.86 (m, 1 H) 4.20 - 4.71 (m, 1 H) 7.10 - 7.25 (m, 4 H) 7.53 (d, *J*=7.00 Hz, 1 H) |
| 166 | 5-(3-Fluoro-phenyl)-3-ureido-thiophene-2-carboxylic acid (R)-piperidin-3-ylamide | 361.1 | 1.46 - 1.48 (m, 2 H) 1.88 - 1.90 (m, 2 H) 2.74 - 2.76 (m, 2 H) 3.16 - 3.18 (m, 2 H) 4.04 - 4.07 (m, 1 H) 6.58 - 6.61 (br s, 1H) 7.18 - 7.18 (m, 1 H) 7.33 - 7.34 (m, 3 H) 8.09 (d, 1 H) 8.27 (s, 1 H) 8.87 - 8.89 (m, 2 H) 9.83 (s, 1 H). |
| 167 | 5-Benzofuran-2-yl-3-ureido-thiophene-2-carboxylic acid (S)-piperidin-3-ylamide | 385 | 1.44 - 1.70 (m, 1 H) 1.72 - 1.98 (m, 2 H) 2.72 - 2.93 (m, 2 H) 3.10 - 3.22 (m, 2 H) 3.79 - 4.33 (m, 1 H) 6.67 (s, 1 H) 7.33 - 7.38 (m, 1 H) 7.70 - 7.74 (m, 1 H) 7.80 - 7.86 (m, 1 H) 7.93 (s, 1 H) 8.09 (d, *J*=7.54 Hz, 1 H) 8.21 (s, 1 H) 8.37 --8.73 (m, 2 H) 9.67 - 10.06 (m, 1 H) |
| 168 | 5-(5-Fluoro-2-methoxy-phenyl)-3-ureido-thiophene-2-carboxylic acid (S)-azepan-3-ylamide | 407 | 1.40 - 1.99 (m, 6 H) 2.89 - 3.26 (m, 3 H) 3.64 - 3.83 (m, 1 H) 3.94 (s, 3 H) 4.44 - 4.79 (m, 1 H) 7.11 - 7.31 (m, 3 H) 7.36 - 7.51 (m, 2 H) 7.92 (d, *J*=2.83 Hz, 1 H) 8.35 (s, 1 H) |
| 169 | 5-(2-Cyano-phenyl)-3-ureido-thiophene-2-carboxylic acid (S)-piperidin-3-ylamide | 370 | 9.89 (s, 1H), 9.19(brs, 2H), 8.10 (d, 1H), 7.99(s, 1H), 7.88(s, 1H), 7.50(m, 3H), 6.62(brs, 1H), 4.18 (m, 1H), 3.18 (m,2H), 2.78 (m, 2H), 1.89-1.42 (m, 4H). |
| 170 | 5-[(E)-2-(4-Chloro-phenyl)-vinyl]-3-ureido-thiophene-2-carboxylic acid (S)-piperidin-3-ylamide | 405 | 1.40 - 1.52 (m, 2 H) 1.64 - 1.88 (m, 2 H) 2.65 - 2.90 (m, 2 H) 3.10 - 3.36 (m, 2 H) 3.88 - 4.10 (m, 1 H) 6.99 (d, 1 H) 7.19 (d, 1 H) 7.35 (s, 1 H) 7.65 (d, 2 H) 7.93 (d, 2 H) 7.98 (s, 1 H) 8.61 (s, 2 H) 9.86 (s, 1 H) |
| 171 | 5-((E)-Styryl)-3-ureido-thiophene-2-carboxylic acid (S)-piperidin-3-ylamide | 371 | 1.46 - 1.64 (m, 2 H) 1.67 - 1.94 (m, 2 H) 2.60 - 2.90 (m, 2 H) 3.11 - 3.34 (m, 2 H) 3.85 - 4.18 (m, 1 H) 6.94 (d, *J*=16.20 Hz, 1 H) 7.20 - 7.28 (m, 1 H) 7.31 (d, *J*=5.84 Hz, 2 H) 7.45 (d, *J*=16.20 Hz, 1 H) 7.55 (d, *J*=7.35 Hz, 2 H) 7.94 (d, *J*=7.54 Hz, 1 H) 7.98 (s, 1 H) 8.61 (s, 2 H) 9.86 (s, 1 H) |
| 172 | 5-(1H-Pyrrol-2-yl)-2-ureido-thiophene-3-carboxylic acid (S)-azepan-3-ylamide | 348 | 11.18 (s, 1H), 10.77 (s, 1H), 8.81 (br s, 2H), 8.06 (d, 1H), 7.30 (s, 1H), 6.98 (br s, 2H), 6.74 (s, 1H), 6.20 (s, 1H), 6.07 (dd, 1H), 4.27 (m, 1H), 3.34 (m, 1H), 3.18 (m, 3H), 1.98 (m, 1H), 1.80 (m, 4H), 1.58 (m, 1H) |
| 173 | 5-Phenyl-3-ureido-thiophene-2-carboxylic acid (S)-piperidin-3-ylamide | 345 | 10.02 (s, 1H), 9.39 (brs, 2H), 8.25 (s, 1H), 8.18(d, 1H), 7.6 (d, 2H), 7.58-7.31 (m, 3H), 4.19 (m, 1H), 3.34-3.12 (m,2H), 2.83 (m, 2H), 1.90-1.51 (m, 4H). |

### Example 174

### 3-Ureido-thiophene-2-carboxylic acid (S)-azepan-3-ylamide

**methyl 3-[(aminocarbonyl)amino]thiophene-2-carboxylate.** A stirred solution of methyl 3-({[(trichloroacetyl)amino]carbonyl}amino)thiophene-2-carboxylate [prepared as in Example 13] (2.0g, 5.8 mmol) in anhydrous methanol (30 mL) was purged with dry ammonia for 20 mins. After stirring for extra 10 mins at rt, precipitation was observed and the product was isolated by filtration (100% yield). LC/MS (ES, M+H=201).

***tert*-butyl (3*S*)-3-[({3-[(aminocarbonyl)amino]-2-thienyl}carbonyl)amino]azepane-1-carboxylate.** To a solution of methyl 3-[(aminocarbonyl)amino]thiophene-2-carboxylate (500 mg, 2.5 mmol) in anhydrous THF (20 mL) was added via cannula a solution of [Me₃Al and *tert-*butyl (3*S*)-3-aminoazepane-1-carboxylate] in THF (preformed by the careful addition of Me₃Al (2.0M in hexanes, 5.0 mL, 10 mmol) to a solution of and *tert*-butyl (3*S*)-3-aminoazepane-1-carboxylate (2.14g, 10 mmol) in 10 mL of THF at 0°C and subsequently stirring at rt for 10 mins). The resulting yellow solution was stirred at rt for 10 h. The reaction mixture was cooled to 0°C and a 10% aqueous solution of potassium tartarate was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave 62% of the title compound as an off- white solid. LC/MS (ES, M+H=383).

**3-Ureido-thiophene-2-carboxylic acid (S)-azepan-3-ylamide.** A solution of *tert*-butyl (3*S*)-3-[({3-[(aminocarbonyl)amino]-2-thienyl}carbonyl)amino]azepane-1-carboxylate (100 mg, 0.26 mmol) in 4.0N HCl in 1,4-dioxane (10 mL) was stirred for 30 mins at rt. The cloudy solution was diluted with dry methanol and the solvents were removed under vacuum. The residue was dissolved in H₂O and placed in a lyophilizer to yield the title compound as white solid. ¹H NMR (d₆-DMSO, 1.30 - 2.00 (m, 6 H) 2.89 - 3.24 (m, 4 H) 3.99 - 4.20 (m, 1 H) 6.92 (d, 1 H) 7.38 (d,1 H)). LC/MS (ES, M+H=283).

### Example 175

### 5-Phenyl-3-ureido-thiophene-2-carboxylic acid (S)-azepan-3-ylamide

**methyl 5-phenyl-3-({[(trichloroacetyl)amino]carbonyl}amino)thiophene-2-carboxylate.** To a stirred solution of methyl 3-amino-5-phenylthiophene-2-carboxylate (1.9 g, 8.0 mmol) in anhydrous THF (16 mL) was added trichloroacetyl isocyanate (0.95 mL, 8.0 mmol) slowly over a period of 15 min. After the addition was complete, a precipitate formed and the reaction stirred for an additional 1h. The desired product was obtained by filtration to give the title compound (99%) as white solid. The product was used in the next step without any further purification. LC/MS (ES, M+H=421).

**5-Phenyl-3-ureido-thiophene-2-carboxylic acid (S)-azepan-3-ylamide.** To a solution of methyl 5-phenyl-3-({[(trichloroacetyl)amino]carbonyl}amino)thiophene-2-carboxylate (200 mg, 0.46 mmol) in anhydrous THF (2.0 mL) was added via cannula a solution of [Me₃Al and (3*S*)-azepan-3-amine] in THF (preformed by the careful addition of Me₃Al (2.0M in hexanes, 1.65 mL, 2.30 mmol) to a solution of (3*S*)-azepan-3-amine (262 mg, 2.30 mmol) in 2.0 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN gave (50%) of the title compound as an off- white solid. The hydrochloride salt was obtained by dissolving the title compound in a solution of 4.0N HCl in 1, 4-dioxane (10 mL) and stirring for 30 mins at rt. The cloudy solution was diluted with dry methanol and the solvents were removed under vacuum. The residue was dissolved in H₂O and placed in a lyophilizer to yield the title compound as white solid. ¹H NMR (300 MHz, DMSO-d₆) δ ppm 1.23 - 2.00 (m, 6 H) 3.48-3.60 (m, 4 H) 3.74-3.79 (m, 1 H) 7.40 - 7.53 (m, 2 H) 7.82 - 7.95 (m, 4H); LC/MS (ES, M+H=359).

### Example 176

### 5-(4-Chloro-phenyl)-3-ureido-thiophene-2-carboxylic acid (S)-piperidin-3-ylamide

**methyl 5-(4-chlorophenyl)-3-({[(trichloroacetyl)amino]carbonyl}amino)thiophene-2-carboxylate.** To a stirred solution of methyl 3-amino-5-(4-chlorophenyl)thiophene-2-carboxylate (3.8 g, 14.3 mmol) in anhydrous THF (28 mL) was added trichloroacetyl isocyanate (1.7 mL, 14.3 mL) slowly over a period of 5 min. After the addition was complete, a precipitate formed and the reaction stirred for an additional 1h. The desired product was obtained by filtration to give the title compound (99%) as white solid. The product was used in the next step without any further purification LC/MS (ES, M+H=456).

**methyl 3-[(aminocarbonyl)amino]-5-(4-chlorophenyl)thiophene-2-carboxylate.** A stirred solution of methyl 5-(4-chlorophenyl)-3-({[(trichloroacetyl)amino]carbonyl}amino)thiophene-2-carboxylate (6.2g, 14.3 mmol) in anhydrous methanol (30 mL) was purged with dry ammonia for 20 mins. After stirring for extra 10 mins at rt, precipitation was observed and the product was isolated by filtration (100% yield). LC/MS (ES, M+H=311).

***tert*-butyl (3*S*)-3-({[3-[(aminocarbonyl)amino]-5-(4-chlorophenyl)-2-thienyl]carbonyl}amino)piperidine-1-carboxylate.** To a solution of methyl 3-[(aminocarbonyl)amino]-5-(4-chlorophenyl)thiophene-2-carboxylate (1.0 g, 3.2 mmol) in anhydrous THF (20 mL) was added via cannula a solution of [Me₃Al and *tert*-butyl (3*S*)-3-aminopiperidine-1-carboxylate] in THF (preformed by the careful addition of Me₃Al (2.0M in hexanes, 10 mL, 20 mmol) to a solution of *tert*-butyl (3*S*)-3-aminopiperidine-1-carboxylate (1.94g, 9.7 mmol) in 10 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as a white solid. LC/MS (ES, M+H=479).

**5-(4-Chloro-phenyl)-3-ureido-thiophene-2-carboxylic acid (S)-piperidin-3-ylamide.** A solution of *tert*-butyl (3*S*)-3-({[3-[(aminocarbonyl)amino]-5-(4-chlorophenyl)-2-thienyl]carbonyl}amino)piperidine-1-carboxylate (300 mg, 0.6 mmol) in 4.0N HCl in 1, 4-dioxane (5 mL) was stirred for 30 mins at rt. The cloudy solution was diluted with dry methanol and the solvents were removed under vacuum. The residue was dissolved in H₂O and placed in a lyophilizer to yield the title compound as white solid. ¹H NMR (300 MHz, DMSO-d₆) δ ppm 1.48 - 1.69 (m, 2 H) 1.71 - 1.88 (m, 2 H) 2.59 - 2.89 (m, 2 H) 3.88 - 4.09 (m, 2 H) 7.46 (d, *J*=8.10 Hz, 2 H) 7.64 (d, *J*=8.29 Hz, 2 H) 8.24 (s, 1 H) 9.04 (d, *J*=7.72 Hz, 1 H) 9.17 (s, 1 H) 9.23 - 9.38 (m, 2 H); LC/MS (ES, M+H=379).

### Example 177

### 5-Benzyl-2-ureido-thiophene-3-carboxylic acid (S)-azepan-3-ylamide

**methyl 5-benzyl-2-({[(trichloroacetyl)amino]carbonyl}amino)thiophene-3-carboxylate.** To a stirred solution of methyl 2-amino-5-benzylthiophene-3-carboxylate (1.0 g, 4.0 mmol) in anhydrous THF (10 mL) was added trichloroacetyl isocyanate (0.5 mL, 4.0 mmol) slowly over a period of 5 min. After the addition was complete, a precipitate formed and the reaction stirred for an additional 1h. The desired product was obtained by filtration to give the title compound (99%) as an off-white solid. The product was used in the next step without any further purification. LC/MS (ES, M+H=435).

**5-Benzyl-2-ureido-thiophene-3-carboxylic acid (S)-azepan-3-ylamide.** To a solution of methyl 5-benzyl-2-({[(trichloroacetyl)amino]carbonyl}amino)thiophene-3-carboxylate (1.5 g, 3.5 mmol) in anhydrous THF (8.0 mL) was added via cannula a solution of [Me₃Al and (3*S*)-azepan-3-amine] in THF (preformed by the careful addition of Me₃Al (2.0M in hexanes, 3.6 mL, 7.1 mmol) to a solution of (3*S*)-azepan-3-amine (808 mg, 7.1 mmol) in 8.0 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as an off- white solid. The hydrochloride salt was obtained by dissolving the title compound in a solution of 4.0N HCl in 1, 4-dioxane (10 mL) and stirring for 30 mins at rt. The cloudy solution was diluted with dry methanol and the solvents were removed under vacuum. The residue was dissolved in H₂O and placed in a lyophilizer to yield the title compound as white solid. ¹H NMR (300 MHz, DMSO-d₆) δ ppm 0.90 - 1.89 (m, 6 H) 2.90 - 3.65 (m, 4 H) 3.76 - 3.87 (m, 1 H) 6.54 (s, 2 H) 7.14 - 7.41 (m, 6 H); LC/MS (ES, M+H=473).

### Example 178

### 2-[(aminocarbonyl)amino]-N-[(3R)-1-azabicyclo[2.2.2]oct-3-yl]-5-phenylthiophene-3-carboxamide

**methyl 2-[(aminocarbonyl)amino]-5-phenylthiophene-3-carboxylate.** A stirred solution of methyl 5-phenyl-2-({[(trichloroacetyl)amino]carbonyl}amino)thiophene-3-carboxylate (63.44 g, 150 mmol) in anhydrous methanol (300 mL) was purged with dry ammonia for 20 mins. After stirring for 2 h at rt, precipitation was observed and the product was isolated by filtration (65 g). ¹H NMR (400 MHz, DMSO-d₆) δ ppm 3.84 (s, 3 H) 7.15 (bs, 1 H), 7.26 (t, 1 H) 7.38 (t, 2 H) 7.43 (s, 1 H), 7.6(d, 2h), 10.14 (s, 1 H). LC/MS (ES, M+H=277).

**2-[(aminocarbonyl)amino]-*N*-[(3*R*)-1-azabicyclo[2.2.2]oct-3-yl]-5-phenylthiophene-3-carboxamide.** To a solution of methyl 2-[(aminocarbonyl)amino]-5-phenylthiophene-3-carboxylate (1.37 g, 5 mmol) in anhydrous THF (15 mL) was added via cannula a solution of [Me₃Al and (3*R*)-quinuclidin-3-amine] in THF (preformed by the careful addition of Me₃Al (2.0M in hexanes, 5 mL, 10 mmol) to a solution of (3R)-quinuclidin-3-amine (1.0 g, 5 mmol) in 10 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% MeCN-H₂O) gave 1.2 g (70%) of the title compound as white solid. 1H NMR (300 MHz, DMSO-d₆) δ ppm 1.60 - 1.74 (m, 1 H) 1.75 - 1.94 (m, 2 H) 1.96 - 2.11 (m, 1 H) 2.11 - 2.25 (m, 1 H) 2.90 - 3.35 (m, 4 H) 3.61 (t, *J*=11.49 Hz, 1 H) 4.10 - 4.45 (m, 1 H) 6.96 (s, 1 H) 7.19 (t, *J*=7.35 Hz, 1 H) 7.33 (t, *J*=7.63 Hz, 2 H) 7.50 (d, *J*=7.35 Hz, 2 H) 7.79 (s, 1 H) 8.19 (d, *J*=5.65 Hz, 1 H); LC/MS (ES, M+H=371).

The following examples **179-212** are synthesized in an analogous manner to that of Example 15 using the appropriate starting materials. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 179 | 5-Phenyl-2-ureido-thiophene-3-carboxylic acid (2-amino-ethyl)-amide | 305 | 11.0 (s, 1H), 7.8 (d, 1H), 7.6(t, 2H), 7.4 (m, 3H), 7.25 (m, 2H), 7.0(s, 1H), 2.9(t, 2H), 2.0(t, 1H), 1.8(t, 2H) |
| 180 | 5-Phenyl-2-ureido-thiophene-3-carboxylic acid (R)-azepan-3-ylamide 1815-Phenyl-2-ureido-thiophene-3-carboxylic acid ((S)-1-piperidin-3-ylmethyl)-amide | 359 | CDCl₃; 11.37 (br s, 1H), 7.56 (d, 2H), 7.35 (t, 2H), 7.24 (m, 1H), 7.14 (s, 1H), 7.04 (d, 1H), 5.27 (br s, 2H), 4.19 (m, 1H), 2.97 (m, 3H), 2.84 (m, 1H), 1.78 (m, 5H), 1.56 (m, 1H) 10.00 (s, 1H), 8.79(brs,1H), 8.42(m, 2H), 7.81(s, 1H), 7.54(d, 2H), 7.39(d, 2H), 7.26(t, 1H), 7.01(brs, 1H), 3.39-3.10 (m, 4H), 2.82-2.52(m, 2H), 2.00 (m, 1H), 1.79(m, 2H), 1.24(m, 2H). |
| 182 | 5-Phenyl-2-ureido-thiophene-3-carboxylic acid ((R)-1-piperidin-3-ylmethyl)-amide | 359 | 1.41 - 2.04 (m, 4 H) 2.67 - 2.92 (m, 2 H) 3.30 - 3.61 (m, 3 H) 4.09 - 4.30 (m, 2 H) 7.18 (s, 1 H) 7.32 - 7.47 (m, 1 H) 7.59 (d, J=7.35 Hz, 2 H) 7.64 - 7.76 (m, 2 H) |
| 183 | 5-(3,4-Dimethoxy-phenyl)-2-ureido-thiophene-3-carboxylic acid piperidin-4-ylamide | 405 | |
| 184 | 5-Phenyl-2-ureido-thiophene-3-carboxylic acid ((S)-1-methyl-azepan-3-yl)-amide | 373 | CDCl₃; 11.36 (br s, 1H), 7.57 (d, 2H), 7.37 (t, 2H), 7.27 (m, 1H), 7.23 (m, 1H), 7.12 (s, 1H), 5.27 (br s, 2H), 4.21 (m, 1H), 2.84 (m, 2H), 2.64 (m, 1H), 2.46 (s, 3H), 2.43 (m, 1H), 1.78 (m, 6H), |
| 185 | 5-Phenyl-2-ureido-thiophene-3-carboxylic acid (5-oxo-[1,4]diazepan-6-yl)-amide | 374 | 10.85 (s, 1H), 9.40 (br s, 1H), 9.21 (br s, 1H), 8.43 (d, 1H), 8.24 (dd, 1H), 7.81 (s, 1H), 7.56 (d, 2H), 7.42 (t, 2H), 7.27 (t, 1H), 7.05 (br s, 2H), 5.01 (t, 1H), 3.58 (m, 1H), 3.45 (m, 2H), 3.34 (m, 2H), 3.05 (m, 1H) |
| 186 | 5-Phenyl-2-ureido-thiophene-3-carboxylic acid (2-dimethylamino-ethyl)-amide | 333 | 10.9 (s, 1H), 10.25 (br s, 1H), 8.7 (s, 1H), 7.9 (s, 1H), 7.5 (d, 2H), 7.3 (t, 3H), 7.25 (t, 2H), 7.0 (s, 2H), 3.6 (m, 2H), 3.3 (m, 2H), 2.8 (s, 6H) |
| 187 | 5-(3,4-Dimethoxy-phenyl)-2-ureido-thiophene-3-carboxylic acid (S)-azepan-3-ylamide | 419 | |
| 188 | 5-Phenyl-2-ureido-thiophene-3-carboxylic acid [(S)-1-(2-amino-ethyl)-azepan-3-yl]-amide | 402 | 1.35 - 2.05 (m, 6 H) 2.98 - 3.33 (m, 4 H) 3.42 - 3.85 (m, 4 H) 4.00 - 4.62 (m, 1 H) 7.02 (t, *J*=7.25 Hz, 1 H) 7.16 (t, *J*=7.35 Hz, 2 H) 7.33 (d, *J*=7.35 Hz, 2 H) 7.98 - 8.63 (m, 2 H) 10.68 (d, *J*=6.03 Hz, 1 H) 11.03 (s, 1 H) |
| 189 | 5-(3-Nitro-phenyl)-2-ureido-thiophene-3-carboxylic acid piperidin-4-ylamide | 390 | |
| 190 | 5-Phenyl-2-ureido-thiophene-3-carboxylic acid ((S)-1-methyl-piperidin-3-yl)-amide | 359 | CDCl₃; 11.36 (br s, 1H), 7.58 (d, 2H), 7.36 (t, 2H), 7.26 (m, 2H), 6.78 (br s, 1H), 5.38 (br s, 2H), 4.21 (m, 1H), 2.84 (m, 2H), 2.64 (m, 1H), 2.46 (s, 3H), 2.43 (m, 1H), 1.78 (m, 6H), |
| | 1915-Phenyl-2-ureido-thiophene-3-carboxylic acid (R)-piperidin-3-ylamide | 343 | 1.52 - 1.78 (m, 2 H) 1.79 - 2.06 (m, 2 H) 2.80 - 3.02 (m, 2 H) 3.07 - 3.24 (m, 1 H) 3.20 - 3.37 (m, 1 H) 3.94 - 4.53 (m, 1 H) 7.03 (s, 1 H) 7.17 - 7.30 (m, 1 H) 7.38 (t, *J*=7.54 Hz, 2 H) 7.55 (d, *J*=8.10 Hz, 2 H) 7.96 (s, 1 H) 8.32 (d, *J*=7.16 Hz, 1 H) 8.68 - 9.57 (m, 2 H) 10.90 (s, 1 H). |
| 192 | 5-Phenyl-2-ureido-thiophene-3-carboxylic acid (1-ethyl-piperidin-3-yl)-amide | 373 | 11.04 (s, 1H), 7.85 (d, 1H), 7.82 (s, 1H), 7.54 (d, 2H), 7.39 (t, 2H), 7.24 (t, 1H), 6.98 (br s, 2H), 3.91 (m, 1H), 2.95 (dd, 1H), 2.76 (dd, 1H), 2.35 (m, 2H), 1.82 (m, 3H), 1.71 (m, 1H), 1.50, (dt, 1H), 1.3 (dq, 1H), 0.99 (t, 3H) |
| 193 | 5-Phenyl-2-ureido-thiophene- 3-carboxylic acid [(S)-1-(2-hydroxy-ethyl)-azepan-3-yl]-amide | 403 | 1.32 - 2.09 (m, 7 H) 3.15 - 3.48 (m, 6 H) 3.82 (m, 2 H) 4.10 - 4.39 (m, 1 H) 6.97 (s, 2 H) 7.34 (t, *J*=7.72 Hz, 2 H) 7.48 (d, *J*=7.35 Hz, 2 H) 7.69 (d, *J*=6.97 Hz, 1 H) 8.17 (dd, *J*=18.56, 7.25 Hz, 1 H) 9.57 (s, 1 H) 10.79 (d, *J*=17.71 Hz, 1 H) |
| 194 | 5-Phenyl-2-ureido-thiophene-3-carboxylic acid (morpholin-2-ylmethyl)-amide | 361 | 0.75 - 1.04 (m, 2 H) 1.19 - 1.51 (m, 4 H) 2.67 - 2.82 (m, 2 H) 2.88 - 3.00 (m, 2 H) 3.08 - 3.51 (m, 4 H) 4.07 - 4.28 (m, 1 H) 7.23 - 7.28 (m, 1 H) 7.39 (t, J=7.44 Hz, 2 H) 7.54 (d, J=7.54 Hz, 2 H) 7.90 (s, 1 H) 8.55 (s, 1 H) 9.61 (s, 2 H) 10.96 (s, 1 H) |
| 195 | 5-Phenyl-2-ureido-thiophene-3-carboxylic acid ((S)-1-ethyl-azepan-3-yl)-amide | 387 | 11.04 (s, 1H), 7.83 (s, 1H), 7.79 (d, 1H), 7.55 (d, 2H), 7.39 (t, 2H), 7.24 (dt, 1H), 6.98 (br s, 2H), 4.04 (m, 1H), 2.75 (dd, 1H), 2.61 (m, 3H), 2.53 (m, 2H), 1.84 (m, 1H), 1.69 (m, 3H), 1.54, (m, 2H), 0.97 (t, 3H) |
| 196 | 5-(3-Trifluoromethyl-phenyl)-2-ureido-thiophene-3-carboxylic acid piperidin-4-ylamide | 413 | |
| 197 | 5-Phenyl-2-ureido-thiophene-3-carboxylic acid (piperidin-2-ylmethyl)-amide | 359 | 1.72 - 1.93 (m, 2 H) 3.24 - 3.40 (m, 2 H) 3.65 - 3.81 (m, 2 H) 4.15 - 4.37 (m, 1 H) 4.61 - 4.80 (m, 1 H) 6.99 - 7.12 (m, 2 H) 7.24 (s, 2 H) 7.53 (d, *J*=7.00 Hz, 2 H) 7.65 (d, *J*=7.00 Hz, 2 H) 8.07 (d, *J*=9.61 Hz, 1 H) 8.16 (s, 1 H) |
| 198 | 5-Phenyl-2-ureido-thiophene-3-carboxylic acid (3-methyl-6-oxo-piperidin-3-yl)-amide | 373 | 2.02 - 2.19 (m, 1 H) 2.43 - 2.59 (m, 2 H) 2.85 - 3.03 (m, 1 H) 3.21 - 3.41 (m, 1 H) 3.47 - 3.67 (m, 1 H) 4.21 - 4.70 (m, 1 H) 7.40 - 7.53 (m, 1 H) 7.54 - 7.68 (m, 2 H) 7.73 (t, *J*=7.35 Hz, 2 H) 8.02 (s, 1 H) 8.27 (d, *J*=7.16 Hz, 1 H) |
| 199 | 5-(3,4-Dihydroxy-phenyl)-2-ureido-thiophene-3-carboxylic acid piperidin-4-ylamide | 377 | |
| 200 | 2-[(aminocarbonyl)amino]-N-[(3R)-1-azabicyclo[2.2.2]oct-3-yl]-5-phenylthiophene-3-carboxamide | 371 | 1.60 - 1.74 (m, 1 H) 1.75 - 1.94 (m, 2 H) 1.96 - 2.11 (m, 1 H) 2.11 - 2.25 (m, 1 H) 2.90 - 3.35 (m, 4 H) 3.61 (t, *J*=11.49 Hz, 1 H) 4.10 - 4.45 (m, 1 H) 6.96 (s, 1 H) 7.19 (t, *J*=7.35 Hz, 1 H) 7.33 (t, *J*=7.63 Hz, 2 H) 7.50 (d, *J*=7.35 Hz, 2 H) 7.79 (s, 1 H) 8.19 (d, *J*=5.65 Hz, 1 H) |
| 201 | 4-{[5-(3,4-Dimethoxy-phenyl)-2-ureido-thiophene-3-carbonyl]-amino}-piperidine-1-carboxylic acid tert-butyl ester | 505 | |
| 202 | 5-Phenyl-2-ureido-thiophene-3-carboxylic acid (2-hydroxy-ethyl)-amide | 306 | d₃-MeOD; 7.55 (m, 3H), 7.35 (m, 2H), 7.2 (m, 1H) 4.2 (t, 2H) 3.6 (t, 2H), 1.2 (s, 2H) |
| 203 | 5-Phenyl-2-ureido-thiophene-3-carboxylic acid (2-oxo-azepan-3-yl)-amide | 373 | 1.27 (d, *J*=12.62 Hz, 1 H) 1.44 - 2.17 (m, 5 H) 2.88 -3.47 (m, 2 H) 4.62 (t, 1 H) 7.25 (t, *J*=7.25 Hz, 1 H) 7.40 (t, *J*=7.72 Hz, 2 H) 7.59 (d, *J*=7.72 Hz, 2 H) 7.84 (d, 1 H) 8.10 (d, *J*=7.16 Hz, 1 H) 11.01 (s, 1 H) |
| 204 | [3-((S)-3-Amino-azepane-1-carbonyl)-5-phenyl-thiophen-2-yl]-urea | 359 | 9.71 (s, 1H), 8.20 (m, 3H), 7.55 (s, 1H), 7.36 (m, 2H), 7.26 (m, 2H), 6.88 (br s, 2H), 3.81 (m, 1H), 3.54 (m, 1H), 3.16 (m, 1H), 2.00 (m, 1H), 1.53 (m, 7H) |
| 205 | [3-((R)-3-Amino-azepane-1-carbonyl)-5-phenyl-thiophen-2-yl]-urea | 359 | CDCl₃; 7.52 (d, 2H), 7.34 (t, 2H), 7.23 (t, 1H), 7.06 (s, 1H), 5.85 (br s, 2H), 3.83 (d, 1H), 3.65 (br s, 2H), 3.27 (m, 1H), 3.21 (m, 1H), 1.89 (m, 3H), 1.76 (m, 2H), 1.39 (m, 2H) |
| 206 | [3-(3-Aminomethyl-piperidine-1-carbonyl)-5-phenyl-thiophen-2-yl]-urea | 359 | 1.09 - 1.36 (m, 1 H) 1.52 - 1.69 (m, 1 H) 1.74 - 1.91 (m, 1 H) 1.93 - 2.19 (m, 1 H) 2.55 - 2.87 (m, 2 H) 2.95 - 3.36 (m, 3 H) 3.55 - 3.60 (m, 2 H) 7.00 (s, 3 H) 7.17 (s, 1 H) 7.27 (d, J=7.35 Hz, 1 H) 7.34 (s, 1 H) 7.40 (t, J=7.63 Hz, 2 H) 7.54 (d, J=7.16 Hz, 2 H) 7.82 (s, 1 H) 10.99 (s, 1 H) |
| 207 | 5-methyl-2-ureido-thiophene-3-carboxylic acid (S)-azepan-3-ylamide | 297 | 1.38 - 1.97 (m, 6 H) 2.16 - 2.25 (m, 3 H) 2.95 - 3.25 (m, 2 H) 3.41 (dd, *J*=8.19, 3.86 Hz, 1 H) 3.62 (dd, *J*=9.89, 4.43 Hz, 1 H) 4.10 - 4.44 (m, 1 H) 7.04 (s, 1 H) 8.09 (d, *J*=7.54 Hz, 1 H) 9.26 (s, 1 H) 9.52 (s, 1 H) 10.68 (s, 1 H) |
| 208 | 5-Isopropyl-2-ureido-thiophene-3-carboxylic acid (S)-azepan-3-ylamide | 326 | 1.08 (d, 6H) 1.67 - 2.22 (m, 6 H) 3.10 - 3.40 (m, 3 H) 3.88 - 4.43 (m, 3 H) 6.99(s, 1 H) |
| 209 | 5-Ethyl-2-ureido-thiophene-3-carboxylic acid (S)-azepan-3-ylamide | 311 | 1.17 (t, 3 H) 1.48 - 2.16 (m, 6 H) 2.77 (q, 2 H) 3.19 -3.47 (m, 3 H) 3.94 - 4.46 (m, 2 H) 6.97(s, 1 H); LC/MS (ES, M+H=311). |
| 210 | 5-Phenyl-3-ureido-thiophene-2-carboxylic acid [1,4]oxazepan-6-ylamide | 361 | 3.72 (m, 2 H), 3.93 (m, 4 H), 4.49 (m, 1 H), 7.07 (s, 2 H), 7.27 (t, 1 H), 7.41 (t, 2 H), 7.57 (d, 2 H), 7.75 (s, 1 H), 8.19 (d, 1 H) 9.15 (s, 2 H), 10.84 (s, 1 H) |
| 211 | 5-tert-Butyl-3-ureido-thiophene-2-carboxylic acid (S)-azepan-3-ylamide | 339 | 1.33 (s, 6 H) 1.36 (s, 3 H) 1.44 - 2.07 (m, 6 H) 2.98 -3.34 (m, 2 H) 3.47 - 3.72 (m, 1 H) 4.19 - 4.31 (m, 1 H) 4.32 - 4.51 (m, 1 H) 7.76 (s, 1 H) 7.83 - 7.89 (m, 1 H) 7.91 (s, 1 H) |
| 212 | 5-(4-Chloro-phenyl)-3-ureido-thiophene-2-carboxylic acid (S)-azepan-3-ylamide | 394 | 1.15 - 2.02 (m, 6 H) 3.52 - 4.05 (m, 5 H) 7.48 (d, J=9.98 Hz, 2 H) 7.68 (d, J=8.48 Hz, 2 H) 8.06 (s, 1 H) |

### Example 213

### 5-(4-Chloro-phenyl)-3-[3-(3,4-dimethoxy-phenyl)-ureido]-thiophene-2-carboxylic acid (S)-piperidin-3-ylamide

**3-amino-5-(4-chlorophenyl)thiophene-2-carboxylic acid**. To a stirred solution of methyl 3-amino-5-(4-chlorophenyl)thiophene-2-carboxylate (10.0g, 37.3 mmol) in MeOH (40 mL) was added 50% w/w solution of NaOH (20 mL). The resulting viscous solution was heated to reflux for 2 h whereupon it was cooled to rt. The mixture was treated cautiously with 2N HCl solution until pH∼3. The aqueous phase was washed exhaustively with EtOAc (5x) and dried (MgSO₄). Evaporation gave the title acid as pale yellow solid. The product was used in the next step without any further purification: LC/MS (ES, M+H=254).

***tert*-butyl (3*S*)-3-({[3-amino-5-(4-chlorophenyl)-2-thienyl]carbonyl}amino)piperidine-1-carboxylate.** To a solution of 3-amino-5-(4-chlorophenyl)thiophene-2-carboxylic acid (5.0 g, 19.7 mmol) in dry DMF (40 mL) were added *tert*-butyl (3*S*)-3-aminopiperidine-1-carboxylate (3.95g, 19.7 mmol) and HATU (15.0g, 39.4 mmol) at 0°C. The resulting pale orange solution was stirred for 10 mins at this temperature whereupon *N*,*N*'-diisopropylethyl amine (7.8 mL, 43.3 mmol) was added drop-wise via a syringe. After the addition finished, the resulting orange solution was warmed to rt and stirred overnight. The mixture was partitioned between EtOAc and H₂O and the organic layer was washed with brine, H₂O and dried (MgSO₄). Evaporation gave a yellow oil. Purification by Biotage-Horizon system™ (30%EtOAc→45%EtOAc-hexanes) gave the desired product. ¹H NMR (300 MHz, DMSO-d₆) δ ppm 1.39 (s, 9 H) 1.40 - 1.45 (m, 1 H) 1.46 - 1.62 (m, 1 H) 1.63 -1.73 (m, 1 H) 1.76 - 1.87 (m, 1 H) 2.65 - 2.78 (m, 2 H) 3.68 - 3.82 (m, 3 H) 6.53 (s, 2 H) 6.98 (s, 1 H) 7.31 (d, *J*=6.97 Hz, 1 H) 7.49 (d, *J*=8.00 Hz, 2 H) 7.61 (d, *J*=8.00 Hz, 2 H); LC/MS (ES, M+H=436).

**5-(4-Chloro-phenyl)-3-[3-(3,4-dimethoxy-phenyl)-ureido]-thiophene-2-carboxylic acid (S)-piperidin-3-ylamide.** To a solution of tert-butyl (3*S*)-3-({[3-amino-5-(4-chlorophenyl)-2-thienyl]carbonyl}amino)piperidine-1-carboxylate (1.0 equiv) in THF (0.5 M) was added 3,4-dimethoxy-phenyl isocyanate (1.1 equiv). The resulting light brown solution was stirred at rt for 2 h whereupon the solvent was evaporated. The residue was treated with 4.0N HCl solution in dioxane (0.5 mL) and the resulting cloudy mixture was stirred at rt for 1 h. Evaporation of the solvent followed by purification by Gilson (20%MeCN-H₂O→98%MeCN-H₂O) gave the desired product. ¹H NMR (300 MHz, DMSO-d₆) δ ppm 1.56 - 1.76 (m, 2 H) 1.83 - 2.03 (m, 2 H) 2.72 - 2.98 (m ,2 H) 3.15 - 3.35 (m, 2 H) 3.39 (s, 6H) 4.10 - 4.27 (m, 1 H) 7.47 (d, *J*=8.29 Hz, 2 H) 7.54 (d, *J*=8.00 Hz, 1 H) 7.62 - 7.77 (m, 3 H) 7.87 (d, *J*=8.29 Hz, 2 H) 8.07 (s, 1 H) 8.39 (d, *J*=7.54 Hz, 1 H) 8.91 - 9.05 (m, 2 H); LC/MS (ES, M+H=516).

The following examples 214-243 are synthesized in a manner analogous to that of Example 213 using the appropriate starting materials. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 214 | 5-(4-Chloro-phenyl)-3-[3-(4-methoxy-phenyl)-ureido]-thiophene-2-carboxylic acid (S)-piperidin-3-ylamide | 486 | 1.40 - 1.90 (m, 4 H) 2.57 - 2.98 (m, 2 H) 2.99 - 3.17 (m, 2 H) 3.32 (s, 3H) 3.90 - 4.48 (m, 1 H) 6.95 (d, 2 H) 7.17 (d, 2H) 7.33 (d, 2 H) 7.60 (d, 2 H) 8.04 - 8.68 (m, 2 H) 9.11 (s, 1 H) 9.96 (s, 1 H) 10.31 (s, 1 H) |
| 215 | ethyl {[(5-(4-chlorophenyl)-2-{[(3S)-piperidin-3-ylamino]carbonyl}-3-thienyl)amino]carbonyl}carba mate | 452 | 1.18 (t, 3 H) 1.43 - 2.02 (m, 4 H) 2.53 - 3.27 (m, 4 H) 3.80 - 4.31 (m, 3 H) 7.49 (d, 2 H) 7.62 (d, 2 H) 8.18 -8.29 (m, 2 H) |
| 216 | 5-(4-Chloro-phenyl)-3-(3-pyridin-2-yl-ureido)-thiophene-2-carboxylic acid (S)-piperidin-3-ylamide | 457 | 1.45 - 1.73 (m, 2 H) 1.75 - 1.95 (m, 2 H) 2.68 - 2.95 (m, 2 H) 3.21 (dd, *J*=35.23, 12.06 Hz, 2 H) 4.06 - 4.20 (m, 1 H) 6.97 (dd, *J*=6.97, 5.27 Hz, 1 H) 7.29 (d, *J*=8.10 Hz, 1 H) 7.49 (d, *J*=8.48 Hz, 2 H) 7.62 (d, *J*=8.40 Hz, 2 H) 7.66 - 7.73 (m, 1 H) 8.09 (d, *J*=7.72 Hz, 1 H) 8.22 (dd, *J*=4.99, 1.22 Hz, 1 H) 8.28 (s, 1 H) 8.55 (s, 1H) 10.12 (s, 1H) |
| 217 | 5-(4-Chloro-phenyl)-3-[3-(4-fluoro-phenyl)-ureido]-thiophene-2-carboxylic acid (S)-piperidin-3-ylamide | 474 | 1.41 - 1.79 (m, 2 H) 1.75 - 1.95 (m, 2 H) 2.56 - 2.95 (m, 2 H) 2.97 - 3.17 (m, 2 H) 3.90 - 4.48 (m, 1 H) 6.93 - 7.15 (m, 2 H) 7.37 - 7.56 (m, 4 H) 7.59 - 7.67 (m, 2 H) 8.04 - 8.68 (m, 2 H) 9.11 (s, 1 H) 9.96 (s, 1 H) 10.31 (s, 1 H) |
| 218 | 5-(4-Chloro-phenyl)-3-[3-(4-cyano-phenyl)-ureido]-thiophene-2-carboxylic acid (S)-piperidin-3-ylamide | 481 | 1.27 - 1.92 (m, 4 H) 2.64 - 2.95 (m, 2 H) 3.09 - 3.31 1 (m, 2 H) 3.86 - 4.17 (m, 1 H) 7.15 - 7.82 (m, 9 H) 8.25 (s, 1 H) 8.68 (s, 1 H) 9.67 (s, 1 H) |
| 219 | 3-[3-(4-Acetyl-phenyl)-ureido]-5-(4-chloro-phenyl)-thiophene-2-carboxylic acid (S)-piperidin-3-ylamide | 498 | 1.15 - 1.55 (m, 2 H) 1.58 - 1.76 (m, 2 H) 2.17 - 2.34 (m, 3 H) 2.55 - 2.79 (m, 2 H) 2.79 - 3.19 (m, 2 H) 3.82 - 4.11 (m, 1 H) 7.33 (d, J=8.48 Hz, 2 H) 7.43 (d, J=8.85 Hz, 2 H) 7.49 (d, J=8.67 Hz, 2 H) 7.69 (d, J=8.67 Hz, 2 H) 8.04 (d, J=7.54 Hz, 1 H) 8.04 - 8.20 (m, 1 H) 8.40 (s, 1 H) |
| 220 | 5-(4-Chloro-phenyl)-3-[3-((1R,2R)-2-phenyl-cyclopropyl)-ureido]-thiophene-2-carboxylic acid (S)-piperidin-3-ylamide | 496 | 0.70 - 1.34 (m, 3 H) 1.39 - 2.03 (m, 5 H) 2.56 - 2.99 (m, 2 H) 2.99 - 3.27 (m, 2 H) 3.92 - 4.26 (m, 1 H) 6.97 - 7.31 (m, 5 H) 7.47 (d, 2 H) 7.60 (d, 2 H) 8.05 (d, 1 H) 8.23 (s, 1 H) |
| 221 | 5-(4-Chloro-phenyl)-3-(3-phenyl-ureido)-thiophene-2-carboxylic acid (S)-piperidin-3-ylamide | 456 | 1.46 - 1.91 (m, 4 H) 2.61 - 3.24 (m, 4 H) 3.84 - 4.33 (m, 1 H) 6.92 (t, 1 H) 7.21 (t, 2 H) 7.42 - 7.53 (m, 4 H) 7.63 (d, 2 H) 8.22 (d, J=7.54 Hz, 1 H) 8.26 (s, 1 H) |
| 222 | 5-(4-Chloro-phenyl)-3-[3-(4-dimethylamino-phenyl)-ureido]-thiophene-2-carboxylic acid (S)-piperidin-3-ylamide | 499 | 1.23 - 2.07 (m, 4 H) 2.43 (s, 3H) 2.44 (s, 3 H) 2.61 -2.96 (m, 2 H) 3.08 - 3.35 (m, 2 H) 3.92 - 4.48 (m, 1 H) 8.03 - 8.37 (m, 9 H) 8.63 (s, 1 H) 9.52 (s, 1 H) 10.41 (s, 1 H) |
| 223 | 3-(3-Benzoyl-ureido)-5-(4-chloro-phenyl)-thiophene-2-carboxylic acid (S)-piperidin-3-ylamide | 484 | 1.53 - 1.94 (m, 2 H) 1.83 - 2.08 (m, 1 H) 2.68 - 3.05 (m, 1 H) 3.84 - 4.41 (m, 5 H) 7.35 - 7.82 (m, 8 H) 8.05 (d, 2 H) |
| 224 | 5-(4-Chloro-phenyl)-3-[3-(2,6-difluoro-phenyl)-ureido]-thiophene-2-carboxylic acid (S)-piperidin-3-ylamide | 492 | 1.44 - 2.04 (m, 4 H) 3.14 - 3.67 (m, 4 H) 4.07 - 4.29 (m, 1 H) 7.33 - 7.44 (m, 2 H) 7.56 (d, 2 H) 7.70 (d, 2 H) 8.19 (d, J=7.72 Hz, 1 H) 8.33 (s, 1 H) 8.66 (s, 1 H) |
| 225 | 5-(4-Chloro-phenyl)-3-[3-(4-methoxy-2-methyl-phenyl)-ureido]-thiophene-2-carboxylic acid (S)-piperidin-3-ylamide | 500 | 1.25 - 1.92 (m, 4 H) 2.94 (s, 3 H) 2.98 - 3.18 (m, 4 H) 3.77 (s, 3 H) 3.91 - 4.18 (m, 1 H) 6.44 - 6.86 (m, 3 H) 7.15 - 7.27 (m, 1 H) 7.41 - 7.51 (m, 2 H) 7.55 - 7.69 (m, 2 H) 8.09 (d, J=7.35 Hz, 1 H) 8.23 (s, 1 H) |
| 226 | 5-(4-Chloro-phenyl)-3-[3-(2-fluoro-phenyl)-ureido]-thiophene-2-carboxylic acid (S)-piperidin-3-ylamide | 474 | 1.18 - 1.89 (m, 2 H) 2.50 - 2.76 (m, 2 H) 3.47 - 4.44 (m, 5 H) 7.00 - 7.31 (m, 4 H) 7.46 (d, 2 H) 7.59 (d, 2 H) 7.92 (d, J=7.91 Hz, 1 H) 8.22 (s, 1 H) |
| 227 | 5-(4-Chloro-phenyl)-3-[3-(3,5-dimethyl-isoxazol-4-yl)-ureido]-thiophene-2-carboxylic acid (S)-piperidin-3-ylamide | 475 | 1.44 - 1.93 (m, 4 H) 2.13 (s, 3 H) 2.30 (s, 3 H) 2.70 -3.26 (m, 4 H) 3.88 - 4.50 (m, 1 H) 7.55 (d, J=8.48 Hz, 2 H) 7.68 (d, 2 H) 8.27 (s, 1 H) 8.30 (d, J=7.72 Hz, 1 H) 9.04 (s, 1 H) 10.43 (s, 1 H) |
| 228 | 3-(3-Benzo[1,3]dioxol-5-yl-ureido)-5-(4-chloro-phenyl)-thiophene-2-carboxylic acid (S)-piperidin-3-ylamide | 500 | 1.47 - 2.02 (m, 4 H) 2.67 - 3.28 (m, 4 H) 4.05 - 4.29 (m, 1 H) 5.75 - 6.12 (m, 2 H) 6.63 - 6.99 (m, 3 H) 7.11 - 7.32 (m, 2 H) 7.46 (d, J=2.45 Hz, 1 H) 7.58 (d, J=8.67 Hz, 1 H) 7.72 (d, J=8.48 Hz, 2 H) 8.25 (d, J=7.91 Hz, 1 H) 8.33 (s, 1 H) 8.55 (s, 1 H) |
| 229 | 5-(4-Chloro-phenyl)-3-[3-(3-fluoro-phenyl)-ureido]-thiophene-2-carboxylic acid (S)-piperidin-3-ylamide | 474 | 1.23 - 2.06 (m, 4 H) 2.47 - 2.95 (m, 2 H) 2.97 - 3.38 (m, 2 H) 3.88 - 4.36 (m, 1 H) 7.05 - 7.86 (m, 9 H) |
| 230 | 5-(4-Chloro-phenyl)-3-[3-(1-phenyl-ethyl)-ureido]-thiophene-2-carboxylic acid (S)-piperidin-3-ylamide | 484 | 1.31 (d, J=6.97 Hz, 3 H) 1.47 - 2.01 (m, 4 H) 2.60 -2.92 (m, 2 H) 3.01 - 3.76 (m, 2 H) 3.95 - 4.33 (m, 1 H) 4.44 - 5.00 (m, 1 H) 7.06 - 7.18 (m, 2 H) 7.22 -7.34 (m, 2 H) 7.45 (d, J=8.67 Hz, 2 H) 7.57 (d, 2 H) 8.09 (d, J=7.72 Hz, 1 H) 8.16 (d, J=7.72 Hz, 1 H) 8.18 (s, 1 H) 9.22 (s, 1 H) 10.04 (s, 1 H) |
| 231 | 5-(4-Chloro-phenyl)-3-[3-(4-nitro-phenyl)-ureido]-thiophene-2-carboxylic acid (S)-piperidin-3-ylamide | 501 | 1.37 - 1.92 (m, 4 H) 2.58 - 3.25 (m, 4 H) 3.91 - 4.39 (m, 1 H) 7.50 (d, 2 H) 7.59 - 7.81 (m, 4 H) 8.16 (d, 2 H) 8.26 (s, 1 H) 8.56 (s, 1 H) 10.50 (s, 1 H) 10.64 (s, 1 H) |
| 232 | 5-(4-Chloro-phenyl)-3-[3-(4-trifluoromethyl-phenyl)-ureido]-thiophene-2-carboxylic acid (S)-piperidin-3-ylamide | 524 | 1.33 - 2.03 (m, 4 H) 2.71 - 3.05 (m, 2 H) 3.03 - 3.37 (m, 2 H) 4.05 - 4.53 (m, 1 H) 7.18 - 7.90 (m, 9 H) 8.29 (d, J=7.54 Hz, 1 H) 8.34 (s, 1 H) 8.76 (s, 1 H) |
| 233 | 5-(4-Chloro-phenyl)-3-[3-(3-cyano-phenyl)-ureido]-thiophene-2-carboxylic acid (S)-piperidin-3-ylamide | 481 | 1.64 - 2.05 (m, 4 H) 2.88 - 3.40 (m, 4 H) 4.17 - 4.48 (m, 1 H) 7.54 - 7.64 (m, 2 H) 7.68 (d, J=8.48 Hz, 2 H) 7.83 (d, 2 H) 8.10 - 8.21 (m, 2 H) 8.42 (d, 1 H) 8.45 (s, 1 H) 8.85 (s, 1 H) |
| 234 | 4-{3-[5-(4-Chloro-phenyl)-2-((S)-piperidin-3-ylcarbamoyl)-thiophen-3-yl]-ureido}-benzoic acid ethyl ester | 528 | 1.24 (t, 3 H) 1.46 - 1.71 (m, 2 H) 1.73 - 1.93 (m, 2 H) 2.60 - 2.91 (m, 2 H) 3.02 - 3.30 (m, 2 H) 4.04 - 4.15 (m, 1 H) 4.21 (q, 2 H) 7.49 (d, J=8.67 Hz, 2 H) 7.56 -7.70 (m, 4 H) 7.84 (d, J=8.85 Hz, 2 H) 8.20 (d, J=7.72 Hz, 1 H) 8.27 (s, 1 H) 8.61 (s, 1 H) |
| 235 | 3-[3-(4-Benzyloxy-phenyl)-ureido]-5-(4-chloro-phenyl)-thiophene-2-carboxylic acid (S)-piperidin-3-ylamide | 562 | 1.33 - 1.99 (m, 4 H) 2.58 - 2.97 (m, 2 H) 3.02 - 3.30 (m, 2 H) 3.88 - 4.28 (m, 1 H) 4.85 - 5.11 (m, 2 H) 6.71 - 7.71 (m, 14 H) 8.13 (d, J=7.54 Hz, 1 H) 9.71 (s, 1 H) 10.21 (s, 1 H) |
| 236 | 5-(4-Chloro-phenyl)-3-[3-(4-phenoxy-phenyl)-ureido]-thiophene-2-carboxylic acid (S)-piperidin-3-ylamide | 548 | 1.35 - 1.97 (m, 4 H) 2.62 - 2.89 (m, 2 H) 3.04 - 3.25 (m, 2 H) 3.95 - 4.23 (m, 1 H) 6.83 - 6.97 (m, 5 H) 7.03 (t, J=7.35 Hz, 1 H) 7.22 - 7.35 (m, 2 H) 7.42 -7.53 (m, 4 H) 7.59 - 7.69 (m, 2 H) 8.27 (s, 1 H) 9.92 (s, 1 H) 10.28 (s, 1 H) |
| 237 | 5-(4-Chloro-phenyl)-3-[3-(3-methyl-5-phenyl-isoxazol-4-yl)-ureido]-thiophene-2-carboxylic acid (S)-piperidin-3-ylamide | 537 | 1.16 - 1.94 (m, 4 H) 2.64 - 2.88 (m, 2 H) 3.11 (s, 3 H) 3.08 - 3.32 (m, 2 H) 3.97 - 4.30 (m, 1 H) 6.92 - 7.92 (m, 10 H) 8.11 - 8.32 (m, 1 H) |
| 238 | 5-(4-Chloro-phenyl)-3-[3-((1S,2R)-2-phenyl-cyclopropyl)-ureido]-thiophene-2-carboxylic acid (S)-piperidin-3-ylamide | 496 | 0.76 - 1.96 (m, 8 H) 2.53 - 2.82 (m, 2 H) 3.57 - 4.03 (m, 3 H) 6.89 - 7.28 (m, 8 H) 7.38 - 7.50 (m, 1 H) 7.54 - 7.69 (m, 1 H) 8.22 (s, 1 H) |
| 239 | 5-(4-Chloro-phenyl)-3-[3-(2,6-dichloro-pyridin-4-yl)-ureido]-thiophene-2-carboxylic acid (S)-piperidin-3-ylamide | 525 | 1.53 - 2.03 (m, 4 H) 2.78 - 3.06 (m, 2 H) 3.41 - 3.91 (m, 3 H) 7.47 - 7.90 (m, 9 H) |
| 240 | 5-(4-Chloro-phenyl)-3-{3-[(S)-1-(2,2,2-trifluoro-acetyl)-piperidin-3-yl]-ureido}-thiophene-2-carboxylic acid (S)-piperidin-3-ylamide | 559 | 1.00 - 2.11 (m, 8 H) 2.99 - 4.28 (m, 10 H) 7.25 - 7.53 (m, 2 H) 7.54 - 7.77 (m, 2 H) 8.22 (d, J=6.40 Hz, 1 H) |
| 241 | 5-(4-chlorophenyl)-3-({[(phenylsulfonyl)amino]carb onyl}amino)-N-[(3S)-piperidin-3-yl]thiophene-2-carboxamide | 520 | 1.34 - 2.04 (m, 4 H) 2.57 - 2.89 (m, 2 H) 2.96 - 3.30 (m, 2 H) 3.94 - 4.30 (m, 1 H) 7.39 - 7.49 (m, 2 H) 7.48 - 7.70 (m, 4 H) 7.71 - 7.81 (m, 1 H) 7.86 - 7.94 (m, 2 H) 7.97 (s, 1 H) 8.35 (d, J=7.72 Hz, 1 H) |
| 242 | 5-(4-chlorophenyl)-3-[({[(4-methylphenyl)sulfonyl]amino} carbonyl)amino]-N-[(3S)-piperidin-3-yl]thiophene-2-carboxamide | 534 | 1.57 - 1.81 (m, 2 H) 1.82 - 1.98 (m, 2 H) 2.41 (s, 3 H) 2.78 - 2.94 (m, 2 H) 3.18 - 3.36 (m, 2 H) 4.10 - 4.24 (m, 1 H) 7.46 (d, *J*=8.29 Hz, 2 H) 7.54 (d, *J*=8.67 Hz, 2 H) 7.69 (d, *J*=8.00 Hz, 2 H) 7.87 (d, *J*=8.29 Hz, 2 H) 8.07 (s, 1 H) 8.38 (d, *J*=7.72 Hz, 1 H) 8.95 (s, 2 H) |
| 243 | 5-(4-Chloro-phenyl)-3-(3-cyclohexyl-ureido)-thiophene-2-carboxylic acid (S)-piperidin-3-ylamide | 462 | 0.81 - 1.34 (m, 8 H) 1.35 - 1.96 (m, 7 H) 2.79 - 2.96 (m, 2 H) 3.24 - 3.73 (m, 3 H) 7.51 (d, J=8.48 Hz, 2 H) 7.59 - 7.72 (m, 3 H) 7.81 - 7.88 (m, 2 H) |

### Example 244

### N-(5-phenyl-3-{[(3S)-piperidin-3-ylamino]carbonyl}-2-thienyl)hydrazinecarboxamide

To a solution of *tert*-butyl (3*S*)-3-{[(2-amino-5-phenyl-3-thienyl)carbonyl]amino}piperidine-1-carboxylate [prepared as in Example 15] (200 mg, 0.5 mmol) in THF (1.0 mL) was added 1,1'-carbonyl diimidazole (240 mg, 1.5 mmol). The resulting cloudy solution was stirred at rt for 1 h whereupon a solution of NH₂NH₂ in THF (200 µL in 1 mL of THF) was added and the resulting dark solution was stirred for 10 h at rt. The mixture was partitioned between EtOAc and H₂O and the organic layer was washed with H₂O, brine and dried (MgSO₄). Evaporation afforded ***tert-*butyl (3*S*)-3-[({2-[(hydrazinocarbonyl)amino]-5-phenyl-3-thienyl}carbonyl)amino]piperidine-1-carboxylate** as a yellow residue. The residue was treated with 4.0N HCl solution in dioxane (2 mL) and the resulting cloudy mixture was stirred at rt for 1 h. Evaporation of the solvent followed by purification by Gilson (5% MeCN-H₂O→98%MeCN-H₂O) gave the desired product. ¹H NMR (300 MHz, DMSO-d₆) δ ppm 1.49 - 1.74 (m, 3 H) 1.84 - 1.97 (m, 2 H) 2.74 - 3.07 (m, 2 H) 3.29 (dd, *J*=34.76, 11.77 Hz, 2 H) 4.04 - 4.30 (m, 1 H) 7.27 (t, *J*=7.25 Hz, 1 H) 7.41 (t, *J*=7.63 Hz, 2 H) 7.55 (d, *J*=7.16 Hz, 2 H) 7.80 (s, 1 H) 8.12 (s, 1 H) 8.66 (s, 2 H); LC/MS (ES, M+H=360).

Examples **245-247** were made in an analogous manner using the appropriate starting materials but utilizing hydroxylamine in place of the hydrazine. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 245 | 3-{[(hydroxyammo)-carbonyl]amino}-5-phenyl-N-[(3S)-piperidin-3-yl]thiophene-2-carboxamide | 361 | 1.66 - 1.66 (m, 2 H) 1.88 - 1.90 (m, 2 H) 2.84 - 2.85 (m, 2 H) 3.68 - 3.69 (m, 2 H) 4.20 - 4.21 (m, 1 H) 7.47 - 7.48 (m, 2 H) 7.68 - 7.68 (m, 3 H) 8.31 - 8.31 (s, 1 H) 8.96 - 8.97 (br s, 1 H) 9.08 - 9.09 (br s, 1 H) 9.26 -9.29 (d, 2 H) 11.08 - 11.10 (s, 1 H) |
| 246 | 5-(4-chlorophenyl)-3-{[(hydroxyamino)carbonyl]a mino}-N-[(3S)-piperidin-3-yl]thiophene-2-carboxamide | 396 | 1.41 - 1.69 (m, 2 H) 1.69 - 1.94 (m, 2 H) 2.59 - 2.91 (m, 2 H) 3.09 - 3.21 (m, 1 H) 3.83 - 3.99 (m, 1 H) 4.02 - 4.20 (m, 1 H) 7.46 (d, *J*=4.71 Hz, 1 H) 7.49 (d, *J*=4.71 Hz, 2 H) 7.54 (s, 1 H) 7.62 (d, *J*=8.48 Hz, 1 H) 7.67 (d, *J*=8.67 Hz, 1 H) 8.16 (d, *J*=7.54 Hz, 1 H) 8.25 (s, 1 H) |
| 247 | 2-{[(hydroxyammo)-carbonyl]amino}-5-phenyl-N-[(3S)-piperidin-3-yl]thio-phene-3-carboxamide | 361 | 1.39 - 1.79 (m, 2 H) 1.79 - 2.16 (m, 2 H) 2.76 - 2.98 (m, 4 H) 3.09 - 3.45 (m, 2 H) 3.95 - 4.06 (m, 1 H) 4.09 - 4.26 (m, 1 H) 7.28 (t, *J*=7.35 Hz, 1 H) 7.36 - 7.51 (m, 3 H) 7.56 (d, *J*=8.40 Hz, 2 H) 7.68 (d, *J*=7.35 Hz, 2 H) 7.74 (s, 1 H) 7.83 (s, 1 H) 8.18 (d, *J*=7.54 Hz, 1 H) |

### Example 248

### 5-Phenyl-3-ureido-thiophene-2-sulfonic acid (S)-piperidin-3-ylamide

***tert*-butyl (3*S*)-3-{[(5-chloro-2-thienyl)sulfonyl]amino}piperidine-1-carboxylate.** 5-Chlorothiophene-2-sulfonyl chloride (1g, 4.6062mmol), *tert*-butyl (3*S*)-3-aminopiperidine-1-carboxylate (1.1070g, 5.5274mmol), and 20mL dichloromethane were added to a 50mL round bottom flask. Diisopropylethylamine was then added with stirring. Let stir one hour. Wash reaction mixture with water, then dry over MgSO₄. Filter, and concentrate filtrate to dryness; 1.75g, 99%, 4.60mmol. ¹H NMR (300 MHz, CDCl₃) δ ppm 1.69 (m, 2 H), 1.80 (m, 2 H), 3.20 (m, 1 H), 3.31 (m, 1 H), 3.41 (m, 2 H), 3.56 (m, 1 H), 4.94 (d, 1 H), 6.94 (d, 1 H), 7.45 (d, 1 H).

***tert*-butyl (3S)-3-{[(3-amino-5-chloro-2-thienyl)sulfonyl]amino}piperidine-1-carboxylate.** *Tert*-butyl (3*S*)-3-{[(5-chloro-2-thienyl)sulfonyl]amino}piperidine-1-carboxylate (1g, 2.7330mmol) and 10mL THF were added to a 25mL flask under dry conditions and a nitrogen atmosphere. Cool to -75°C and add n-BuLi (2.5M, 2.19mL, 5.4660mmol) slowly dropwise. Let warm slowly to -20°C. Slowly add 2,4,6-triisopropylbenzenesulfonyl azide, as a solution in 5mL dry THF, at -20°C. Let the reaction warm to room temperature. Add 10mL water. Separate organic layer from aqueous layer. Extract aqueous layer with dichloromethane, and combine organic fractions. To this add Hexadecyltri-n-butylphosphonium bromide (.1387g, .2733mmol), then Sodiumborohydride (.1199g, 3.1703mmol) in 5mL water slowly dropwise. Stir at room temperature overnight. Extract reaction mixture with ethyl acetate. Concentrate organic washings. Separate by normal phase MPLC; 0-50% B (A = Hexanes, B = EtOAc), .2822g, 25.4%, ¹H NMR (300 MHz, DMSO-d₆) δ ppm 1.04 (m, 2 H), 1.38 (m, 1 H), 1.53 (m, 1 H), 1.86 (s, 1 H), 2.45 (m, 1 H), 2.71 (m, 1 H), 3.42 (d, 1 H), 3.60 (m, 1 H), 5.89 (s, 2 H), 6.42 (s, 1 H), 7.55 (d, 1 H).

***tert*-butyl (3*S*)-3-[({3-[(aminocarbonyl)amino]-5-chloro-2-thienyl}sulfonyl)amino]-piperidine-1-carboxylate.** *Tert*-butyl (3*S*)-3-{[(3-amino-5-chloro-2-thienyl)sulfonyl]amino}piperidine-1-carboxylate (427.8 mg, 1.08 mmol) and 10 mL dry THF were added to a dry and nitrogen purged 25 mL flask. Trichloroacetylisocyanate (0.385 mL, 3.24 mmol, 3 eq.) was added dropwise via syringe with stirring. This reaction mixture was stirred for 15 min, and then the solvent was removed in vacuo. Excess reagent was removed under high vacuum to give tert-butyl (3S)-3-({[5-chloro-3-({[(trichloroacetyl)amino]carbonyl}amino)-2-thienyl]sulfonyl}amino)piperidine-1-carboxylate. Dissolve residue in 5 mL methanol, then add 2M ammonia in methanol (1.62 mL, 3.24 mmol, 3 eq.). Stir 30 minutes. Remove solvent in vacuo. Purify with MPLC using a normal phase column, 50-100 % B (A=Hexanes, B=Ethyl Acetate). Product elutes at ∼90%B. 248.2 mg product obtained. ¹H NMR (300 MHz, DMSO-d₆) δ ppm 1.22 (m, 2 H), 1.39 (m, 2 H), 1.45 (s, 9 H), 1.71 (m, 1 H), 1.83 (m, 1 H), 2.82 (m, 2 H) 3.19 (m, 1 H), 3.73 (d, 1 H), 6.83 (s, 1 H), 8.42 (s, 2 H), 8.53 (s, 1 H).

***tert*-butyl (3*S*)-3-[({3-[(aminocarbonyl)amino]-5-phenyl-2-thienyl}sulfonyl)amino]piperidine-1-carboxylate.** *Tert*-butyl (3*S*)-3-[({3-[(aminocarbonyl)amino]-5-chloro-2-thienyl}sulfonyl)amino]piperidine-1-carboxylate (0.1156 g, 0.2634 mmol), phenylboronic acid (.0642g, .5267mmol, 2 eq.), cesium carbonate (.0304 g, .7901 mmol, 3 eq.), and Tetrakis(triphenylphosphine)palladium(0) were added to a nitrogen purged 25 mL round bottom flask equipped with a magnetic stir bar. Add 1 mL water and 3 mL dioxane. Heat to 80°C for 2 hr. Allow reaction to cool. Remove aqueous layer. Concentrate Organic layer and purify on MPLC with normal phase column. Run solvent gradient from 60-70%B (A=Hexanes, B=Ethyl Acetate). 20 mg isolated. ¹H NMR (300 MHz, CDCl₃) δ ppm 1.35 (s, 9 H), 1.46 (m, 2 H), 1.62 (m, 1 H), 1.73 (m, 1 H), 3.10 (m, 2 H), 3.26 (m, 1 H), 3.38 (m, 1 H), 3.72 (s, 1 H), 7.30 (m, 3 H), 7.53 (d, 2 H), 8.09 (s, 1 H), 8.33 (s, 1 H).

**5-Phenyl-3-ureido-thiophene-2-sulfonic acid (S)-piperidin-3-ylamide.** *tert*-butyl (3*S*)-3-[({3-[(aminocarbonyl)amino]-5-phenyl-2-thienyl}sulfonyl)amino]piperidine-1-carboxylate (20 mg) was stirred in 1 mL 4N HCl in MeOH for 2 hours. The solvents were evaporated and the residue was placed under a high vacuum yielding the title compound, 15mg, HCl salt. ¹H NMR (300 MHz, DMSO-d₆) δ ppm 1.53 (m, 2 H), 1.79 (d, 2 H), 2.78 (q, 2 H), 3.13 (t, 2 H), 3.69 (m, 2 H), 6.79 (s, 2 H), 7.46 (m, 3 H), 7.67 (d, 2 H), 8.19 (s, 1 H), 8.49 (s, 1 H), 8.64 (s, 1 H), 8.77 (s, 2 H). LCMS (ES, M+H=381).

Example **249** was made in an analogous manner utilizing 3-fluorophenyl boronic acid in place of the phenylboronic acid. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 249 | 5-(3-Fluoro-phenyl)-3-ureido-thiophene-2-sulfonic acid (S)-piperidin-3-ylamide | 399 | 1.53 (m, 2 H), 1.81 (d, 2 H), 2.79 (m, 2 H), 3.16 (t, 2 H), 6.77 (s, 2 H), 7.31 (s, 1 H), 7.54 (m, 3 H), 8.21 (s, 1 H), 8.40 (s, 1 H), 8.49 (d, 1 H), 8.62 (m, 2 H) |

### Example 250

### 5-Piperidin-1-yl-2-ureido-thiophene-3-carboxylic acid (S)-piperidin-3-ylamide

**tert-butyl (3S)-3-[({2-[(aminocarbonyl)amino]-5-piperidin-1-yl-3-thienyl}carbonyl)amino]piperidine-1-carboxylate.** tert-butyl (3S)-3-[({2-[(aminocarbonyl)amino]-5-bromo-3-thienyl}carbonyl)amino]piperidine-1-carboxylate [prepared as in Example 15] (0.5g, 1.12mmol) and 5mL DMF were added to a 25mL flask. .1mL piperidine was then added, and the reaction stirred at room temperature overnight. Water was then added to the reaction mixture to precipitate the crude product, and this was filtered. The solid was purified by normal phase MPLC; gradient 30-70% B (A = Hexanes, B = EtOAc), .2433g, ¹H NMR (300 MHz, DMSO-d₆) δ ppm 1.50 (m, 2 H), 1.63 (m, 6 H), 1.88 (m, 2 H), 2.86 (q, 2 H), 3.00 (t, 4 H), 3.15 (d, 1 H), 3.26 (d, 1 H,) 4.17 (m, 1 H), 4.36 (s, 7 H), 6.57 (s, 1 H), 6.79 (s, 1 H), 7.97 (d, 1 H), 9.02 (m, 1 H), 9.16 (m, 1 H), 10.64 (s, 1 H). LCMS (ES, M=H=452).

**5-Piperidin-1-yl-2-ureido-thiophene-3-carboxylic acid (S)-piperidin-3-ylamide.** tert-butyl (3S)-3-[({2-[(aminocarbonyl)amino]-5-piperidin-1-yl-3-thienyl}carbonyl)amino]piperidine-1-carboxylate (243.3mg, 0.5388mmol) was dissolved in 3mL methanol. 4N HCl in dioxane (2mL) was added, and the reaction was stirred at room temperature for 2 hours. The reaction was concentrated in vacuo. The product was put under high vacuum for several hours; 240.2mg, 0.6192mmol, ¹H NMR (300 MHz, DMSO-d₆) δ ppm 1.50 (m, 2 H), 1.63 (m, 6 H), 1.88 (m, 2 H), 2.86 (q, 2 H), 3.00 (t, 4 H), 3.15 (d, 1 H), 3.26 (d, 1 H,) 4.17 (m, 1 H), 4.36 (s, 7 H), 6.57 (s, 1 H), 6.79 (s, 1 H), 7.97 (d, 1 H), 9.02 (m, 1 H), 9.16 (m, 1 H), 10.64 (s, 1 H). LCMS (ES, M+H=352).

### Example 251

### 2-Phenyl-5-ureido-thiazole-4-carboxylic acid (S)-azepan-3-ylamide

**ethyl 3-nitriloalaninate.** To a stirred solution of ethyl (2*E*)-cyano(hydroxyimino)acetate (10 g) in H₂O (30 mL) and saturated aq. NaHCO₃ (60 mL) was added portion-wise sodium dithionite (35 g) over 10 mins. The cloudy yellow solution was stirred for 30 mins at rt whereupon NaCl was added and the resulting slurry stirred for further 15 mins at rt. The mixture was diluted with CH₂Cl₂ and the aqueous phase was extracted with CH₂Cl₂ (4x). The organic extracts dried (MgSO₄) and evaporation gave the title compound as yellow oil (25% yield) that was used directly to the next step.

**ethyl *N*-benzoyl-3-nitriloalaninate.** To a stirred solution of ethyl 3-nitriloalaninate (1.14 g) in CH₂Cl2 (18 mL) at 0°C were added BzCl (1.2 mL) and Et₃N (2.3 mL). The resulting cloudy orange solution was stirred at rt for 2h whereupon it was diluted with EtOAc and washed with H₂O, brine and dried (MgSO₄). Evaporation gave a dark brown oil which was purified by Gilson (5%-95% MeCN-H₂O) to give the title compound as pale yellow solid (55% yield). LC/MS (ES, M+H=233).

**ethyl 5-amino-2-phenyl-1,3-thiazole-4-carboxylate.** To a stirred solution of ethyl *N*-benzoyl-3-nitriloalaninate (1.0 g) in dry toluene (20 mL) was added Lawesson's reagent (1.8 g) and the resulting mixture was heated to reflux for 24 h. The mixture was diluted with EtOAc and the organic extracts were washed with H₂O, brine and dried (MgSO₄). Evaporation of the solvents gave a brown oil. Purification by Gilson (5%-95% MeCN-H₂O) afforded the title compound as yellow solid (35%). LC/MS (ES, M+H=249).

**ethyl 2-phenyl-5-({[(trichloroacetyl)amino]carbonyl}amino)-1,3-thiazole-4-carboxylate.** To a stirred solution of ethyl 5-amino-2-phenyl-1,3-thiazole-4-carboxylate (50 mg) in anhydrous THF ( 1 mL) was added trichloroacetyl isocyanate (24 µL) slowly over a period of 5 min. After the addition was complete, a precipitate formed and the reaction stirred for an additional 1h. The desired product was obtained by filtration (99% yield) as a yellow solid. The product was used in the next step without any further purification. LC/MS (ES, M+H=436).

### tert-butyl (3S)-3-[({5-[(aminocarbonyl)amino]-2-phenyl-1,3-thiazol-4-yl}carbonyl)amino]azepane-1-carboxylate.

To a solution of ethyl 2-phenyl-5- ({[(trichloroacetyl)amino]carbonyl}amino)-1,3-thiazole-4-carboxylate (40 mg) in anhydrous THF (2 mL) was added via cannula a solution of [Me₃Al and *tert*-butyl (3*S*)-3-aminoazepane-1-carboxylate] in THF (preformed by the careful addition of Me₃Al (2.0M in hexanes, 500 µL) to a solution of *tert*-butyl (3*S*)-3-aminoazepane-1-carboxylate in 5 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as yellow solid (50% yield). LC/MS (ES, M+H=460).

**2-Phenyl-5-ureido-thiazole-4-carboxylic acid (S)-azepan-3-ylamide.** A solution of *tert*-butyl (3*S*)-3-[({5-[(aminocarbonyl)amino]-2-phenyl-1,3-thiazol-4-yl}carbonyl)amino]azepane-1-carboxylate (30 mg) in 4.0N HCl in 1, 4-dioxane (3 mL) was stirred for 30 mins at rt. The cloudy solution was diluted with dry methanol and the solvents were removed under vacuum. The residue was dissolved in H₂O and placed in a lyophilizer to yield the title compound as white solid (quantitative yield). ¹H NMR (300 MHz, DMSO-d₆) δ ppm 0.85 - 2.04 (m, 6 H) 2.84 - 3.21 (m, 3 H) 3.72 - 3.98 (m, 1 H) 4.10 - 4.58 (m, 1 H) 7.26 - 7.50 (m, 3 H) 7.81 - 7.95 (m,2 H) 8.23 (d, *J*=8.85 Hz, 1 H); LC/MS (ES, M+H=361).

### Example 252

### 2-Methyl-5-ureido-thiazole-4-carboxylic acid azepan-3-ylamide

**ethyl 5-amino-2-methyl-1,3-thiazole-4-carboxylate.** To a stirred solution of ethyl *N*-acetyl-3-nitriloalaninate (1.0 eq) in dry toluene (40 mL) was added Lawesson's reagent (0.5 eq) and the resulting mixture was heated to reflux for 24 h. The mixture was diluted with EtOAc and the organic extracts were washed with H₂O, brine and dried (MgSO₄). Evaporation of the solvents gave a brown oil. Purification by Gilson (5%-95% MeCN-H₂O) afforded the title compound as yellow solid (50%). LC/MS (ES, M+H=187).

**ethyl 2-methyl-5-({[(trichloroacetyl)amino]carbonyl}amino)-1,3-thiazole-4-carboxylate.** To a stirred solution of ethyl 5-amino-2-methyl-1,3-thiazole-4-carboxylate (1 equiv) in anhydrous THF (10 mL) was added trichloroacetyl isocyanate (1 eq) slowly over a period of 5 min. After the addition was complete, a precipitate formed and the reaction stirred for an additional 1h. The desired product was obtained by filtration (99% yield) as a yellow solid. The product was used in the next step without any further purification. LC/MS (ES, M+H=374).

***tert*-butyl (3*S*)-3-[({5-[(aminocarbonyl)amino]-2-methyl-1,3-thiazol-4-yl}carbonyl)amino]azepane-1-carboxylate.** To a solution of ethyl 2-methyl-5-({[(trichloroacetyl)amino]carbonyl}amino)-1,3-thiazole-4-carboxylate (1 eq) in anhydrous THF (20 mL) was added via cannula a solution of [Me₃Al and (3*S*)-3-aminoazepane-1-carboxylate] in THF (preformed by the careful addition of Me₃Al (2.0M in hexanes, 4 eq) to a solution of (3*S*)-3-aminoazepane-1-carboxylate (4 eq) in 25 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as yellow solid (50% yield). LC/MS (ES, M+H=398).

**2-Methyl-5-ureido-thiazole-4-carboxylic acid azepan-3-ylamide.** A solution of tert-butyl (3S)-3-[({5-[(aminocarbonyl)amino]-2-methyl-1,3-thiazol-4-yl}carbonyl)amino]azepane-1-carboxylate (1 eq) in 4.0N HCl in 1, 4-dioxane (20 mL) was stirred for 30 mins at rt. The cloudy solution was diluted with dry methanol and the solvents were removed under vacuum. The residue was dissolved in H₂O and placed in a lyopholizer to yield the title compound as white solid (quantitative yield). ¹H NMR (d₆-DMSO, 300 MHz) δ ppm 0.80 - 2.00 (m, 6 H) 2.48 (s, 3H) 2.78 - 3.16 (m, 3 H) 3.70-4.20 (m, 2 H). LC/MS (ES, M+H=298).

Example **253** was made in an analogous manner as Example 252 using the appropriate starting materials. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 253 | 2-p-Tolyl-5-ureido-thiazole-4-carboxylic acid (S)-piperidin-3-ylamide | 360 | 2.04 (s, 3 H) 2.94 - 3.26 (m, 4H) 3.72 - 3.98 (m, 1 H)4.20-4.58 (m, 1 H) 7.36 (d,2 H) 7.95 (d, 2 H) 8.23 (d, 1 H) |

### Example 254

### 2-Phenyl-5-ureido-oxazole-4-carboxylic acid (S)-piperidin-3-ylamide

**ethyl 5-amino-2-phenyl-1,3-oxazole-4-carboxylate.** To a stirred solution of ethyl N-benzoyl-3-nitriloalaninate (1.0 g) in dry dioxane (20 mL) was added a solution of HCl in dioxane (4.0 M, 20 mL) and the resulting mixture was heated to reflux for 10 h. Evaporation of the solvent gave the desired product as white solid; LC/MS(ES,M+H=233).

**ethyl 2-phenyl-5-({[(trichloroacetyl)amino]carbonyl}amino)-1,3-oxazole-4-carboxylate.** To a stirred solution of ethyl 5-amino-2-phenyl-1,3-oxazole-4-carboxylate (300 mg, 1.29 mmol) in anhydrous THF ( 2.6 mL) was added trichloroacetyl isocyanate (153 µL, 1.29 mmol) slowly over a period of 5 min. After the addition was complete, a precipitate formed and the reaction stirred for an additional 1h. The desired product was obtained by filtration (99% yield) as a yellow solid. The product was used in the next step without any further purification LC/MS (ES, M+H=421).

***tert*-butyl (3*S*)-3-[({5-[(aminocarbonyl)amino]-2-phenyl-1,3-oxazol-4-yl}carbonyl)amino]piperidine-1-carboxylate.** To a solution of ethyl 2-phenyl-5-({[(trichloroacetyl)amino]carbonyl}amino)-1,3-oxazole-4-carboxylate (320 mg, 1.2 mmol) in anhydrous THF (10 mL) was added via cannula a solution of [Me₃Al and *tert*-butyl (3*S*)-3-aminopiperidine-1-carboxylate] in THF (preformed by the careful addition of Me₃Al (2.0M in hexanes, 6.2 mL, 12.3 mmol) to a solution of *tert*-butyl (3*S*)-3-aminopiperidine-1-carboxylate (1.29 g, 6.45 mmol) in 20 mL of THF at 0°C and subsequently stirring at rt for 10 mins). The resulting yellow solution was stirred at rt for 10 h. The reaction mixture was cooled to 0°C and a 10% aqueous solution of Rochelle's salt was added slowly to quench the reaction. The mixture was partitioned between EtOAc and H₂O, the aqueous layer was extracted with EtOAc (3x) and the combined organic extracts were washed with H₂O, brine and dried (MgSO₄). Evaporation gave a pale yellow solid. Purification by Gilson (5%-95% H₂O/MeCN) gave the title compound as yellow solid (50% yield). LC/MS (ES, M+H=460).

**2-Phenyl-5-ureido-oxazole-4-carboxylic acid (S)-piperidin-3-ylamide.** A solution of *tert-*butyl (3*S*)-3-[({5-[(aminocarbonyl)amino]-2-phenyl-1,3-oxazol-4-yl}carbonyl)amino]piperidine-1-carboxylate (20 mg) in 4.0N HCl in 1, 4-dioxane (3 mL) was stirred for 30 mins at rt. The cloudy solution was diluted with dry methanol and the solvents were removed under vacuum. The residue was dissolved in H₂O and placed in a lyophilizer to yield the title compound as white solid (quantitative yield). ¹H NMR (300 MHz, DMSO-d₆) 1.50 - 1.70 (m, 2 H) 1.80 - 2.03 (m, 2 H) 3.04- 3.40 (m, 5 H) 7.10-7.65 (m, 5 H). LC/MS (ES, M+H=329).

### Example 255

### {2-Fluoro-4-[4-((S)-piperidin-3-ylcarbamoyl)-5-ureido-thiophen-2-yl]-ohenyl}-carbamic acid ethyl ester

***tert*-butyl (3S)-3-({[2-[(aminocarbonyl)amino]-5-(4,4,5,5-tetramethyl-1,3,2-dioxaborolan-2-yl)-3-thienyl]carbonyl}amino)piperidine-1-carboxylate.** *tert*-butyl (3*S*)-3-[({2-[(aminocarbonyl)amino]-5-bromo-3-thienyl}carbonyl)amino]piperidine-1-carboxylate [prepared as in Example 15] (2.0 g, 4.474 mmol), was added to pinacol diborone (1.7 g, 6.711 mmol), Na₂CO₃ (1.42 g, 13.422 mmol), NaI (738.2 mg, 4.921 mmol), CuI (25 mg, cat.), and PdCl₂(dppf) (100 mg" 0.134 mmol) into a reaction flask. The solids were dispersed in 60 ml of anhydrous MeOH and 2 ml of anhydrous acetone, degassed under vacuum, then flushed with nitrogen. The reaction was heated at 60°C for 30 mins. LC-MS showed ∼31% of boronic acids, ∼13% of debrominated compound, ∼51% of boronate, and 5% of a dimer. Then the reaction was cooled to room temperature, filtered, and evaporated to give 3.2 g of the product as a light green powder.

***tert*-butyl (3*S*)-3-{[(2-[(aminocarbonyl)amino]-5-{4-[(ethoxycarbonyl)amino]-3-fluorophenyl}-3-thienyl)carbonyl]amino}piperidine-1-carboxylate.** *tert*-butyl (3*S*)-3-({[2-[(aminocarbonyl)amino]-5-(4,4,5,5-tetramethyl-1,3,2-dioxaborolan-2-yl)-3-thienyl]carbonyl}amino)piperidine-1-carboxylate (800mg, effective <450 mg, 0.91 mmol), Ethyl (4-bromo-4-Fluorophenyl) carbamate (393 mg, 1.5 mmol), Cs₂CO₃ (1.3 g, 4.0 mmol), and Pd(PPh₃)₄ (70 mg, 0.06 mmol) were dispersed in dioxane (4 ml), and water (1 ml) to a reaction vial. The reaction vial was degassed under vacuum and replenished with nitrogen followed by heating to 80°C for 2 hr. The reaction mixture was cooled to rt and concentrated *in vacuo.* The residue was dissolved in DMSO, filtered, and then purified by LCPrep Chromatography to give the title compound (79 mg).

**{2-Fluoro-4-[4-((S)-piperidin-3-ylcarbamoyl)-5-ureido-thiophen-2-yl]-phenyl}-carbamic acid ethyl ester.** *tert*-butyl (3*S*)-3-{[(2-[(aminocarbonyl)amino]-5-{4-[(ethoxycarbonyl)amino]-3-fluorophenyl}-3-thienyl)carbonyl]amino}piperidine-1-carboxylate (79 mg, mmol) was dissolved in 2 ml of MeOH, and charged with 2 ml of 4N HCl/Dioxane. The reaction mixture was stirred for 3 hours, concentrated *in vacuo,* and purified by ? to yield the title compound (65.0 mg, 13% (3 steps)). ¹H NMR (d₆-DMSO); 10.91 (s, 1H), 9.37 (s, 1H), 9.21 (s, 1H), 9.08 (s, 1H), 8.31 (d, 1H), 8.01 (s, 1H), 7.65 (t, 1H), 7.3 (d, 1H), 7.31 (d, 1H), 7.04 (br, 2H), 4.21 (s, 1H), 4.12 (q, 2H), 3.17 (d, 2H), 2.95 (m, 2H), 1.91 (d, 2H), 1.69 (m, 2H), 1.24 (t, 3H). LCMS (ES, M+H=450).

The following examples **256-258** in the table below were prepared using analogous methodology to example **255** using the appropriate starting materials. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 256 | 5-(4-Acetyl-phenyl)-2-ureido-thiophene-3-carboxylic acid (S)-piperidin-3-ylamide | 387 | 10.99 (s, 1H), 9.24 (s, 1H), 9.09 (s, 1H), 8.45 (d, 1H), 8.23 (s, 1H), 7.99 (d, 2H), 7.71 (d, 2H), 7.10 (br, 2H), 4.23 (s, 1H), 3.30 (d, 1H), 3.14 (d, 1H), 2.94 (m, 2H), 2.58 (s, 3H), 1.93 (d, 2H), 1.67 (m, 2H). |
| 257 | {2,6-Difluoro-4-[4-((S)-piperidin-3-ylcarbamoyl)-ureido-thiophen-2-yl]-phenyl}-carbamic acid ethyl ester | 468 | 10.95 (s, 1H), 9.28 (s, 1H), 9.14 (s, 2H), 8.43 (d, 1H), 8.22 (s, 1H), 7.59 (m, 1H), 7.31 (d, 2H), 7.13 (br, 1H), 4.25 (s, 1H), 4.09 (q, 2H), 3.13 (d, 2H), 2.96 (m, 2H), 1.92 (d, 2H), 1.67 (m, 2H), 1.22 (t, 3H). |
| 258 | 5-(4-Amino-3,5-difluoro-phenyl)-2-ureido-thiophene-3-carboxylic acid (S)-piperidin-3-ylamide | 396 | 10.84 (s, 1H), 9.06 (s, 1H), 8.96 (s, 1H), 8.18 (d, 1H), 7.77 (s, 1H), 7.09 (d, 2H), 6.97 (d, 1H), 4.17 (s, 1H), 4.14 (m, 1H), 3.17 (m, 2H), 2.89 (m, 2H), 1.91 (d, 2H), 1.64 (m, 2H). |

### Example 259

### 2-({Amino-[(E)-toluene-4-sulfonylimino]-methyl}-amino)-5-phenylthiophene-3-carboxylic acid (S)-piperidin-3-ylamide

**N-[1-Amino-1-methylsulfanyl-meth-(E)-ylidene]-4-methyl-benzenesulfonamide.** A solution of N-(Bis-methylsulfanyl-methylene)-4-methyl-benzenesulfonamide (500 mg, 1.8 mmol) in dry acetonitrile (30 mL) was purged with ammonia gas at 0°C for 2 h. Upon warming up the solution to room temperature, the desired product precipitated and collected by filtration.

**2-({Amino-[(E)-toluene-4-sulfonylimino]-methyl}-amino)-5-phenylthiophene-3-carboxylic acid (S)-piperidin-3-ylamide.** To a solution of *tert*-butyl (3*S*)-3-[({2-[(aminocarbonyl)amino]-5-phenyl-3-thienyl}carbonyl)amino]piperidine-1-carboxylate [as prepared in Example 15] (95 mg, 0.23 mmol) in NMP (1.1 mL) was added N-[1-Amino-1-methylsulfanyl-meth-(E)-ylidene]-4-methyl-benzenesulfonamide (57.5 mg, 0.23 mmol) and the resulting solution was heated at 160°C for 24 h. The resulting dark solution was diluted with EtOAc and H₂O. The organic layer was washed with H₂O, brine and dried (MgSO₄). Evaporation afforded a dark residue that was purified by Gilson HPLC (MeCN-H₂O 5%→95%) to afford the title compound (7% yield).

Example **260** was prepared in a similar manner using N-[1-Amino-1-methylsulfanyl-meth-(E)-ylidene]-cyanamide. MS **is M+H unless noted.**

| **Ex. No.** | **Compound Name** | **MS** | **¹H NMR (300 MHz; d₆-DMSO; δ ppm) unless otherwise noted** |
|---|---|---|---|
| 260 | 2-{[(E)-amino(cyanoimino)-methyl]amino}-5-phenyl-N-[(3R)-piperidin-3-yl]thiophene-3-carboxamide | 369 | 1.14-1.94 (m, 4 H) 2.55 - 2.87 (m, 2 H) 3.73 - 4.10 (m, 3 H) 6.74 - 7.79 (m, 7 H) |

### Example 261

### 5-Phenyl-2-(3-pyrazin-2-yl-ureido)-thiophene-3-carboxylic acid (S)-azepan-3-ylamide

**pyrazine-2-carbohydrazide.** To a stirred solution of methyl pyrazine-2-carboxylate (11.1 g, 80 mmol) in 140 mL of EtOH was added hydrazine hydrate (15.6 mL, 320 mmol). The resultant solution was heated to reflux for 2h. The solvent was removed under reduced pressure and dried under high vacuum to yield the title amide (11.1 g, 100%) as a white solid. The product was used in subsequent steps without purification. ¹H NMR (d₆-DMSO δ 10.1, br s, 1H; δ 9.12, d, 1H; δ 8.83, d, 1H; δ 8.70, dd, 1H; δ 4.64, br s, 2H), LC/MS (APCI, ES, M+H=139).

**pyrazine-2-carbonyl azide.** pyrazine-2-carbohydrazide (11.1 g, 80 mmol) was dissolved in 140 mL of water and charged with 6N HCl (13.3 mL, 80 mmol) and cooled to 0°C. To the stirred reaction mixture was added a solution of sodium nitrite (8.3 g, 120 mmol) in 80 mL of water was added slowly over a period of 15-30 minutes using an addition funnel. After the addition was complete the reaction was warmed to room temperature and stirred for an additional 5h. The solution was the neutralized by the careful addition of solid NaHCO₃ and then extracted with CHCl₃ (3x). The pooled organic fractions were dried over Na₂SO₄, filtered, concentrated and dried under high vacuum overnight to yield 2.5 g (21 %) the title acyl azide. The product was used in subsequent steps without purification. ¹H NMR (d₆-DMSO δ 9.30, d, 1H; δ 9.03, d, 1H; δ 8.90, dd, 1H).

***tert*-butyl (3*S*)-3-{[(5-phenyl-2-{[(pyrazin-2-ylamino)carbonyl]amino}-3-thienyl)carbonyl]amino}azeoane-1-carboxylate.** A solution of *tert*-butyl (3*S*)-3-{[(2-amino-5-phenyl-3-thienyl)carbonyl]amino}azepane-1-carboxylate [prepared as in Example 1] (0.71 g, 1.7 mmol) and pyrazine-2-carbonyl azide (0.5 g, 3.4 mmol) in 20 mL of anhydrous DME was refluxed for 2h. The solvent was removed under reduced pressure and the crude product was purified using ISCO MPLC (40-60% EtOAc/hexanes) to give the title 0.45 g (50%) compound as a light yellow solid. ¹H NMR (d₆-DMSO δ 12.6, br s, 0.5H; δ 12.5, br s, 0.5H; δ 10.94, s, 0.5H; δ 10.92, s, 0.5H; δ 8.93, s, 0.5H; δ 8.90, s, 0.5H; δ 8.34, d, 1H; δ 8.30, t, 1H; δ 8.08, d, 0.5H; δ 7.94, d, 0.5H; δ 7.91, s, 0.5H; δ 7.82, s, 0.5H; δ 7.60, d, 2H; δ 7.43, t, 2H; δ 7.29, t, 1H; δ 4.27, m, 0.5H; δ 4.19, m, 0.5H; δ 3.69, m, 1H; δ 3.45, m, 1H; δ 3.20, m, 2H; δ 1.79, m, 3H; δ 1.60, m, 2H; δ 1.43, s, 4.5H; δ 1.38, s+m, 5.5H), LC/MS (APCI, ES, M+H=537).

**5-Phenyl-2-(3-pyrazin-2-yl-ureido)-thiophene-3-carboxylic acid (S)-azepan-3-ylamide.** To a stirred solution of *tert*-butyl (3*S*)-3-{[(5-phenyl-2-{[(pyrazin-2-ylamino)carbonyl]amino}-3-thienyl)carbonyl]amino}azepane-1-carboxylate (0.45 g, 0.84 mmol) in 10 mL of MeOH is added 10 mL (40 mmol) of 4.0 N HCl in dioxane. The solution was stirred at room temperature for 4h and then concentrated under vacuum. The residue was partially recrystallized by triturating in refluxing 2-propanol to yield the title compound are a light orange solid (0.30 g, 75%). ¹H NMR (d₆-DMSO δ 12.6, br s, 1H; δ 10.9, s, 1H; δ 9.49, br s, 1H; δ 9.20, br s, 1H; δ 8.88, s, 1H; δ 8.51, d, 1H; δ 8.36, dd, 1H; δ 8.30, d, 1H; δ 8.07, s, 1H; δ 7.62, d, 2H; δ 7.43, t, 2H; δ 7.29, t, 1H; δ 4.42, m, 1H; δ 3.33, m, 1H; δ 3.23, m, 2H; δ 3.10, m, 1H; δ 2.02, m, 1H; δ 1.86, m, 4H; δ 1.62, m, 1H;), LC/MS (APCI, ES, M+H=437).

### Example 262

### 5-Phenyl-2-(3-pyrazin-2-yl-ureido)-thiophene-3-carboxylic acid (S)-piperidin-3-ylamide

***tert*-butyl (3*S*)-3-{[(2-amino-5-phenyl-3-thienyl)carbonyl]amino}piperidine-1-carboxylate.** To a stirred solution of 2-amino-5-phenylthiophene-3-carboxylic acid [prepared as in Example 1] (6.2 g, 28.3 mmol) in 40 mL of anhydrous DMF is added (S)-3-Amino-azepane-1-carboxylic acid tert-butyl ester (6.2 g, 28.3 mmol) and BOP (18.8 g, 42.4 mmol). The reaction mixture was stirred overnight at room temperature. The solution was diluted with water and EtOAc. The organic layer was separated and set aside. The remaining aqueous layer was extracted with EtOAc (2x) and then the combined organic extracts were pooled and washed with brine. The resultant EtOAc solution was dried over Na₂SO₄, filtered, and concentrated under vacuum to yield a brown solid. Purification was performed by flash Biotage MPLC (SiO₂, 33% EtOAc/hexanes) to give 5.0 g (44%) an off-white solid. ¹H NMR (d₆-DMSO δ 7.64, s, 1H; δ 7.49, br s, 2H; δ 7.47, d, 1H; δ 7.40, t, 2H; δ 7.35, t, 2H; δ 7.17, t, 1H; δ 3.74, m, 2H; δ 2.79, m, 2H; δ 1.88, m, 1H; δ 1.74, m, 1H; δ 1.44, m, 3H; δ 1.39, s, 9H), LC/MS (APCI, ES, M+H=402).

***tert-butyl* (3*S*)-3-{[(5-phenyl-2-{[(pyrazin-2-ylamino)carbonyl]amino}*-*3-thienyl)carbonyl]amino}piperidine-1-carboxylate.** A solution of *tert*-butyl (3*S*)-3-{[(2-amino-5-phenyl-3-thienyl)carbonyl]amino}piperidine-1-carboxylate (2.0 g, 5 mmol) and pyrazine-2-carbonyl azide (1.5 g, 10 mmol) in 20 mL of anhydrous DME was refluxed for 2h. The solvent was removed under reduced pressure and the crude product was purified using ISCO MPLC (40-60% EtOAc/hexanes) to give the title 2.0 g (77%) compound as a light yellow solid. ¹H NMR (d₆-DMSO δ 12.5, br s, 1H; δ 10.95, s, 1H; δ 8.93, s, 1H; δ 8.36, m, 1H; δ 8.31, d, 1H; δ8.01, br s, 1H; δ 7.90, s, 1H; δ 7.61, d, 2H; δ 7.44, t, 2H; δ 7.29, t, 1H; δ 3.74, m, 2H; δ 2.83, m, 2H; δ 1.93, m, 1H; δ 1.77, m, 1H; δ 1.57, m, 1H; δ 1.47, m, 2H; δ 1.39, s, 9H), LC/MS (APCI, ES, M+H=523).

**5-Phenyl-2-(3-pyrazin-2-yl-ureido)-thiophene-3-carboxylic acid (S)-piperidin-3-ylamide.** To a stirred solution of *tert*-butyl (3*S*)-3-[(5-phenyl-2-{[(pyrazin-2-ylamino)carbonyl]amino}-3-thienyl)carbonyl]amino}piperidine-1-carboxylate (2.0 g, 3.8 mmol) in 20 mL of MeOH is added 20 mL (80 mmol) of 4.0 N HCl in dioxane. The solution was stirred at room temperature for 4h and then concentrated under vacuum. The residue was partially recrystallized by triturating in refluxing 2-propanol to yield the title compound are a lightly colored solid (1.6 g, 92%). ¹H NMR (d₆-DMSO δ 12.58, br s, 1H; δ 10.96, s, 1H; δ 9.35, br s, 1H; δ 9.12, br s, 1H; δ 8.89, s, 1H;δ8.52,d, 1H; δ 8.34,m, 1H;88.31,m, 1H;δ.15,s, 1H; δ 7.64, d, 2H; δ 7.43, t, 2H; δ 7.29, t, 1H; δ 4.31, m, 1H; δ 3.33, m, 1H; δ 3.15, m, 1H; δ 2.96, m, 2H; δ 1.95, m, 2H; δ 1.71, m, 2H), LC/MS (APCI, ES, M+H=423).

Example **263** was synthesized in a similar manner as Example 262 using the appropriate starting materials. **MS** is M+H unless noted.

| **Ex. No.** | **Compound Name** | **MS** | **¹H NMR (300 MHz; d₆-DMSO; δ ppm) unless otherwise noted** |
|---|---|---|---|
| 263 | 5-(4-Chloro-phenyl)-3-(3-pyrazin-2-yl-ureido)-thiophene-2-carboxylic acid (S)-piperidin-3-ylamide | 458 | 1.51 - 1.79 (m, 2 H) 1.78 - 1.97 (m, 2 H) 2.66 - 2.92 (m, 2 H) 3.58 - 3.81 (m, 1 H) 3.96 - 4.32 (m, 1 H) 4.93 - 5.23 (m, 1 H) 7.20 (s, 1 H) 7.49 (d, *J*=8.48 Hz, 4 H) 7.65 (d, *J*=8.40 Hz, 2 H) 7.76 (d, *J*=8.40 Hz, 2 H) 8.17 (d, *J*=7.54 Hz, 1 H) 8.21 (d, *J*=2.64 Hz, 1 H) 8.24 - 8.28 (m, 2 H) |

## Claims

1. A compound of formula (I): 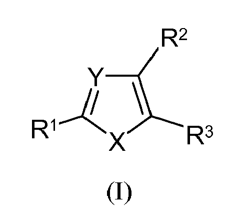 wherein:
X is selected from NH, S and O;
Y is selected from CH or N;
R¹ is selected from cyano, isocyano, C₁₋₆alkyl, -NR¹¹R¹², C₁₋₆alkoxy, C₂₋₆alkenyl, C₂₋₆alkynyl, cycloalkyl, cycloalkenyl, aryl, and heterocyclyl, provided R¹ is not thienyl; and wherein R¹ may be optionally substituted on one or more carbon atoms by one or more R⁹; and wherein if said R¹ contains an -NH- moiety, the nitrogen of said moiety may be optionally substituted by a group selected from R¹⁰;
R² and R³ are each independently selected from -C(=O)NR⁶R⁷ -SO₂NR¹⁶R¹⁷, -NHC(=O)NHR⁴, and -NHC(=NR⁸)NH₂;
R⁴ is selected from H, OH, -NR¹¹R¹², benzyl, C₁₋₆alkoxy, cycloalkyl, cylcoalkenyl, aryl, heterocyclyl, mercapto, CHO, -COaryl, -CO(C₁₋₆alkyl), -CONR³⁰R³¹, -CO₂(C₁₋₆alkyl), -CO₂aryl, -CO₂NR³⁰R³¹, -Salkyl, -SO(C₁₋₆alkyl), -SO₂(C₁₋₆alkyl), -Saryl, -SOaryl, -SO₂aryl,-SO₂NR³NR³¹ and -(C₁₋₆alkyl)SO₂ NR³⁰R³¹ wherein R⁴ may be optionally substituted on one or more carbon atoms by one or more R¹⁵; and wherein if said heterocyclyl contains a -NH- moiety, the nitrogen may be optionally substituted by a group selected from R¹⁴;
R⁶ and R⁷ are each independently selected from H, OH, OCH₃, C₁₋₆alkoxy, -NH₂, - NHCH₃, -N(CH₃)₂, (C₁₋₃alkyl)NR¹¹R¹², -CH₂CH₂OH, cycloalkyl, and a 5, 6, or 7- membered heterocyclyl ring containing at least one nitrogen atom, provided R⁶ and R⁷ are not both H; alternatively R⁶ and R⁷ taken together with the N to which they are attached form a heterocyclic ring; wherein R⁶ and R⁷ independently of each other may be optionally substituted on one or more carbon atoms by one or more R¹⁸; and wherein if said heterocyclyl contains a -NH- moiety, the nitrogen of said moiety may be optionally substituted by a group selected from R¹⁹;
R⁸ is selected from cyano, isocyano, -SO₂(C₁₋₆alkyl), -SO₂-aryl; -SO₂cycloalkyl, - SO₂cycloalkenyl, -SO₂heterocyclyl, and CF₃; wherein R⁸ may be optionally substituted on one or more carbon atoms by one or more R²³;
R⁹, R¹⁵, R¹⁸, R²³, R²⁴ and R³³ are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl) -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R⁹, R¹⁵, R¹⁸, R²³, R²⁴ and R³³ independently of each other may be optionally substituted on carbon by one or more R²⁰ and on nitrogen of any moiety that contains an NH or NH₂ by R²¹;
R¹⁰, R¹⁴, R¹⁹, R²⁵ and R³⁴ are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H, -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R¹⁰, R¹⁴, R¹⁹, R²⁵ and R³⁴ independently of each other may be optionally substituted on carbon by one or more R²² and on nitrogen of any moiety that contains an NH or NH₂ by R²³ ;
R¹¹ and R¹² are independently selected from H, C₁₋₆alkyl, cycloalkyl, aryl, heterocyclyl; alternatively R¹¹ and R¹² taken together with the N to which they are attached form a heterocyclic ring; wherein R¹¹ and R¹² independently of each other may be optionally substituted on carbon by one or more R³³_{;} and wherein if said heterocyclyl contains a -NH- moiety, the nitrogen of said moiety may be optionally substituted by a group selected from R³⁴;
R¹⁶ and R¹⁷ are each independently selected from H, OH, OCH₃, C₁₋₆alkoxy, NH₂, -NHCH₃, -N(CH₃)₂, (C₁₋₃alkyl)NR¹¹R¹², -CH₂CH₂OH, cycloalkyl, aryl, or a 5, 6 or 7-membered heterocyclyl ring containing at least one nitrogen atom, provided R¹⁶ and R¹⁷ are not both H; alternatively R¹⁶ and R¹⁷ taken together with the N to which they are attached form an optionally substituted heterocyclic ring; wherein R¹⁶ and R¹⁷ independently of each other may be optionally substituted on one or more carbon atoms by one or more R²⁴; and wherein if said heterocyclyl contains an -NH- moiety, the nitrogen of said moiety may be optionally substituted by a group selected from R²⁵;
R²⁰, R²² and R³² are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl) -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R²⁰, R²¹ and R³² independently of each other may be optionally substituted on carbon by one or more R²⁶ and on nitrogen of any moiety that contains an NH or NH₂ by R²⁷;
R²¹, R²³ and R³⁵ are each independently selected from halogen, nitro, -NR³⁰R³¹ cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl), -CO₂(NR³⁰R³¹), mercapto, -S(C₁₋₆alkyl), -SO(C₁₋₆alkyl), -SO₂(C₁₋₆alkyl), -SO₂NR³⁰R³¹; wherein R²¹, R²³ and R³⁵ independently of each other may be optionally substituted on carbon by one or more R²⁸ and on nitrogen of any moiety that contains an NH by R²⁹ ;
R²⁶ and R²⁸ are each independently selected from halogen, nitro, -NR³⁰R³¹ cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁-₆alkyl)CO(C₁₋₆alkyl), -NHSO₂(C₁₋₆alkyl), carboxy, -amidino, -CHO, -CONR³⁰R³¹, -CO(C₁₋₆alkyl), -COheterocyclyl, -COcycloalkyl, -CO₂H -CO₂(C₁₋₆alkyl), -CO₂(aryl) -CO₂(NR³⁰R³¹), mercapto, -S(C₁₋₆alkyl), -SO(C₁₋₆alkyl), -SO₂(C₁₋₆alkyl), -SO₂NR³⁰R³¹;
R²⁷ and R²⁹ are each independently selected from halogen, nitro, -NR³⁰R³¹, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR³⁰R³¹, -N(C₁₋₆alkyl)CONR³⁰R³¹, -NHCOalkyl, -NHCO₂(C₁₋₆alkyl); -NHCO₂H, -N(C₁₋₆alkyl)CO(C₁-₆alkyl), -NHSO₂(C₁₋₆alkyl), carboxy, -amidino, -CHO, -CONR³⁰R³¹, -CO(C₁₋₆alkyl), - COheterocyclyl, -COcycloalkyl, -CO₂H, -CO₂(C₁₋₆alkyl), -CO₂(aryl) -CO₂(NR³⁰R³¹), mercapto, -S(C₁₋₆alkyl), -SO(C₁₋₆alkyl), -SO₂(C₁₋₆alkyl), -SO₂NR³⁰R³¹;
R³⁰ and R³¹ are each independently selected from halogen, nitro, -NH₂, cyano, isocyano, C₁₋₆alkyl, C₂₋₆alkenyl, C₂₋₆alkynyl, aryl, cycloalkyl, heterocyclyl, hydroxy, keto(=O), -O(C₁₋₆alkyl), -Oaryl, -OCOalkyl, -NHCHO, -N(C₁₋₆alkyl)CHO, -NHCONR¹¹R¹², -N(C₁₋₆alkyl)CONR¹¹R¹², -NHCOalkyl, -NHCO₂(C₁₋₆alkyl); -NHCO₂H, -N(C₁₋₆alkyl)CO(C₁-₆alkyl), -NHSO₂(C₁₋₆alkyl), carboxy, -amidino, -CHO, -CONR³⁰R³¹, -CO(C₁₋₆alkyl), - COheterocyclyl, -COcycloalkyl, -CO₂H, -CO₂(C₁₋₆alkyl), -CO₂(aryl) -CO₂(NR³⁰R³¹), mercapto, -S(C₁₋₆alkyl), -SO(C₁₋₆alkyl), -SO₂(C₁₋₆alkyl), -SO2NR¹¹R¹²; wherein R³⁰ and R³¹ independently of each other may be optionally substituted on carbon by one or more R³²; and wherein if said heterocyclyl contains a -NH- or NH₂ moiety, the nitrogen of said moiety may be optionally substituted by a group selected from R³⁵;
or a pharmaceutically acceptable salt thereof;
provided that when X is S; Y is CH; R₂ is C(=O)NR⁶R⁷; and R³ is NHC(=O)NHR⁴; then R¹ cannot be 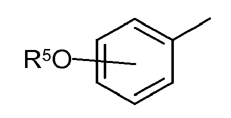 wherein R⁵ is selected from H, optionally substituted carbocyclyl, or optionally substituted C₁₋₆alkyl; with the further proviso that said compound is not
5-Methyl-2-ureido-thiophene-3-carboxylic acid (1-ethyl-piperidin-3-yl)-amide; [3-((S)-3-Amino-azepane-1-carbonyl)-5-ethyl-thiophen-2-yl]-urea;
2-Morpholin-4-yl-4-ureido-thiazole-5-carboxylic acid (S)-piperidin-3-ylamide; 2-Methyl-5-ureido-oxazole-4-carboxylic acid (S)-piperidin-3-ylamide; 5-(4-Chloro-phenyl)-3- {3-[(R)-1-(2,2,2-trifluoro-acetyl)-piperidin-3-yl]-ureido}-thiophene-2-carboxylic acid (S)-piperidin-3-ylamide; or
N-(3- {[(3S)-3-aminoazepan-1-yl]carbonyl}-5-pyridin-2-yl-2-thienyl)urea.

2. A compound of formula (I), or a pharmaceutically acceptable salt thereof, according to claim 1, wherein R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰.

3. A compound of formula (I), or a pharmaceutically acceptable salt thereof, according to claim 1 or 2 wherein R¹ is aryl optionally substituted on one or more carbon atoms by one or more R⁹.

4. A compound of formula(I), or a pharmaceutically acceptable salt thereof, according to any one of claims 1-3 wherein one of R² and R³ is -SO₂N R¹⁶R¹⁷ and the other is -NHC(=O)NHR⁴.

5. A compound of formula (I), or a pharmaceutically acceptable salt thereof, according to any one of claims 1-4 wherein one of R² and R³ is -C(=O)NR⁶R⁷ and the other is -NHC(=O)NHR⁴.

6. A compound of formula (I), or a pharmaceutically acceptable salt thereof, according to any one of claims 1-5 wherein one of R² and R³ is C(=O)NR⁶R⁷ and the other is -NHC(=O)NHR⁴; R⁶ is H and R⁷ is a 5, 6, or 7-membered heterocycyclyl ring containing at least one nitrogen atom; and wherein said heterocyclyl may be optionally substituted on one or more carbon atoms by one or more R¹⁸; and further wherein if said heterocyclyl contains an -NH- moiety, the nitrogen of said moiety may be optionally substituted by a group selected from R¹⁹.

7. A compound of formula (I), or a pharmaceutically acceptable salt thereof, according to any one of claims 1 to 5 wherein R⁶ and R⁷ taken together with the N to which they are attached form an optionally substituted heterocyclic ring which may be optionally substituted on one or more carbon atoms by one or more R¹⁸; and wherein if said heterocyclyl contains a -NH- moiety, the nitrogen of said moiety may be optionally substituted by a group selected from R¹⁹.

8. A compound of formula (II), or a pharmaceutically acceptable salt thereof, 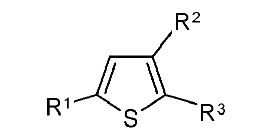 wherein R¹, R², and R³ are as defined in any one of claims 1-7

9. A compound, or pharmaceutically acceptable salt according to any one of claims 1, 2, 5, 6 and8 wherein
R² is -C(=O)NR⁶R⁷;
R³ is -NHC(=O)NHR⁴;
R⁶ is H; R⁷ is a 5, 6, or 7-membered heterocycyclyl ring containing at least one nitrogen atom; wherein said heterocyclyl may be optionally substituted on one or more carbon atoms by one or more R¹⁸; and further wherein if said heterocyclyl contains an -NH- moiety, the nitrogen of said moiety may be optionally substituted by a group selected from R¹⁰;and
R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁹.

10. A compound, or pharmaceutically acceptable salt thereof, according to any one of claims 1, 2, 5, 6, 8 and 9 wherein
R³ is -C(=O)NR⁶R⁷;
R² is -NHC(=O)NHR⁴
R⁶ is H; R⁷ is a 5, 6, or 7-membered heterocycyclyl ring containing at least one nitrogen atom wherein R⁷ may be optionally substituted on one or more carbon atoms by one or more R¹⁸; and wherein if said heterocyclyl contains a -NH- moiety, the nitrogen of said moiety may be optionally substituted by a group selected from R¹⁹; and R¹ is selected from cycloalkyl, cycloalkenyl, aryl, and heterocyclyl, provided R¹ is not thienyl; and wherein R¹ may be optionally substituted on one or more carbon atoms by one or more R⁹; and further wherein if said heterocyclyl contains an -NH- moiety, the nitrogen of said moiety may be optionally substituted by a group selected from R¹⁰

11. A compound, or pharmaceutically acceptable salt, according to claim 1 selected from 5-(3-Fluoro-phenyl)-3-ureido-thiophene-2-carboxylic acid (S)-piperidin-3-ylamide; 5-Phenyl-2-ureido-thiophene-3-carboxylic acid (S)-piperidin-3-ylamide; 5-(3,5-Difluoro-phenyl)-2-ureido-thiophene-3-carboxylic acid (S)-piperidin-3-ylamide; 5-(4-Fluoro-phenyl)-2-ureido-thiophene-3-carboxylic acid (S)-piperidin-3-ylamide; 5-(4-Chloro-phenyl)-2-ureido-thiophene-3-carboxylic acid (S)-piperidin-3-ylamide; 5-(3-Chloro-phenyl)-2-ureido-thiophene-3-carboxylic acid (S)-piperidin-3-ylamide; 5-[4-(Piperidine-1-carbonyl)-phenyl]-2-ureido-thiophene-3-carboxylic acid (S)-piperidin-3-ylamide;
5-(4-Cyano-phenyl)-3-ureido-thiophene-2-carboxylic acid (S)-piperidin-3-ylamide; 5-[4-(Piperidine-1-carbonyl)-phenyl]-3-ureido-thiophene-2-carboxylic acid (S)-piperidin-3-ylamide;
5-(3,4-Difluoro-phenyl)-3-ureido-thiophene-2-carboxylic acid (S)-piperidin-3-ylamide; 5-(3-Chloro-phenyl)-3-ureido-thiophene-2-carboxylic acid (S)-piperidin-3-ylamide; 5-(2,3-Difluoro-phenyl)-3-ureido-thiophene-2-carboxylic acid (S)-piperidin-3-ylamide; 5-(2,4-Difluoro-phenyl)-3-ureido-thiophene-2-carboxylic acid (S)-piperidin-3-ylamide; 5-(3,5-Difluoro-phenyl)-3-ureido-thiophene-2-carboxylic acid (S)-piperidin-3-ylamide; 5-Phenyl-3-ureido-thiophene-2-carboxylic acid (S)-piperidin-3-ylamide; and 5-(4-Chloro-phenyl)-3-ureido-thiophene-2-carboxylic acid (S)-piperidin-3-ylamide.

12. A compound of formula (I) or a pharmaceutically acceptable salt thereof, as claimed in any one of claims 1-11, for use in the treatment or prophylaxis of disorders associated with cancer.

13. A compound of formula (I) or a pharmaceutically acceptable salt thereof, as claimed in any one of claims 1-11, for the use in treatment or prophylaxis of neoplastic disease such as cervical cancer , cancer of the head and neck, carcinoma of the breast, ovary, lung(non small cell), pancreas,, colon, prostate or other tissues, as well as leukemias and lymphomas, tumors of the central and peripheral nervous system, and other tumor types such as melanoma, fibrosarcoma and osteosarcoma.

14. A compound of formula (I) or a pharmaceutically acceptable salt thereof, as claimed in any one of claims 1-11, for use in the treatment or prophylaxis of proliferative diseases including autoimmune, inflammatory, neurological, and cardiovascular diseases.

15. A method of limiting cell proliferation in a human or animal comprising administering to said human or animal a therapeutically effective amount of a compound of formula (I) or a pharmaceutically acceptable salt thereof, as claimed in any one of claims 1-11.

16. A method of treatment of a human or animal suffering from cancer comprising administering to said human or animal a therapeutically effective amount of a compound of formula (I) or a pharmaceutically acceptable salt thereof, as claimed in any one of claims 1-11.

17. A method of prophylaxis treatment of cancer comprising administering to a human or animal in need of such treatment a therapeutically effective amount of a compound of formula (I) or a pharmaceutically acceptable salt thereof, as claimed in any one of claims 1-11.

18. A method of treatment of a human or animal suffering from a neoplastic disease such as cervical cancer, cancer of the head and neck, carcinoma of the breast, ovary, lung (non small cell), pancreas, colon, prostate or other tissues, as well as leukemias and lymphomas, tumors of the central and peripheral nervous system, and other tumor types such as melanomasarcomas including fibrosarcoma and osteosarcoma, malignant brain tumors, comprising administering to said human or animal a therapeutically effective amount of a compound of formula (I) or a pharmaceutically acceptable salt thereof, as claimed in any one of claims 1-11.

19. A method of treatment of a human or animal suffering from a proliferative disease such as autoimmune, inflammatory, neurological, and cardiovascular diseases comprising administering to said human or animal a therapeutically effective amount of a compound of formula (I) or a pharmaceutically acceptable salt thereof, as claimed in any one of claims 1-11.

20. A method of treating cancer comprising administering to a human a compound of formula (I) or a pharmaceutically acceptable salt thereof, as claimed in any one of claims 1-11, and an anti-tumor agent.

21. A method of treating cancer comprising administering to a human or animal a compound of formula (I) or a pharmaceutically acceptable salt thereof, as claimed in any one of claims 1-11, and a DNA damaging agent.

22. A method for the treatment of infections associated with cancer comprising administering to a human or animal in need of such treatment a therapeutically effective amount of a compound of formula (I) or a pharmaceutically acceptable salt thereof, as claimed in any one of claims 1-11.

23. A method for the prophylaxis treatment of infections associated with cancer comprising administering to a human or animal in need of such treatment a therapeutically effective amount of a compound of formula (I) or a pharmaceutically acceptable salt thereof, as claimed in any one of claims 1-11.

24. A pharmaceutical composition comprising a compound of formula (I) or a pharmaceutically acceptable salt thereof, as claimed in any one of claims 1-11, together with at least one pharmaceutically acceptable carrier, diluent or excipent.

25. The use of a compound of formula (I) or a pharmaceutically acceptable salt thereof, as claimed in any one of claims 1-11, in the preparation of a medicament.

26. The use of a compound of formula (I) or a pharmaceutically acceptable salt thereof, as claimed in any one of claims 1-11, in the preparation of a medicament for the treatment or prophylaxis of cancer.

27. The use of a compound of formula (I) or a pharmaceutically acceptable salt thereof, as claimed in any one of claims 1-11, in the preparation of a medicament for the treatment or prophylaxis of neoplastic disease such as carcinoma of the breast, ovary, lung, colon, prostate or other tissues, as well as leukemias and lymphomas including CLL and CML, tumors of the central and peripheral nervous system, and other tumor types such as melanoma, fibrosarcoma and osteosarcoma.

28. The use of a compound of formula (I) or a pharmaceutically acceptable salt thereof, as claimed in any one of claims 1-11, in the preparation of a medicament for the treatment or prophylaxis of proliferative diseases including autoimmune, inflammatory, neurological, and cardiovascular diseases.

29. A method of inhibiting CHK1 kinase comprising administering to an animal or human in need of said inhibiting a therapeutically effective amount of a compound of formula (I) or a pharmaceutically acceptable salt thereof, as claimed in any one of claims 1-11.

30. The use of a compound of formula (I) or a pharmaceutically acceptable salt thereof, as claimed in any one of claims 1-11, in the preparation of a medicament for use in the inhibition of CHK1 kinase activity.

31. The use of a compound of formula (I) or a pharmaceutically acceptable salt thereof, as claimed in any one of claims 1-11, in the preparation of a medicament for use in limiting cell proliferation.

32. A process for the preparation of a compound of formula (I) or a pharmaceutically acceptable salt thereof, as defined in any one of claims 1 to 11, which comprises:
a. reacting a compound with formula (III) wherein A is thienyl and L is a displaceable
group 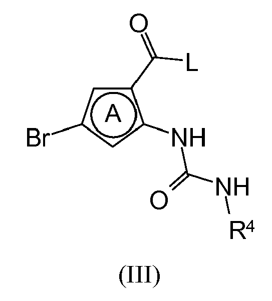 with an amine of formula (IV); 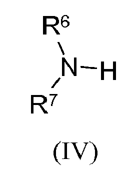 to yield a compound of formula (V) 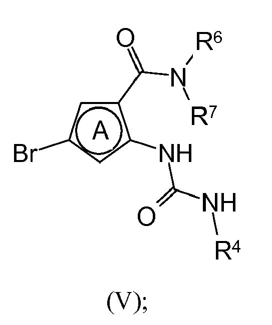
b. reacting a compound with formula (V) with a boronic acid or ester
to form a compound of formula (I); and
c. optionally
i) converting a compound of the formula (I) into another compound of the formula (I); and/or
ii) forming a pharmaceutically acceptable salt thereof.

33. A process for the preparation of a compound of formula (I) or a pharmaceutically acceptable salt thereof, as defined in any one of claims 1 to 11, which comprises:
a. reacting a compound of formula (VII) wherein A is thienyl and R is a hydrocarbon radical; 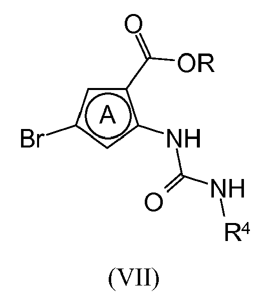 with a boronic acid or ester to form a compound of formula (VIII): 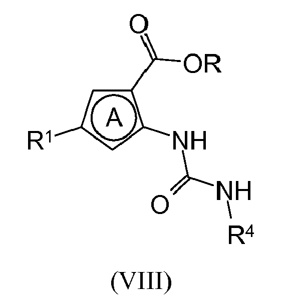
b. reacting a compound of formula (VIII) with an amine of formula (IV) 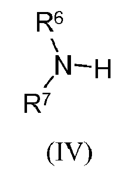 to form a compound of formula (I); and
c. optionally,
i) converting a compound of the formula (I) into another compound of the formula (I); and/or
ii) forming a pharmaceutically acceptable salt thereof.

34. A process for the preparation of a compound of formula (I) or a pharmaceutically acceptable salt thereof, as defined in any one of claims 1 to 11 which comprises:
a. reacting a compound of formula (IX) wherein A is thienyl and R is a hydrocarbon
radical; 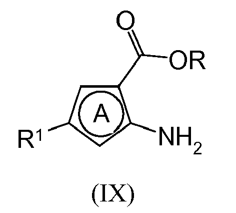 with a concentrated hydroxide base to form a compound of formula (X); 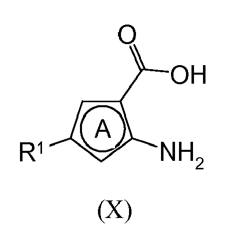
b. reacting the compound of formula (X) with an amine of formula (IV) 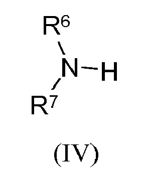 to form a compound of formula (XI) 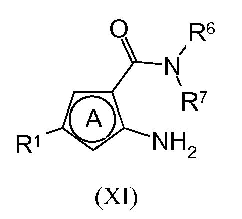
c. reacting the compound of formula (XI) with a compound selected from compounds of
formulas (XII), (XIII) and a carbonylation reagent or (XIV); 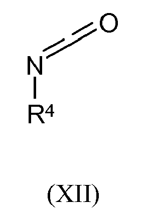 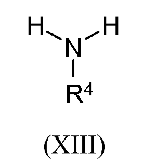 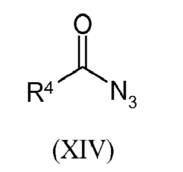 to form a compound of formula (I); and
d. optionally,
i) converting a compound of the formula (I) into another compound of the formula (I); and/or
ii) forming a pharmaceutically acceptable salt thereof.

35. A compound of formula (XV), (XVI) or (XI) 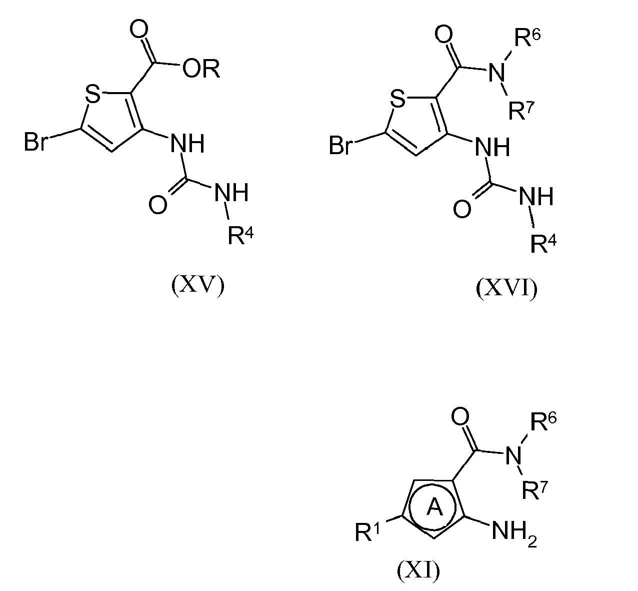 wherein R¹ is aryl and R⁴, R⁶ and R⁷ are as defined in formula (I), A is a thienyl ring and R is a hydrocarbon radical and provided that the compound of formula (XI) is not 3-Amino-5-(4-chloro-phenyl)-thiophene-2-carboxylic acid [(1R,2R)-2-(2,4-difluoro-phenyl)-2-hydroxy-1-methyl-3-[1,2,4]triazol-1-yl-propyl]-amide.

36. The use of compounds of formulas (XV), (XVI), (XI) 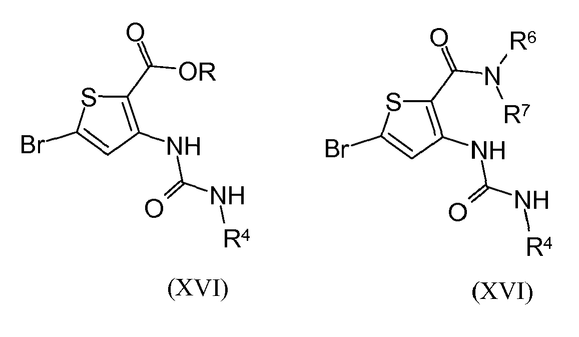 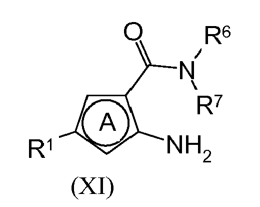 wherein R¹ is aryl and R⁴, R⁶ and R⁷ are as defined in formula (I), A is a thienyl ring and R is a hydrocarbon radical or pharmaceutically acceptable salts or an *in vivo* hydrolysable precursor in the manufacture of a compound of formula (I) according to any one of claims 1-11.
